# EUROPEAN PATENT SPECIFICATION

(11) **EP 3 119 909 B1**
(45) Date of publication and mention of the grant of the patent: **13.11.2019**
(21) Application number: 15764167.1
(22) Date of filing: 18.03.2015
(51) Int. Cl.: C12Q 1/68, G01N 33/68

(54) **DUX4-INDUCED GENE EXPRESSION IN FACIOSCAPULOHUMERAL MUSCULAR DYSTROPHY (FSHD)**
DUX4-INDUZIERTE GENEXPRESSION IN FAZIOSKAPULOHUMERALER MUKSELDYSTROPHIE (FSHD)
EXPRESSION GÉNIQUE INDUITE PAR DUX4 EN DYSTROPHIE MUSCULAIRE FACIO-SCAPULO-HUMÉRALE (FSHD)

(30) Priority: 18.03.2014 US 201461955062 P
(43) Date of publication of application: 25.01.2017
(73) Proprietor: Fred Hutchinson Cancer Research Center, Seattle, WA 98109 (US); University of Rochester Medical Center, Rochester, New York 14642 (US); Leiden University Medical Center, 2333 ZA Leiden (NL)
(72) Inventor: TAPSCOTT, Stephen, J., Seattle, WA 98109 (US); TAWIL, Rabi, Rochester, NY 14642 (US); VAN DER MAAREL, Silvere, 2333 ZA Leiden (NL); YAO, Zizhen, Seattle, WA 98109 (US)
(74) Representative: J A Kemp LLP
(86) International application number: PCT/US2015/021301
(87) International publication number: WO 2015/143062

(56) References cited:
- WO-A2-2013/019623
- WO-A2-2013/033627
- US-A1- 2013 288 976
- US-A1- 2013 347 136
- YOSHIAKI CHINEN ET AL: "The leucine twenty homeobox ( LEUTX ) gene, which lacks a histone acetyltransferase domain, is fused to KAT6A in therapy-related acute myeloid leukemia with t(8;19)(p11;q13) : Novel KAT6A-Leutx Fusion in Therapy-Related AML", GENES CHROMOSOMES & CANCER., vol. 53, no. 4, 21 January 2014 (2014-01-21), pages 299-308, XP055390267, US ISSN: 1045-2257, DOI: 10.1002/gcc.22140
- ZIZHEN YAO ET AL: "DUX4-induced gene expression is the major molecular signature in FSHD skeletal muscle", HUMAN MOLECULAR GENETICS, vol. 23, no. 20, 26 May 2014 (2014-05-26), pages 5342-5352, XP055390284, gb ISSN: 0964-6906, DOI: 10.1093/hmg/ddu251
- GENG ET AL.: 'DUX4 activates germline genes, retroelements and immunemediators: Implications for facioscapulohumeral dystrophy.' DEV CELL . vol. 22, no. 1, 2012, ISSN 1534-5807 page 38051, XP055075932
- DATABASE GENBANK [Online] 27 January 2010 ncbi: 'Synthetic construct Homo sapiens clone IMAGE:100070098; IMAGE:100012225; FLH257484.01L tripartite motif-containing 43 (TRIM43) gene , encodes complete protein, Direct Submission', XP055227598 Database accession no. EU447016

### SEQUENCE LISTING

<110> Tapscott, Stephen J.
   Yao, Zizhen
   Tawil, Rabi
   Van der maarel, Silvere
<120> DUX4-INDUCED GENE EXPRESSION IN FACIOSCAPULOHUMERAL MUSCULAR DYSTROPHY (FSHD)
<130> FHCC.P0041WO
<140> UNKNOWN
   <141> 2015-03-18
<150> 61/955,062
   <151> 2014-03-18
<160> 85
<170> PatentIn version 3.5
<210> 1
   <211> 161
   <212> DNA
   <213> Homo sapiens
<400> 1
   ccgacagaag caagcacatt tccacactcg ggttcctcct gactctccta ggttccacta      60
   gaggagagta agtgccagtg ttccgccaac tcaggaactg accagaccct gcaggcacag     120
   tgagaagact cccgggagaa aactcaggag taaaaatgga g                         161
<210> 2
   <211> 181
   <212> **DNA**
   <213> Homo sapiens
<400> 2
   ccggtgttcc tcagctgcct cttcgatggg atggtgtttg tgagccccgt gctcctgaga      60
   gttggagcac agcaagcaga ggagactctt gtccatgtca cagaacatct tctttgtttg     120
   cctatgggtc ccacatattt gtttctcaga gctcaggaat tgccagagac tggcttttct     180
   g                                                                     181
<210> 3
   <211> 539
   <212> DNA
   <213> Homo sapiens
<400> 3
   ctctttcact ctcctccgcc atgcccgacc cggctaaatc tgctcctgcc cccaaaaagg 60
   gctccaagaa agccgtaacc aaggcccaga aaaaggacgg caagaagcgc aagcgcagcc 120
   gcaaagagag ttactctatc tacgtgtaca aggtgctgaa gcaagtccac cccgacaccg 180
   gcatctcatc gaaggccatg ggcatcatga actccttcgt caatgacatc tttgagcgca 240
   tcgctggcga ggcttcccgc ctggcgcatt acaacaagcg ctcgaccatc acctccaggg 300
   agatccagac ggccgtgcgc ctgctgctgc ccggggagct ggccaagcac gccgtgtccg 360
   agggcacaaa ggccgtcacc aagtacacca gctccaagtg agctctcgca gctgccagca 420
   atccaaaggc tcttttcaga gccactcacg cttccagaga aagagcctgt gcattttgct 480
   tgtagtcttt tgggtggact ccggccggcc tccattagga gggaagacgg cgggaaaag 539
<210> 4
   <211> 122
   <212> DNA
   <213> Homo sapiens
<400> 4
   gtttatcacg ttaaccggac aatcaaattt gttggccagt gttttcctag tggtgaaatc 60
   aggataaccg ttctctccaa atatttcctt aagttcctgc aataattctt cagaaaattt 120
   at 122
<210> 5
   <211> 324
   <212> DNA
   <213> Homo sapiens
<400> 5
   cagttctgac tgaagcggtc tccagctctg aagcggtctc cagctctgaa gctatctcca 60
   gctctgaagc ggtctccagc tctgaagcga tctccagctc tgaagcggtc tccagctctg 120
   aaacgatctc cagctctgaa acaatctcca gctctgaagc ggtctccagc tctgaaacaa 180
   tctccagctc tgaagcggtc tccagctctg aaacagtctc cagctctgaa gcggtctcca 240
   gctctgaagc ggcctccagc tatgaagcgg tctccagctt tgactccagt tgtttccagc 300
   tcttctggac tgacttcttt ccag 324
<210> 6
   <211> 76
   <212> DNA
   <213> Homo sapiens
<400> 6
   ctcggaattg gttgagcctg tccctcagtc cagtgatggg ccctgcactg agctctggat 60
   tcagaggctg gggcat 76
<210> 7
   <211> 60
   <212> DNA
   <213> Homo sapiens
<400> 7
   caggagctct gcagcacaca gctgatcgaa cccactcatt tctagaggac accatgtttg
<210> 8
   <211> 59
   <212> DNA
   <213> Homo sapiens
<400> 8
   gaaagctagt cacacatcag ctcagtgttc ggcccgggat tacccagtca accaaggag 59
<210> 9
   <211> 293
   <212> DNA
   <213> Homo sapiens
<400> 9
   ggagcatctg aagagtttaa gaccttgggg aaagagagtc ttgctgcttg cttgtgagac 60
   ttgccttgga aggtagataa gttcactgca tcctccagct ttttatttct ggtgagttct 120
   ccaagcactg agatgtgaac tggcctcatt cccttacaat gatactgtta ccagtagaag 180
   agattccagt tactggcagc atatctgcat gggtccataa gcaacttcag tccttgcctc 240
   ctcagaagaa agaattcgac tgaagggcat acagcggaaa aagagactga gca 293
<210> 10
   <211> 74
   <212> DNA
   <213> Homo sapiens
<400> 10
   agcctcacca ccattgctgt ctggtaccag cactgcttcc agttcaccag gttcagccca 60
   gtgacaaggc agag 74
<210> 11
   <211> 65
   <212> DNA
   <213> Homo sapiens
<400> 11
   cagcacttca tcctgccatc ttgtgaagaa gttgtcttgc ttcgcctttg ccttcctcca 60
   tgatt 65
<210> 12
   <211> 107
   <212> DNA
   <213> Homo sapiens
<400> 12
   tagtgacacc taaaaccaca gcaaaagatg gcagagcttc caccagccta ggttcctgaa 60
   tgactgcatg gagtgtgccc cagcccctgc tgacagttgg actttat 107
<210> 13
   <211> 176
   <212> DNA
   <213> Homo sapiens
<400> 13
   cagagactgg agttatgcag ccacaggcca aggaatgcca gcagccacca gaagctacaa 60
   gaggcaggaa acagattctc ccctagaacc tctggaggga gcaagtccct gctgacacct 120
   tgattttggc ccagtgaaac tgatttcaga cgtatggcct ccagaactgt gacaag 176
<210> 14
   <211> 82
   <212> DNA
   <213> Homo sapiens
<400> 14
   gaagaagctc tgagcaccgg agtgtggtga cagcgacctc cataaagacc tggctccatt 60
   ggattttcct gtttggtttc tg 82
<210> 15
   <211> 40
   <212> DNA
   <213> Homo sapiens
<400> 15
   tggtggaaga ggatggcgta caaagatttc agcggaagag 40
<210> 16
   <211> 136
   <212> DNA
   <213> Homo sapiens
<400> 16
   aaggactgca cttagaactg cattttggcg acctctgaaa ttcagtactg cagtgaatga 60
   gcttctgatc ttgaggtgaa cttaacaaaa ttatttttgg aaaaatcgtt gtgggaacca 120
   ttaaaagaac tccagg 136
<210> 17
   <211> 253
   <212> DNA
   <213> Homo sapiens
<400> 17
   ctattttcct ctgaacgggc tcagaaagct caggaaggcc ccagagagct tgctttccgg 60
   agattcccca tgccgccgtt aagaccaatt cggaaatcga ggcgtgagcc aagcttcaag 120
   cgtagccaga ggcgcaaatc tcagcccctc cccaaggccc tgaggtgcct tccccaggaa 180
   gacccaagta ccagccacca gtccctgtgg ctactggctg agttcagctg cagtgatcac 240
   agcacaaaac aag 253
<210> 18
   <211> 70
   <212> DNA
   <213> Homo sapiens
<400> 18
   tgggaaaata ataaacagag ctgtggaaca tatctttggg catggagtgt tggaaaagga 60
   ccttcttgag 70
<210> 19
   <211> 62
   <212> DNA
   <213> Homo sapiens
<400> 19
   cctggagcta ctgcttggtt ctctgagagg tcccagcacc ctgcaaactg agtccagatc 60
   tg 62
<210> 20
   <211> 62
   <212> DNA
   <213> Homo sapiens
<400> 20
   cctggagcta ctgcttggtt ctctgagagg tcccagcacc ctgcaaactg agtccagatc 60
   tg 62
<210> 21
   <211> 111
   <212> DNA
   <213> Homo sapiens
<400> 21
   gcaagactgc ctgggagaaa gattccccac tgctgtgtgt agaccgagga atcccaaggc 60
   tactcaagga gactcaatac agggacctca gacccactga tcagtgagtt g 111
<210> 22
   <211> 75
   <212> DNA
   <213> Homo sapiens
<400> 22
   agggaattca acacagaaga agctgctgat ttttgggaaa agaggaggcc atgcagtaaa 60
   gaattcgagt ggtcg 75
<210> 23
   <211> 134
   <212> DNA
   <213> Homo sapiens
<400> 23
   ggactgcact tagaactgca ttttggcgac ctctgaaatt cagtactgca gtgaatgagc 60
   ttctgacctt gaggtgaact taacaaaatt atttttggaa aaatcgttgt gggaaccatt 120
   aaaagaactc cagg 134
<210> 24
   <211> 45
   <212> DNA
   <213> Homo sapiens
<400> 24
   cccagtggaa gcagctggag gacagaggag cttccagcag aagag 45
<210> 25
   <211> 58
   <212> DNA
   <213> Homo sapiens
<400> 25
   ggtagagtga agtcctacag agttatcagg ttccagaccc tgccttctct tctgaaag 58
<210> 26
   <211> 200
   <212> DNA
   <213> Homo sapiens
<400> 26
   tgcacacaaa ggaccatgtg aagacactgt aggaaggcag ccatctgaaa gccaggaaga        60
   gaggcctcag aagaaaccaa acctgctgac cccttgatct tggacttccc agcctccaga       120
   gctgtgagga aataaatgta tgctgtttga gccacccagt ttgtgatatt tgattgtgac       180
   agtgctagca gactaataca                                                   200
<210> 27
   <211> 26
   <212> DNA
   <213> Homo sapiens
<400> 27
   ccagcttact gatcagtggg tctgag 26
<210> 28
   <211> 137
   <212> DNA
   <213> Homo sapiens
<400> 28
   acagcacata gagttaactg cattcaggtg acctctgaaa gtcaactgtg cagggaatga        60
   gctcctgatc ttggggagta cttaaaagaa ttctttcttg gaagaattac tgcaggaaac       120
   attcatagaa ccttggg                                                      137
<210> 29
   <211> 136
   <212> DNA
   <213> Homo sapiens
<400> 29
   cagcacttag agttaactgc attcaggtga cctctgaaag tcaactctgc agggaacgag        60
   gtcctgatct tggggagtac ttaaaagaat tttctcttgg aagaatcact gccagaaaca        120
   ttcatagaac cttggg                                                       136
<210> 30
   <211> 103
   <212> DNA
   <213> Homo sapiens
<400> 30
   ccttgccctc cctccatggg atgccctcca ccatgttatg atgcagtaga aggccttcac        60
   cagatgttgc ccctctacct ttgacttccc agcctccaaa atg                         103
<210> 31
   <211> 54
   <212> DNA
   <213> Homo sapiens
<400> 31
   tactgctcgg ttctctgaga ggttgcagca ccctgcaaac tgagtccaga tctg 54
<210> 32
   <211> 44
   <212> DNA
   <213> Homo sapiens
<400> 32
   gaacagatca ggactcagga tgcaagaccc tggtcatctc caag 44
<210> 33
   <211> 222
   <212> DNA
   <213> Homo sapiens
<400> 33
   attcttgtta cagttgagag attctaattg tgagagggat tcaacaagag gaagcttctc      60
   cattgctgac tttaccatga aagggtcaat gggacaagaa cttgagaagc tgagggtagt     120
   ccttgcggag acaacaggga cttcagtcct acaaccacag caactgaatt ctgccaacaa     180
   cctgagcaag cttggaagaa ggttcttctg caaagcctcc ag                        222
<210> 34
   <211> 290
   <212> DNA
   <213> Homo sapiens
<400> 34
   ggagcatctg aagactttaa gaccttgggg aaagagtctt gctgcttgct tgtgggactt      60
   gccttggaag gtagataagt tcactgcatc ctccaacttt ttatttttgg tgagttctcc     120
   aagcactgag atgtgaactg gcctcattcc cttacaatga tactgttacc agtagaagag     180
   attccagtta ctggcagcat agctgcatgg gtccataagc aacttcagtc cttgcctcct     240
   agaagaaaga attcgaccga agggcataca gtggaaaaag agactgagcg                290
<210> 35
   <211> 70
   <212> DNA
   <213> Homo sapiens
<400> 35
   ctaccaacag gaaaccttct ctttgggtcc tgaagtattt tcatatggaa ttgtgagttt      60
   ttgaccgaag                                                             70
<210> 36
   <211> 140
   <212> DNA
   <213> Homo sapiens
<400> 36
   cccatgctgc ccccatctcc tgatttgtga aattcatcag gtgtgcaccc ttctaatggc 60
   tgctccgatg aaaacagtaa aaacccatca agccagacca agaggagtga cctacttata 120
   tcagattgtg ttaactttct 140
<210> 37
   <211> 111
   <212> DNA
   <213> Homo sapiens
<400> 37
   accagctctc aggtctgtgg gtcggaggcc gggctgctta gtcttcccgg aaggtcttgg 60
   gtttccttgg tctacccagc agcagccagg agtctccctc gcaggcagtc t 111
<210> 38
   <211> 147
   <212> DNA
   <213> Homo sapiens
<400> 38
   gtcttctaca cgccacttcg ctgggctcct gtctctgtcg ctgagaggca ctctggagag 60
   tctgcggcag cctctgtcac acagggacct gcactggtcg tgggagccgc agagaggccc 120
   ccgggacacg gaggaattcg gaaaatg 147
<210> 39
   <211> 246
   <212> DNA
   <213> Homo sapiens
<400> 39
   caccaagcca gggaggtgac aagtatgtgg taaaatccga gcacgcagaa tttgggggaa 60
   caggactcta gactgcctct agagagagct gtattgaagg ccctgcgacc cagcacagag 120
   aagggaatgg tgctctaggt ccctgcctct gcagaagaca aatccaggaa gagaaccagg 180
   aaactgcaga gtagccagcc acatgcgaga aaaatctgaa ggagacgaaa atgtttcagg 240
   agaaag 246
<210> 40
   <211> 44
   <212> DNA
   <213> Homo sapiens
<400> 40
   gaacagatca ggactcagga tgcaagaccc tggtcatctc caag 44
<210> 41
   <211> 146
   <212> DNA
   <213> Homo sapiens
<400> 41
   cctagcctca cagcctacat ctttctccca tgctggatgc ttcctgcctt tgaacatcgg 60
   actccaagtt cttcagtctg gggacttgga ctggcccttc ttgctcctca gcttgcagac 120
   agcctattgt gggacctcgt gatcat 146
<210> 42
   <211> 139
   <212> DNA
   <213> Homo sapiens
<400> 42
   aataggatgt gtggagacac tgggagaaca tggatctata agccatgttg ggaagtctgg 60
   aacagatccc tctctcacgg ccctcagaag gaaccaactc tgctgacttc ttgatgtcag 120
   acttctggcc tccagaacc 139
<210> 43
   <211> 126
   <212> DNA
   <213> Homo sapiens
<400> 43
   caagaattga ggttgctgca gacccataca gattcactgc tgttaactca gacaccttct 60
   acccctaaca ggtatggggt aggaccctct ctggaatcgg gctcttatgg cctacagtca 120
   aacaag 126
<210> 44
   <211> 95
   <212> DNA
   <213> Homo sapiens
<400> 44
   cagaaagaaa gcaactatct gcatgctagg aagtggtcct caccagaagc caactacttg 60
   ggcaccctga tctcggacat ccagcctcca gaact 95
<210> 45
   <211> 194
   <212> DNA
   <213> Homo sapiens
<400> 45
   cacaagggga agtcagggat tttaagcact aggcgatttt gacatgcaca catgaaaaga        60
   aatacagatg gcttctggga gcagtgagtg tctccacctg acagccagca aggaacttag       120
   acatacacct gcaaggaact ggattctgtc aacgtgaatg agcatggaag gctattcttc       180
   ccctgagcct ccag                                                         194
<210> 46
   <211> 57
   <212> DNA
   <213> Homo sapiens
<400> 46
   atgcaatcat acccatggct ttggccccaa gcccaagaga agatccagga atcccag 57
<210> 47
   <211> 170
   <212> DNA
   <213> Homo sapiens
<400> 47
   gaatggaatt atcgaatgca atcgaataga atcatcgaat ggactcgagt ggaatcatcg        60
   aatggaatgg aatggaatag tcaatgaact cgaatggaat catcattgaa tggaatcgaa       120
   tggaatcatt gagtggaatc gaatggaatc acgatcaaat ggaatcgaat                  170
<210> 48
   <211> 275
   <212> DNA
   <213> Homo sapiens
<400> 48
   cttggaaagg tttcttagct gctcttaagg aggcacagga aaagatggtt cctttctttc        60
   tttgctgttg ttgcccctgg ataaggagcc tgaagatgtg tgtggctgcc ttgtggctat       120
   gacggctgtg gctgtaaagg ctctgtactg aaggcatggc cacctcactg agtatgacag       180
   cagggagatg ggaagagcca gatttctcat gcggatatga agacattaac tgagccctgg       240
   atcctactct gcctttgaac ttcatgttgt atgag                                  275
<210> 49
   <211> 198
   <212> DNA
   <213> Homo sapiens
<400> 49
   gatcaggaga ccagggaatg tgagcggcct ctagaagctg ggaacagccc tcaggtgaca 60
   gtcagcaaga aaacaaggac ctcagtcctc caactgcaag taactgaatt cagccaacga 120
   cacaatgagc aggaaacgga tcctcctcta gagcatctgg aaacagacgc agccctgctg 180
   accctttgat ttctgctg 198
<210> 50
   <211> 36
   <212> DNA
   <213> Homo sapiens
<400> 50
   agcagctgga ggacagagga gcttccagca gaagag 36
<210> 51
   <211> 243
   <212> DNA
   <213> Homo sapiens
<400> 51
   cccttccact gtgtggaagc tttactttca cttttgcttt actttggctt taataaatct 60
   tgacccagca ctctctttag gtctgcatgc ttctcaaatt gagctgtaac actcactgct 120
   gcggtccaag ccttcattct ttgaagcccg tggaaccaca aactcttcaa ttgataaaaa 180
   ccttcgatcg gaagagactt ctcatctcat ttctgggggc ctgtccggga tttctctgaa 240
   gtg 243
<210> 52
   <211> 95
   <212> DNA
   <213> Homo sapiens
<400> 52
   ctggatttgt gactgctggg ctttgaagag ccaggagtga cttctaatct ggatatctac 60
   agagcttcta gactgagacc attcatagta gcctt 95
<210> 53
   <211> 133
   <212> DNA
   <213> Homo sapiens
<400> 53
   atctgggtgt ctgttattac cataattcaa atgagaggaa gatctgaaca agaatgagag 60
   tgaagcaaga gcaaaaggca ttgcagagga tgaggaaaag aagagttcac agactctgaa 120
   atgttgatcc agg 133
<210> 54
   <211> 79
   <212> DNA
   <213> Homo sapiens
<400> 54
   agaagacagc catctgcaag ccaagaagag agccctcacc agaacataat catgcttttg 60
   ttatggcagc caaagctgg 79
<210> 55
   <211> 177
   <212> DNA
   <213> Homo sapiens
<400> 55
   aagagcgcgt agatgccaca aagtaggtgt gtcacggaag aaagtgctta gggaagattc 60
   ttgcaggaga tctaattatc gcttttcgtc acttcttggg accctggcgt tccctgctct 120
   ccacttgcgg aagatggtta tagtagtgac agagctggca caatgctttc acctccg 177
<210> 56
   <211> 888
   <212> DNA
   <213> Homo sapiens
<400> 56
   gttcaaggca tcacagaggg acctgatgtc atcacgagag agtttcgtga gggtgaagct 60
   caggtatttc aagtctggct gataaaagag cacctcaaag agcagaacac tctggccaga 120
   aaaggaaacg ttatttattc tgcaaaaaca aagaaaattt tggatgagtt tcatctaatg 180
   ggagcacagc ctgcacacct gtgtttgtct ctcatcatcc atcatgaact cattctctga 240
   tttcacagcc caatgacaca gctcagtagt tctcaacttg agagattttg cccccacaag 300
   ggacagctgg cagtgtctgg acactatttt ggttatctgg acaaggggat gctattagga 360
   tctagagggt agagaccagg gatgcatctt aacaccctac aacgcacaga acaaccccta 420
   tgatgaagaa tgatctgcat gagatgtctg taagtgctga ggttgagcaa ctgctctgga 480
   tgttaattct ttgcttgtgc agttagcctc gactgaaagc tagaggtgag atgaataggt 540
   catctccgtg acatactcga gtcgaagtcg attctcaact cacccaagct tctgaaggcg 600
   acagctggga tgcctcagct ggttacacca ggtcacaaag gtcgactcgc tgagggtgct 660
   gtcctgcacc tggagctctc tgaggtgccc gctggtggtg agcacagagc agatgtgatg 720
   ccaacagatg aggctgtaat ccgacctgtg gtcaagaaag gacgagcttt ccacaggcag 780
   gcggtcaaca cgagccactt gtaacacata tacgcactac gctgtcttct caaacagaag 840
   atgtctgagg cagagcctcc ctcagtcaga aggaacggga cccttcag 888
<210> 57
   <211> 110
   <212> DNA
   <213> Homo sapiens
<400> 57
   gagcatctga agagtttaag accttgggga aagagagtct tgctgcttgc ttgtgggact 60
   tgccttggaa ggtagataag ttcactgcat cctcccactt tttatttctg 110
<210> 58
   <211> 103
   <212> DNA
   <213> Homo sapiens
<400> 58
   cgaagatctc ccaatcgaca cgaaccaagg aattcaaact ctcctcaggg gcaggatacg 60
   tctccaggct taacttgctc agcccactgg tgtggcgcag cag 103
<210> 59
   <211> 33
   <212> DNA
   <213> Homo sapiens
<400> 59
   caagcctgga gttcctgctt ggctcttcct gag 33
<210> 60
   <211> 63
   <212> DNA
   <213> Homo sapiens
<400> 60
   ctggccacca aagtgtatga tgagaaattt gctgataatt ttattgagga tcccttgtat 60
   atg 63
<210> 61
   <211> 81
   <212> DNA
   <213> Homo sapiens
<400> 61
   cttccaccat gggatgatcc atcaggaggg tcctcacgag atgaagtcct ctcaaccttg 60
   ggcttcctag cttccaaaac t 81
<210> 62
   <211> 127
   <212> DNA
   <213> Homo sapiens
<400> 62
   agatcctctt gggctgcctg acttccctca gtgtacacat cagctcagcc cgaagtgggg 60
   cgaagatctc ccaatcgaca cgaaccaagg aattcaaact ctcctcaggg gcaggatacg 120
   tctccag 127
<210> 63
   <211> 55
   <212> DNA
   <213> Homo sapiens
<400> 63
   cactcattcc tggagctact gcttggttcc ctgagaggtc ccagaactct gcaaa 55
<210> 64
   <211> 57
   <212> DNA
   <213> Homo sapiens
<400> 64
   agcactcatt cctggagcta ctgcttggtt ccctgagagg tcccagaact ctgcaaa 57
<210> 65
   <211> 126
   <212> DNA
   <213> Homo sapiens
<400> 65
   tagctttgca actggctttg gggacttccg aaagctacca gcactgcact gtgagactct        60
   catccctgag ctgaatccat ctgattcgac agcaagcttt ggtgagaaca tagatatatt       120
   tctgag                                                                  126
<210> 66
   <211> 79
   <212> DNA
   <213> Homo sapiens
<400> 66
   caggagtggg gtcagcagga ggaactctac agctatggga gaacctgcgt tcacctcttt        60
   tccaagcctg cctgttctg                                                     79
<210> 67
   <211> 191
   <212> DNA
   <213> Homo sapiens
<400> 67
   gattcttata aaaggacata gaagagacaa gctcagtttt ctccaaagga gaaggagcac        60
   acttagaggg agctgtattt tggtgacctc taaaagtcag tactgcggtg aatgagctcc       120
   agaacttgag gagtacttaa cagaattatt tctggaagaa tcactgtggg aaccattcaa       180
   agaacccagc g                                                            191
<210> 68
   <211> 78
   <212> DNA
   <213> Homo sapiens
<400> 68
   acccacagca ctcattcctg gagctactgc ttggttccct gagaggtccc agaactctgc        60
   aaagtgagtc cagcgctg                                                      78
<210> 69
   <211> 415
   <212> DNA
   <213> Homo sapiens
<400> 69
   aaacatgaat tctggaatct cgcaagactt ccagatggaa attacctgcc ccatctgcat 60
   gaattacttc atagacccag tcaccataga ctgtgggcac agcttttgca ggccctgttt 120
   ctacttcaac tggcaagaca tcccaattct tactcagtgc tttgaatgca tgaagacaac 180
   atggcagaga aacctgaaaa ctaacattca tttgaagcag atggcttccc ttgccagaaa 240
   agccagtctc tggctattcc tgagctctga ggagcaaatg tgtggcactc acagggagac 300
   aaagaagata ttctgtgaag tggacaggag cctgctctgt ttgctgtgct ccagctctca 360
   ggagcaccgg tatcacagac accgtcccat tgagtgggct gctgaggaac accgg 415
<210> 70
   <211> 58
   <212> DNA
   <213> Homo sapiens
<400> 70
   gaactactgc ttgattctct gagagatccc agcaccctac aaactgagtc cagatctg 58
<210> 71
   <211> 35
   <212> DNA
   <213> Homo sapiens
<400> 71
   agctggaggc caggggagaa actccagaag gagag 35
<210> 72
   <211> 582
   <212> DNA
   <213> Homo sapiens
<400> 72
   gaggtggaaa cttcaagtgc tggatttaca ggatgtctgt gagaacttct ggatggtttg 60
   gtctgaagct atggcccgtg ggtgcttcct caatgccaag aggaacaaaa aaccagtgca 120
   ggactgtcca aggatgagag gacggcagcc cttgactgtg ttcgtagaac tttggctcaa 180
   gaacaggact ctggatgaac acctcacctg cctccttcta tgggtcaagc agaggaaaga 240
   tttactacac ctgtgctgta agaagctgaa aattttggga atgcccttcc gcaatatcag 300
   aagcatcctg aaaatggtga acctagactg tatccaggag gtggaagtga attgcaagtg 360
   ggtactgccc atcctgacac agtttacccc atacctgggc cacatgagga atcttcagaa 420
   gctcgttctc tcccacatgg atgtctctcg ctacgtttcc ccagagcaga agaaggagat 480
   tgttacccag ttcaccactc agttcctcaa gctgcactgc ctccaaaagc tttatatgaa 540
   ctctgtttct ttcctcgaag gccacctgga ccagctgctc ag 582
<210> 73
   <211> 58
   <212> DNA
   <213> Homo sapiens
<400> 73
   gaactactgc ttgattctct gagagatccc agcaccctac aaactgagtc cagatctg 58
<210> 74
   <211> 62
   <212> DNA
   <213> Homo sapiens
<400> 74
   cctggagttc ctgcttggct cttcctgagg tctgagcacc ttctaaacta caaccagatc 60
   tg 62
<210> 75
   <211> 32
   <212> DNA
   <213> Homo sapiens
<400> 75
   ttcttacagg gttttggaga catattacac ag 32
<210> 76
   <211> 139
   <212> DNA
   <213> Homo sapiens
<400> 76
   gccgtggtgg ctggttccct gtctcttcag tagagagtct agaccccacc cagtcttcat 60
   gtacggccga ccgcaggctg agatggaaca ggaggctggg gagctgagcc ggtggcaggc 120
   ggcgcaccag gctgcccag 139
<210> 77
   <211> 231
   <212> DNA
   <213> Homo sapiens
<400> 77
   cacactctgc ctttggacgt gagagagagc gcacctttca cttgagcttc aacatgggaa 60
   agggaaatga agactccgat ctccactgct cctccatcca gtgctccact gaccagcccc 120
   ctttccaaca gatctccttt acagaaaagg gctcagatga gaagaaacca ttcaaagaaa 180
   aaggcaagac cgccttctcc cattccagtg agaagcacat acaaaggcaa g 231
<210> 78
   <211> 124
   <212> DNA
   <213> Homo sapiens
<400> 78
   aacaaagagc acgcggcgct ggccgccggc actcgcgccc tgaggctgcg gccccggagc 60
   gcccggcggc ggtttcggcg cgcggccggg ctggcgatgg aagatggaag gaaggagcgc 120
   agcg 124
<210> 79
   <211> 179
   <212> DNA
   <213> Homo sapiens
<400> 79
   gtttgccgct ttccgagcgc caaggagcgg agcgcgctca gctgcgccca gagccttcgg 60
   ccggacctga aaaagcgaga gggagagcga gcaaaaggcg cgatccagag agccaggcaa 120
   gcggggcgcc gacctcgggc ggccccggcc cgcgcccttg cccctcggcc gggcggacg 179
<210> 80
   <211> 119
   <212> DNA
   <213> Homo sapiens
<400> 80
   catactccat acctgggatt tccgcctcgc cgctctccga ctgcttccag acatgcaggg 60
   gccctgggtg ctgctcctgc tgggcctgag gctacagctc tccctgggca tcatcccag 119
<210> 81
   <211> 129
   <212> DNA
   <213> Homo sapiens
<400> 81
   ctctctctgc agggctggcc cctggcaagg cccgggacag gaaggcctac acggtcctcc 60
   tatacggaaa cggtccaggc tatgtgctca aggacggcgc ccggccggat gttaccgaga 120
   gcgagagcg 129
<210> 82
   <211> 726
   <212> DNA
   <213> Homo sapiens
<400> 82
   cctaggtgat actatcaggc tttggactgg gggaggtggt caatgatata aaagaagctg 60
   ccctccctgc cttcaagtta cttataacag agtcataaac agaggcaagg ctgtcacact 120
   gatttttgtc aacttttata aaatgcctta ataaattcta agcacgataa taattgcttt 180
   aaatggatta tgtcatttaa tctttgcaat aattctacaa agcagaaact tttattatcc 240
   tcattttaca gataaggaag ctgaggttta gaaagaaggc aacttcccaa atatcatgca 300
   gctggtaaat gatcaagcca ggacctgatc tgagtcaatt tgattctggt ttctatgctc 360
   ttaatcccaa ttttatacta taacacccca ctttcaatac tagacagatc aacgaggcag 420
   aaggttaaca aggatatcca gctagctttt gataagctat aaactagttc ctataattaa 480
   atgtggtgga ggacaggaag agggagtgac caagttaaaa aagaagagtt cagtgaaggc 540
   tggagtgacc agggatgggc tcatgaggag atgaggttta aaatgagtca tagagcagta 600
   agcttgtaat ataacttaaa taccatctaa tttgttcact gacctttaca gatatggaaa 660
   ctgagccctc acacatggac tgaattctga caagcagagg ataaaggcag aacaacatga 720
   ctgaag 726
<210> 83
   <211> 208
   <212> DNA
   <213> Homo sapiens
<400> 83
   aattattaaa tgcccgtgtt ccctgtaagg cagcatttaa tcatgaggca ctccaaagaa 60
   tatgagatag aaggggaggg gtaggaggag agggaggagg gtaggaagac agtttgcatt 120
   cttgcaacat taaaccaaag ggacttggag tgcagatggc atccttcggt tcttccagac 180
   aagctgcaag acgctgacca tggccaag 208
<210> 84
   <211> 107
   <212> DNA
   <213> Homo sapiens
<400> 84
   tgcagggatt gcaaggacac ctgctgtatt taaaatgctg cattttcttg gcataccatt 60
   taagacaaat tacaaaaacc tataactgaa gcctgttttt ccaaaag 107
<210> 85
   <211> 334
   <212> DNA
   <213> Homo sapiens
<400> 85
   ctgataagtt tgtcttttct ctggattttt cttgcagatt tatcaggatg agcttccagg      60
   ccccacgcag actcctggag ctggcagggc agagcctgct gagggaccag gccttggcca     120
   tctccgtcct ggatgagctg cccagggagc tcttcccccg actgttcgtg gaggccttca     180
   ctagcagacg ctgcgaggtt ctgaaggtga tggtgcaggc ctggcccttc ccctgcctcc     240
   ctctggggtc cctgatgaag acgcctgatc tggagatctt acattatgta gtggatggga     300
   ttgattgcct gcttgcccaa aaggttcgcc ccag                                 334

## Description

### BACKGROUND OF THE INVENTION

### 1. Field of the Invention

The present disclosure relates generally to medicine, diagnostic and therapeutic methods. The invention is a method of determining the presence of, or risk of developing, facioscapulohumeral dystrophy (FSHD) in a patient.

### 2. Description of Related Art

Facioscapulohumeral muscular dystrophy (FSHD) is a progressive neuromuscular disorder caused by contractions of repetitive elements within the macrosatellite D4Z4 on chromosome 4q35. Currently, the diagnostic test for FSHD1 requires pulse-field gel electrophoresis and Southern blotting to detect the contraction of the D4Z4 repeats, and there are no commercially available diagnostic tests for FSHD2. FSHD1 can be genetically diagnosed by the contraction of the number of D4Z4 repeats to 10 or fewer repeats on a specific 4qA haplotype. FSHD2 is associated with decreased DNA methylation of the D4Z4 repeats on the same 4qA haplotype. Clinical trials have been discouraged by the fact that FSHD is a highly variable and slowly progressing disease whereas the efficacy of therapeutic interventions is ideally established over short periods of time.

Therefore, molecular biomarkers of FSHD are needed for determining the presence or risk of developing FSHD and would greatly facilitate FSHD therapeutic development and clinical research.

WO 2013/019623 (HUTCHINSON FRED CANCER RES [US], et al) published 7 February 2013 discloses methods for increasing, decreasing or maintaining the innate immune response in a mammalian subject comprising modulating the expression of DUX4-fl, or modulating the expression of beta-defensin 3 (DEFB103).

US 2013/347136 (EMERSON JR CHARLES P [US], et al) published 26 December 2013 discloses compositions and methods for identifying new treatments for Facioscapulohumeral muscular dystrophy (FSHD), and uses thereof.

US 2013/288976 (VAN DER MAAREL SILVERE M [NL], et al) published 31 October 2013 discloses a method of screening a human subject to determine if said subject has a genetic predisposition to develop, or is suffering from Facioscapulohumeral dystrophy (FSHD).

Geng et al., Dev Cell, vol. 22, no. 1, 2012, page 38051 identifies genes associated with germline and early stem cell development as targets of the DUX4 transcription factor, a leading candidate gene for FSHD.

WO 2013/033627 (UNIV CALIFORNIA [US], et al) published 7 March 2013 discloses methods, compositions and kits for evaluating a diagnosis, prognosis, or response to treatment of a subject with a disorder such as rheumatoid arthritis or osteoarthritis.

Yoshiaki Chinen et al., Genes Chromosomes & Cancer, vol. 53, no. 4, (2014-01-21), pages 299-308 shows that the leucine twenty homeobox (LEUTX) gene is fused to KAT6A in therapy-related acute myeloid leukemia with t(8;19)(p11;q13).

Zizhen Yao et al., vol. 23, no. 20, 26 May 2014 (2014-05-26), pages 5342-5352 describes that DUX4 target gene expression is the major molecular signature in FSHD muscle.

### SUMMARY OF THE INVENTION

Aspects of the disclosure provide disease testing, risk prediction, prognostic and/or diagnostics methods based on the detection of biomarkers. Methods and compositions are based, in part, on the discovery that expression of certain biomarkers in patients or subjects can determine the presence or risk of developing DUX-4-related diseases such as muscular dystrophy, or particularly Facioscapulohumeral dystrophy (FSHD). In particular aspects, one or more DUX-4 biomarkers that can be used may be one or more gene or exons listed in Table 2 or any novel exons listed in Table 1 or any genes or sequences listed in any tables herein or any DUX-4 target genes or sequences (e.g., U.S. Patent Application NO. 14/236,003, filed on July 27, 2012, international application NO. PCT/US12/48557, filed on July 27, 2012). The chromosome start and end positions may refer to human genome assembly hgl9 (corresponds to Genome Reference Consortium GRCh37). The Table 2 gene annotations are based on GENCODE version 19 (available at World Wide Web gencodegenes.org/releases/19.html).

**Table 1**

| seqnames | start | end | width | strand | gene.name | gene.id | known | SEQ ID NO. | SEQUENCE |
|---|---|---|---|---|---|---|---|---|---|
| chr5 | 178124587 | 178124747 | 161 | - | NA | Novel 17574 | FALSE | 1 | |
| chr2 | 96260002 | 96260182 | 181 | - | NA | Novel 13250 | FALSE | 2 | |
| chr6 | 27861221 | 27861759 | 539 | + | NA | Novel 17723 | FALSE | 3 | |
| chr16 | 75703261 | 75703382 | 122 | + | NA | Novel 7492 | FALSE | 4 | |
| chr4 | 25649655 | 25649978 | 324 | + | SLC34A2 | ENSG000001 57765.7 | FALSE | 5 | |
| chr11 | 89773055 | 89773130 | 76 | - | NA | Novel 4275 | FALSE | 6 | |
| chr19 | 40269508 | 40269567 | 60 | + | LEUTX | ENSG000002 13921.6 | FALSE | 7 | |
| chr19 | 58181877 | 58181935 | 59 | + | ZSCAN4 | ENSG000001 80532.6 | FALSE | 8 | |
| chr11 | 71537346 | 71537638 | 293 | - | CTD-2313N18.5 | ENSG000002 48671.3 | FALSE | 9 | |
| chr5 | 17626085 | 17626158 | 74 | - | NA | Novel 17135 | FALSE | 10 | |
| chr8 | 117688622 | 117688686 | 65 | - | EIF3H | ENSG000001 47677.6 | FALSE | 11 | |
| chr10 | 93539029 | 93539135 | 107 | + | NA | Novel 2474 | FALSE | 12 | |
| chr11 | 108537249 | 108537424 | 176 | - | NA | Novel 4328 | FALSE | 13 | |
| chr8 | 211074 | 211155 | 82 | + | NA | Novel 19631 | FALSE | 14 | |
| chr12 | 108274461 | 108274500 | 40 | + | NA | Novel 4789 | FALSE | 15 | TGGTGGAAGAGGATGGCGTACAAAGATTTCAGCGGAAGAG |
| chr11 | 89540267 | 89540402 | 136 | - | TRIM49 | ENSG000001 68930.9 | FALSE | 16 | |
| chr16 | 9068951 | 9069203 | 253 | - | USP7 | ENSG000001 87555.10 | FALSE | 17 | |
| chr3 | 72831574 | 72831643 | 70 | - | SHQ1 | ENSG000001 44736.9 | FALSE | 18 | |
| chr1 | 13167116 | 13167177 | 62 | - | HNRNPCL 1 | ENSG000001 79172.7 | FALSE | 19 | |
| chr1 | 13194534 | 13194595 | 62 | + | PRAMEF1 | ENSG000001 16721.9 | FALSE | 20 | |
| chr5 | 17626249 | 17626359 | 111 | - | NA | Novel 17138 | FALSE | 21 | |
| chr10 | 76900361 | 76900435 | 75 | + | SAMD8 | ENSG000001 56671.8 | FALSE | 22 | |
| chr11 | 89765617 | 89765750 | 134 | + | TRIM49C | ENSG000002 04449.2 | FALSE | 23 | |
| chr22 | 32590332 | 32590376 | 45 | - | RFPL2 | ENSG000001 28253.9 | FALSE | 24 | CCCAGTGGAAGCAGCTGGAGGACAGAGGAGCTTCCAGCAGAAGAG |
| chr20 | 52214170 | 52214227 | 58 | - | ZNF217 | ENSG000001 71940.9 | FALSE | 25 | |
| chr1 | 42915207 | 42915406 | 200 | + | PPCS | ENSG000001 27125.7 | FALSE | 26 | |
| chr5 | 17512525 | 17512600 | 26 | + | NA | Novel 16621 | FALSE | 27 | CCAGCTTACTGATCAGTGGGTCTGAG |
| chr11 | 48965037 | 48965173 | 137 | + | TRIM51CP | ENSG000002 49910.2 | FALSE | 28 | |
| chr11 | 49862897 | 49863032 | 136 | - | TRIM51FP | ENSG000002 19061.4 | FALSE | 29 | |
| chr9 | 91921432 | 91921534 | 103 | + | CKS2 | ENSG000001 23975.4 | FALSE | 30 | |
| chr1 | 12833906 | 12833959 | 54 | + | PRAMEF1 | ENSG000001 16721.9 | FALSE | 31 | |
| chr19 | 48307772 | 48307815 | 44 | - | TPRX1 | ENSG000001 78928.4 | FALSE | 32 | GAACAGATCAGGACTCAGGATGCAAGACCCTGGTCATCTCCAAG |
| chr5 | 78112492 | 78112713 | 222 | + | NA | Novel 16743 | FALSE | 33 | |
| chr8 | 7225284 | 7225573 | 290 | - | FAM66B | ENSG000002 15374.5 | FALSE | 34 | |
| chr11 | 94752052 | 94752121 | 70 | + | NA | Novel 3658 | FALSE | 35 | |
| chr3 | 140476420 | 140476559 | 140 | - | NA | Novel 15611 | FALSE | 36 | |
| chr5 | 17492055 | 17492165 | 111 | + | NA | Novel 16611 | FALSE | 37 | |
| chr19 | 11805614 | 11805760 | 147 | - | NA | Novel 11289 | FALSE | 38 | |
| chr10 | 3985161 | 3985406 | 246 | + | NA | Novel 2170 | FALSE | 39 | |
| chr19 | 48362485 | 48362528 | 44 | + | TPRX2P | ENSG000002 59009.3 | FALSE | 40 | GAACAGATCAGGACTCAGGATGCAAGACCCTGGTCATCTCCAAG |
| chr17 | 34443465 | 34443610 | 146 | + | CTB-91J4.1 | ENSG000002 67118.1 | FALSE | 41 | |
| chr1 | 99139865 | 99140003 | 139 | - | NA | Novel 1736 | FALSE | 42 | |
| chr8 | 27170348 | 27170473 | 126 | - | TRIM35 | ENSG000001 04228.8 | FALSE | 43 | |
| chr3 | 98695309 | 98695403 | 95 | - | NA | Novel 15513 | FALSE | 44 | |
| chr10 | 65773722 | 65773915 | 194 | - | NA | Novel 2824 | FALSE | 45 | |
| chr11 | 44494682 | 44494738 | 57 | + | NA | Novel 3350 | FALSE | 46 | |
| chr10 | 42383521 | 42383690 | 170 | + | NA | Novel 2314 | FALSE | 47 | |
| chr5 | 157705581 | 157705855 | 275 | + | NA | Novel 16974 | FALSE | 48 | |
| chr5 | 16814012 | 16814209 | 198 | + | NA | Novel 16593 | FALSE | 49 | |
| chr22 | 29833684 | 29833719 | 36 | + | RFPL1 | ENSG000001 28250.5 | FALSE | 50 | AGCAGCTGGAGGACAGAGGAGCTTCCAGCAGAAGAG |
| chr6 | 115319341 | 115319583 | 243 | + | NA | Novel 18012 | FALSE | 51 | |
| chr11 | 89798507 | 89798601 | 95 | - | TRIM64B | ENSG000001 89253.7 | FALSE | 52 | |
| chr1 | 71479998 | 71480130 | 133 | - | PTGER3 | ENSG000000 50628.16 | FALSE | 53 | |
| chr16 | 46863111 | 46863189 | 79 | + | NA | Novel 7399 | FALSE | 54 | |
| chr6 | 130088273 | 130088449 | 177 | - | ARHGAP1 8 | ENSG000001 46376.6 | FALSE | 55 | |
| chr19 | 56372609 | 56373496 | 888 | - | NA | Novel 12124 | FALSE | 56 | |
| chr12 | 8320120 | 8320229 | 110 | + | ZNF705A | ENSG000001 96946.5 | FALSE | 57 | |
| chr1 | 13379280 | 13379382 | 103 | + | NA | Novel 256 | FALSE | 58 | |
| chr1 | 13184298 | 13184330 | 33 | - | HNRNPCL 1 | ENSG000001 79172.7 | FALSE | 59 | CAAGCCTGGAGTTCCTGCTTGGCTCTTCCTGAG |
| chr5 | 63434738 | 63434800 | 63 | - | RP11-158J3.2 | ENSG000002 48285.1 | FALSE | 60 | |
| chr5 | 83621975 | 83622055 | 81 | + | NA | Novel 16768 | FALSE | 61 | |
| chr1 | 12987881 | 12988007 | 127 | - | NA | Novel 1250 | FALSE | 62 | |
| chr1 | 13673449 | 13673503 | 55 | - | HNRNPCL 1 | ENSG000001 79172.7 | FALSE | 63 | |
| chr1 | 13452594 | 13452650 | 57 | - | HNRNPCL 1 | ENSG000001 79172.7 | FALSE | 64 | |

**Table 2**

| seqnames | start | end | width | strand | gene.name | gene.id | known | SEQ ID NO. | SEQUENCE |
|---|---|---|---|---|---|---|---|---|---|
| chr2 | 96150354 | 96150479 | 126 | - | TRIM43B | ENSG00000144010.7 | TRUE | 65 | |
| chr19 | 7021364 | 7021442 | 79 | - | CTB-25J19.1 | ENSG00000196589.4 | TRUE | 66 | |
| chr11 | 89575165 | 89575355 | 191 | + | TRIM53BP | ENSG00000166013.11 | TRUE | 67 | |
| chr1 | 13673434 | 13673511 | 78 | - | PRAMEF1 4 | ENSG00000204481.6 | TRUE | 68 | |
| chr11 | 89732495 | 89732909 | 415 | - | TRIM53AP | ENSG00000225581.3 | TRUE | 69 | |
| chr1 | 13611493 | 13611550 | 58 | - | XX-FW84067D 5.1 | ENSG00000204485.2 | TRUE | 70 | |
| chr19 | 56280507 | 56280541 | 35 | + | RFPL4AL1 | ENSG00000229292.1 | TRUE | 71 | AGCTGGAGGCCAGGGGAGAAACTCCAGAAGGAGAG |
| chr1 | 13219000 | 13219581 | 582 | - | PRAMEF2 6 | ENSG00000229571.2 | TRUE | 72 | |
| chr1 | 13390708 | 13390765 | 58 | - | PRAMEF8 | ENSG00000182330.6 | TRUE | 73 | |
| chr1 | 13184265 | 13184326 | 62 | - | HNRNPCP 5 | ENSG00000179412.8 | TRUE | 74 | |
| chr11 | 55072505 | 55072536 | 32 | - | RP11-72M10.5 | ENSG00000254828.1 | TRUE | 75 | TTCTTACAGGGTTTTGGAGACATATTACACAG |
| chr1 | 44584522 | 44584660 | 139 | + | KLF17 | ENSG00000171872.4 | TRUE | 76 | |
| chr4 | 190948182 | 190948412 | 231 | - | FRG2 | ENSG00000205097.2 | TRUE | 77 | |
| chr8 | 18871073 | 18871196 | 124 | - | PSD3 | ENSG00000156011.12 | TRUE | 78 | |
| chr14 | 64319683 | 64319861 | 179 | + | SYNE2 | ENSG00000054654.11 | TRUE | 79 | |
| chr2 | 233271553 | 233271671 | 119 | + | ALPPL2 | ENSG00000163286.3 | TRUE | 80 | |
| chr2 | 233245949 | 233246077 | 129 | + | ALPP | ENSG00000163283.6 | TRUE | 81 | |
| chr9 | 84887671 | 84888396 | 726 | + | RP11-15B24.5 | ENSG00000228430.4 | TRUE | 82 | |
| chr12 | 13248533 | 13248740 | 208 | - | GSG1 | ENSG00000111305.14 | TRUE | 83 | |
| chr1 | 171833327 | 171833433 | 107 | + | DNM3-IT1 | ENSG00000233540.1 | TRUE | 84 | |
| chr1 | 13698072 | 13698405 | 334 | - | PRAMEF1 9 | ENSG00000204480.6 | TRUE | 85 | |

The invention comprises a method of determining the presence of, or risk of developing, facioscapulohumeral dystrophy (FSHD) in a patient, comprising:
a) measuring in a sample obtained from a patient increased expression of at least one FSHD biomarker relative to an expression level in a normal reference standard or normal control sample, wherein the FSHD biomarker comprises LEUTX; and
b) identifying the patient as having a risk of developing FSHD.

Thus, the disclosure provides a method of determining the presence of or risk of developing facioscapulohumeral dystrophy (FSHD) in a patient. The disclosed method may comprise measuring in a sample obtained from a patient increased expression of at least one FSHD biomarker as relative to a normal reference standard or normal control sample. In particular aspects of the disclosure, the FSHD biomarker may comprise a gene listed in Table 2 or any exons in Table 2 (SEQ ID NOs: 65-85). In alternative aspects of the disclosure, the FSHD biomarker may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, or 64 novel exons (or any range derivable therein) or at least that number of novel exons (or any range derivable therein) listed in Table 1 (SEQ ID NO:1-64). In further aspects, the FSHD biomarker may comprise additional biomarkers not listed in Table 1 or Table 2 or may comprise two or more biomarkers in Table 1 and/or Table 2.

In additional aspects, the method may comprise identifying the patient as having a risk of developing FSHD. The method may further comprise monitoring the patient or recommending, prescribing, determining or administering a FSHD treatment for the subject based on the risk.

The method may still further comprise calculating a risk score for the patient based on the expression levels. In further aspects, the method may further comprise monitoring the patient for FSHD for the expression level of one or more FSHD biomarkers or additional monitoring methods such as observation or testing of muscle biopsy in a patient.

In other aspects, the method may comprise determining that a sample in the patient does not have increased or decreased expression levels or has increased or decreased expression levels in one or more FSHD biomarkers. Because the expression level is correlated with the risk, response or prognosis related with FSHD, the method may further comprise identifying the patient for the risk, response to treatment or prognosis related with FSHD. For example, the method may comprise identifying the patient not having the increased expression as likely being at low risk for FSHD, responsive to a treatment or having good prognosis; and identifying the patient having the increased expression as likely being at high risk for FSHD, having poor response to a treatment or having poor prognosis. In other embodiments, for different FSHD biomarkers, the method may comprise identifying the patient having the increased expression as likely being at low risk for FSHD, responsive to a treatment or having good prognosis; and identifying the patient not having the increased expression as likely being at high risk for FSHD, having poor response to a treatment or having poor prognosis

In particular aspects, the method may comprise determining or monitoring the expression level of one or more FSHD biomarkers described herein in a patient that has been administered a FSHD treatment or intervention, including any known or candidate FSHD treatment or intervention. In other aspects, the method may further comprise administering or refraining from administering a treatment or intervention to a patient that has been determined to be responsive or resistant to the treatment or intervention. The candidate therapy or intervention may be selected based on the one or more FSHD biomarkers for further testing or characterization, such as clinical trials.

In certain aspects, the method may be defined as treating a patient patient that is determined to have, suspected of having, or at risk of developing FSHD. In other aspects, the method may comprise evaluating or monitoring a measured expression level of one or more FSHD biomarkers listed in Table 1 or Table 2 in a patient as compared to a control sample or a reference level. The evaluating or monitoring may be performed on a patient that has been previously administered a FSHD treatment, a test drug, or a candidate treatment.

In other aspects, the method may further comprise administering the FSHD treatment to the patient having a measured expression level indicating a response to the FSHD treatment; or alternatively, administering a different FSHD treatment to the patient having a measured expression level indicating a non-response or resistance to the FSHD treatment.

For example, the increased or non-decreased measured expression level as compared to a normal control, a normal reference standard level or a previously measured expression level in the patient indicates non-response or resistance. In additional aspects, the decreased or non-increased measured expression level as compared to a normal control, a normal reference standard level or a previously measured expression level in the patient indicates response to the treatment.

In other aspects, depending on the FSHD markers used, the decreased or non-increased measured expression level as compared to a normal control, a normal reference standard level or a previously measured expression level in the patient indicates non-response or resistance. In additional aspects, the increased or non-decreased measured expression level as compared to a normal control, a normal reference standard level or a previously measured expression level in the patient indicates response to the treatment.

In other aspects, the method may further comprise administering the FSHD treatment that has been determined to be effective based on the measured expression level as compared to a control sample or a reference level to a different patient. For example, the increased, non-decreased or decreased expression level as compared to a normal control, a normal reference standard level or a previously measured expression level in the patient indicates the treatment not likely being effective. In other aspects, the increased, non-decreased or decreased measured expression level as compared to a normal control, a normal reference standard level or a previously measured expression level in the patient indicates the treatment likely being effective or efficacy.

In further aspects, the method may comprise determining the expression level of one or more FSHD biomarkers. In still further aspects, the method may comprise obtaining a sample of the subject or patient or obtaining a sample from the subject or patient. Non-limiting examples of the sample include a tissue sample, a blood sample, a urine sample, a saliva sample, a serum sample, a plasma sample, a tear sample, a hair follicle sample, a fetus sample, or a fecal sample.

In particular embodiments, the sample is a tissue sample such as a muscle sample. In further embodiments, the sample may be a blood sample or serum sample.

The term subject or patient may refer to an animal (for example a mammal), including but not limited to humans, non-human primates, rodents, dogs, or pigs.

The methods of obtaining provided in the disclosure include methods of biopsy such as fine needle aspiration, core needle biopsy, vacuum assisted biopsy, incisional biopsy, excisional biopsy, punch biopsy, shave biopsy or skin biopsy. In certain aspects the sample is obtained from a biopsy from muscle tissue by any of the biopsy methods previously mentioned or known to a person skilled in the art. In other aspects the sample may be obtained from any of the tissues provided herein that include but are not limited to gall bladder, skin, heart, lung, breast, pancreas, liver, muscle, kidney, smooth muscle, bladder, intestine, brain, prostate, esophagus, or thyroid tissue.

In certain aspects, any medical professional such as a doctor, nurse or medical technician may obtain a biological sample for testing. In further aspects, the patient or subject may obtain a biological sample for testing without the assistance of a medical professional, such as obtaining a whole blood sample, a serum sample, a urine sample, a fecal sample, a buccal sample, or a saliva sample.

In further embodiments, the sample may be a fresh, frozen or preserved sample or a fine needle aspirate. In particular embodiments, the sample is a formalin-fixed, paraffin-embedded (FFPE) sample. An acquired sample may be placed in short term or long term storage by placing in a suitable medium, excipient, solution, or container. In certain cases storage may require keeping the sample in a refrigerated, or frozen environment. The sample may be quickly frozen prior to storage in a frozen environment. In certain instances the frozen sample may be contacted with a suitable cryopreservation medium or compound. Examples of cryopreservation mediums or compounds include but are not limited to: glycerol, ethylene glycol, sucrose, or glucose.

Some embodiments further involve isolating nucleic acids such as DNA, ribonucleic or RNA from a biological sample or in a sample of the patient. Certain embodiments may not involve isolating nucleic acids as the step is only optional. Other steps may or may not include amplifying a nucleic acid in a sample and/or hybridizing one or more probes to an amplified or non-amplified nucleic acid.

Disclosed are methods for detecting the expression level of one or more FSHD biomarkers by assaying the nucleic acids or proteins. Nucleic acid assay methods may include, but not be limited to, next generation sequencing, single-molecule real-time sequencing, mass spectrometry, Northern hybridization, single nucleotide primer extension

(SNuPE), quantitative PCR, digital PCR, ddPCR (digital droplet PCR), nCounter (nanoString), BEAMing (Beads, Emulsions, Amplifications, and Magnetics) (Inostics), ARMS (Amplification Refractory Mutation Systems), RNA-Seq, TAm-Seg (Tagged-Amplicon deep sequencing), PAP (Pyrophosphorolysis-activation polymerization, or a microarray-based expression profiling. Non-limiting protein assay methods comprise Western-blot, ELISA (enzyme-linked immunosorbent assay), RIA (radioimmunoassay), competitive EIA (competitive enzyme immunoassay), DAS-ELISA (double antibody sandwich ELISA), immunocytochemistry, mass spectrometry, protein microarrays or biochips.

The methods may further comprise assaying nucleic acids in a sample. In certain embodiments, a microarray may be used to measure or assay the level of FSHD biomarker expression in a sample. The methods may further comprise recording the FSHD biomarker expression level or risk in a tangible medium or reporting the expression level or risk to the patient, a health care payer, a physician, an insurance agent, or an electronic system.

In some aspects, methods will involve determining or calculating a risk or prognosis score based on data concerning the expression level of at least one FSHD biomarker, meaning that the expression level of the FSHD biomarker is at least one of the factors on which the score is based. A risk or prognosis score will provide information about the patient, such as the general probability whether the patient is likely to develop FSHD or is sensitive to a particular therapy. In certain embodiments, a risk or prognosis value is expressed as a numerical integer or number that represents a probability of 0% likelihood to 100% likelihood that a patient has a chance of developing FSHD, poor response to a treatment, or poor prognosis.

In some aspects, the risk or prognosis score is expressed as a number that represents a probability of 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% likelihood (or any range derivable therein) that a patient has a chance of developing FSHD, poor response to a treatment, or poor prognosis. Alternatively, the probability may be expressed generally in percentiles, quartiles, or deciles.

A difference between or among weighted coefficients or expression levels or between or among the weighted comparisons relative to a normal reference standard or normal control sample may be, be at least or be at most about 0.1, 0.2, 0.3, 0.4, 0.5, 0.6, 0.7, 0.8, 0.9, 1.1, 1.2, 1.3, 1.4, 1.5, 1.6, 1.7, 1.8, 1.9, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0. 19.5, 20.0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 410, 420, 425, 430, 440, 441, 450, 460, 470, 475, 480, 490, 500, 510, 520, 525, 530, 540, 550, 560, 570, 575, 580, 590, 600, 610, 620, 625, 630, 640, 650, 660, 670, 675, 680, 690, 700, 710, 720, 725, 730, 740, 750, 760, 770, 775, 780, 790, 800, 810, 820, 825, 830, 840, 850, 860, 870, 875, 880, 890, 900, 910, 920, 925, 930, 940, 950, 960, 970, 975, 980, 990, 1000 times or -fold (or any range derivable therein).

In some aspects, determination or calculation of a diagnostic, prognostic, or risk score is performed by applying classification algorithms based on the expression values of one or more FSHD biomarkers with differential expression p values of about, between about, or at most about 0.005, 0.006, 0.007, 0.008, 0.009, 0.01, 0.011, 0.012, 0.013, 0.014, 0.015, 0.016, 0.017, 0.018, 0.019, 0.020, 0.021, 0.022, 0.023, 0.024, 0.025, 0.026, 0.027, 0.028, 0.029, 0.03, 0.031, 0.032, 0.033, 0.034, 0.035, 0.036, 0.037, 0.038, 0.039, 0.040, 0.041, 0.042, 0.043, 0.044, 0.045, 0.046, 0.047, 0.048, 0.049, 0.050, 0.051, 0.052, 0.053, 0.054, 0.055, 0.056, 0.057, 0.058, 0.059, 0.060, 0.061, 0.062, 0.063, 0.064, 0.065, 0.066, 0.067, 0.068, 0.069, 0.070, 0.071, 0.072, 0.073, 0.074, 0.075, 0.076, 0.077, 0.078, 0.079, 0.080, 0.081, 0.082, 0.083, 0.084, 0.085, 0.086, 0.087, 0.088, 0.089, 0.090, 0.091, 0.092, 0.093, 0.094, 0.095, 0.096, 0.097, 0.098, 0.099, 0.1, 0.2, 0.3, 0.4, 0.5, 0.6, 0.7, 0.8, 0.9 or higher (or any range derivable therein). In certain aspects, the diagnostic, prognostic, or risk score is calculated using one or more statistically significantly differentially expressed biomarkers (either individually or as difference pairs), including expression levels of biomarkers in Tables 1 and/or Table 2.

Any of the methods described herein may be implemented on a tangible computer-readable medium comprising computer-readable code that, when executed by a computer, causes the computer to perform one or more operations. In some aspects, there is a tangible computer-readable medium comprising computer-readable code that, when executed by a computer, causes the computer to perform operations comprising receiving information corresponding to an expression level of at least one FSHD biomarker in a sample from a subject, wherein the FSHD biomarker comprises a gene listed in Table 2 and/or an exon listed in Table 1. The operations may further comprise determining a difference value in the expression level using the information corresponding to the expression level as compared with a reference standard or control sample. The operations may further comprise determining a risk of developing FSHD based on an increased expression determined by the difference value in the expression level

Additional aspects concern a method of detecting expression of a gene or exon listed in Table 1 or 2 comprising contacting a nucleic acid sample with cDNA or a complement thereof described herein under conditions to hybridize the DNA to a target nucleic acid molecule in the sample; and, detecting the target nucleic acid molecule. In some aspects, the nucleic acid sample comprises RNA transcripts, while in other aspects it alternatively or additional comprises cDNA of RNA transcripts. In some aspects, the probe comprises a label or other detectable or screenable moiety. In some cases, the label or moiety is enzymatic, colorimetric, radioactive, fluorescent, or luminescent.

In some aspects, receiving information comprises receiving from a tangible data storage device information corresponding to the expression levels from a tangible storage device. In additional aspects the medium further comprises computer-readable code that, when executed by a computer, causes the computer to perform one or more additional operations comprising: sending information corresponding to the difference value to a tangible data storage device, calculating a risk score for the patient, recommending, prescribing, determining a FSHD treatment for the subject based on the risk.

The tangible, computer-readable medium further comprise computer-readable code that, when executed by a computer, causes the computer to perform one or more additional operations comprising calculating a prognosis score for the patient. The operations may further comprise making recommendations comprising: administering a treatment comprising a FSHD treatment to a patient that is determined to have a decreased expression level.

Disclosed is a kit comprising a plurality of primers or probes specific for determining expression levels of one or more FSHD biomarkers comprising at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 (or any value or range derivable therein) genes or exons listed in in Table 1 or Table 2. A kit is also disclosed as comprising a plurality of antibodies specific for determining expression levels of one or more FSHD biomarkers comprising at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 (or any value or range derivable therein) gene or exons listed in in Table 1 or Table 2.

Some aspects concern an isolated nucleic acid comprising, consisting of, identical to, or complementary to at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 410, 420, 425, 430, 440, 441, 450, 460, 470, 475, 480, 490, 500, 510, 520, 525, 530, 540, 550, 560, 570, 575, 580, 590, 600, 610, 620, 625, 630, 640, 650, 660, 670, 675, 680, 690, 700, 710, 720, 725, 730, 740, 750, 760, 770, 775, 780, 790, 800, 810, 820, 825, 830, 840, 850, 860, 870, 875, 880, 890, 900, 910, 920, 925, 930, 940, 950, 960, 970, 975, 980, 990, 1000, or 2000 (or any range or value derivable therein) contiguous nucleotides of a first exon listed in Table 1 or Table 2 or any of SEQ ID NOs: 1-85. The isolated nucleic acid may be RNA or DNA. The isolated nucleic acid may comprise at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 410, 420, 425, 430, 440, 441, 450, 460, 470, 475, 480, 490, 500, or more contiguous nucleotides from at least one neighboring (immediate adjacent) exon but does not comprise one or more of the nucleotides of one or more intervening introns between the first exon and the neighboring exon. For example, a neighboring exon and intervening introns can be identified by comparing the first exon to genome sequences with annotated exons. Because of its lacking at least part of the intervening intron sequences, such an isolated nucleic acid that is double stranded and/or composed of DNA is nonnaturally occurring. In particular aspects, the isolated nucleic acid may be a cDNA molecule, which is not naturally occurring.

There is disclosed a method of amplifying a target DNA sequence. The method may comprise comprising providing a reaction mixture comprising a double-stranded target DNA, a pair of primers specific for determining expression levels of one or more FSHD biomarkers comprising at least one gene or exon listed in Table 1 or Table 2 and a polymerase; and amplifying the target nucleic acid. The amplification may involve the use of thermal cycling or not. At least or at most 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 thermal cycles may be used for amplifying the target nucleic acid.

One or more FSHD biomarkers described herein may include at least or at most 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 genes (or any value or range derivable therein) of genes or exons listed in Table 1 and/or Table 2 or any tables described herein. The biomarkers may comprise additional genes or exons or any sequences known in the art.

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

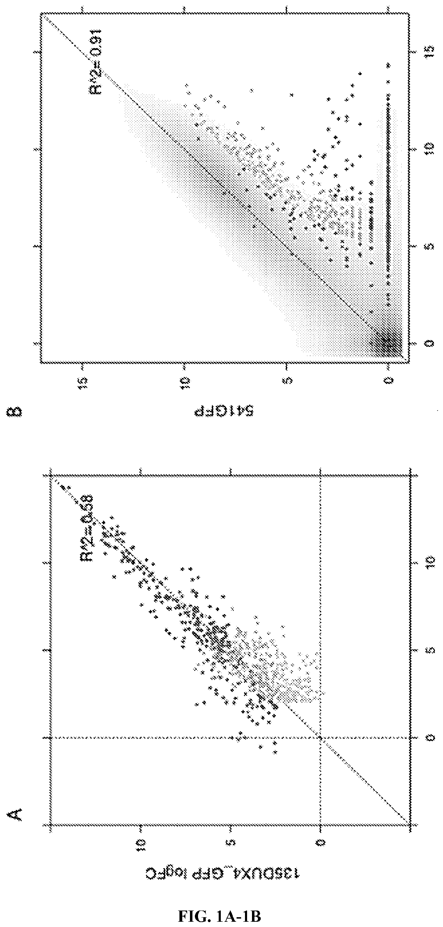

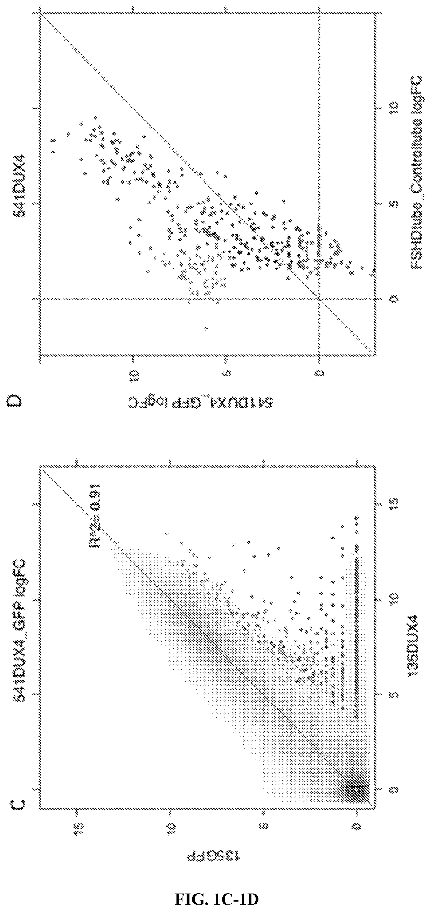

**FIGS. 1A-1D** - Dux4 induced genes mis-expressed in FSHD muscle cells. **FIG. 1A****.** DUX4 vs. GFP moderated log-fold change of DUX4 up-regulated genes in MB135 and MB541 cultured myoblast cells transduced with lentiviral DUX4. **FIGS. 1B**, **C**, Pairwise comparison of gene expression in DUX4-transduced and the control GFP-transduced MB541 (**FIG. 1B**) and MB135(**FIG. 1C**) cells. Gene counts were transformed as described in Methods. Genes are colored as in **FIG. 1A**. **FIG. 1D**. Comparison of the log-fold change of genes up-regulated by DUX4 in MB541 cells (y-axis) to genes up-regulated in FSHD myotubes compared to control myotubes (x-axis) (genes increased in FSHD compared to control cells that were up-regulated by DUX4 > 5 log-fold in MB541; increased in FSHD cells and up-regulated by DUX4 in MB51 with log-FC between one and five; increased in FSHD cells and up-regulated by DUX4 in MB541 with log-FC less than one; not increased in FSHD cells compared to controls but up-regulated by DUX4 in MB541 cells).

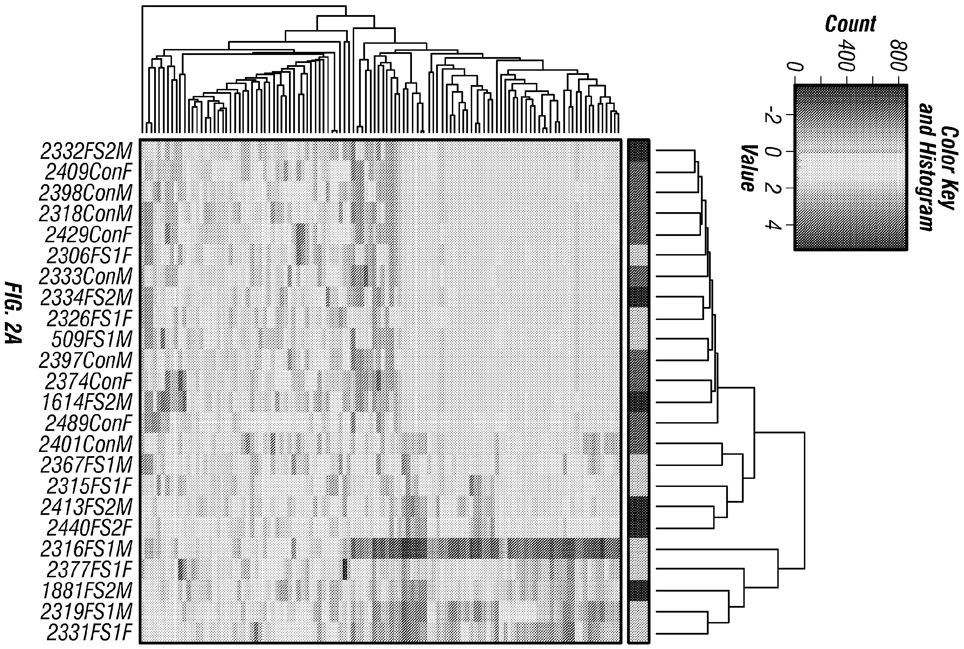

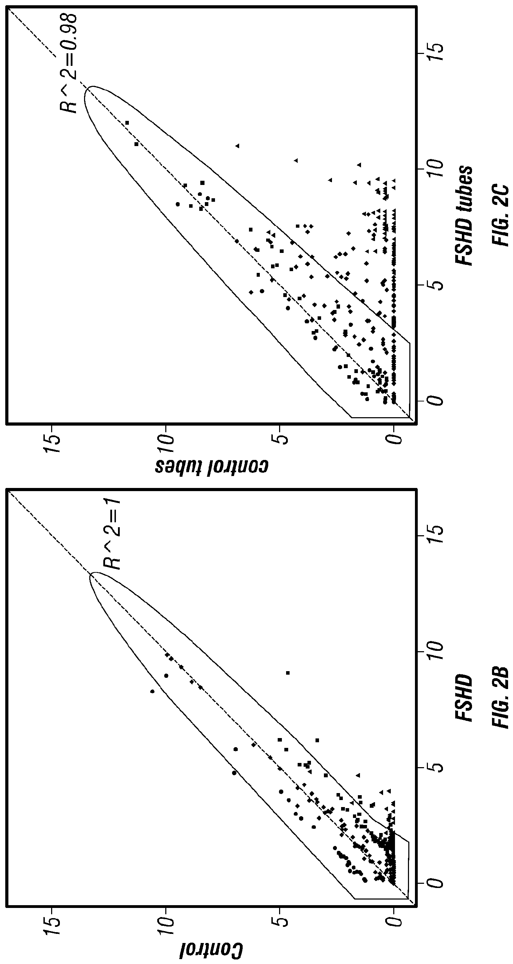

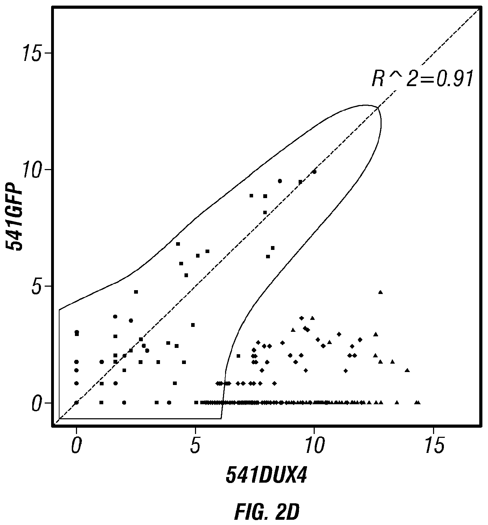

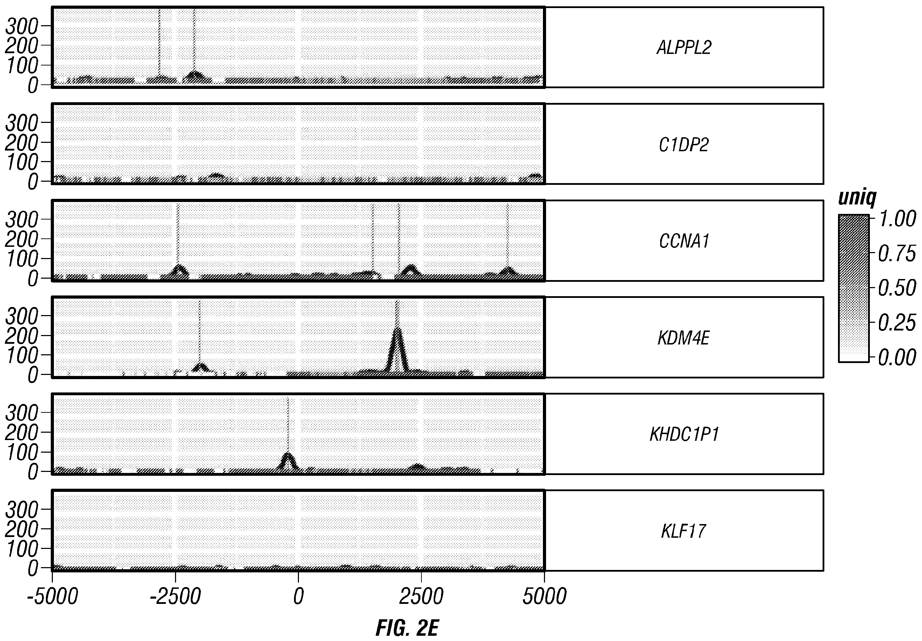

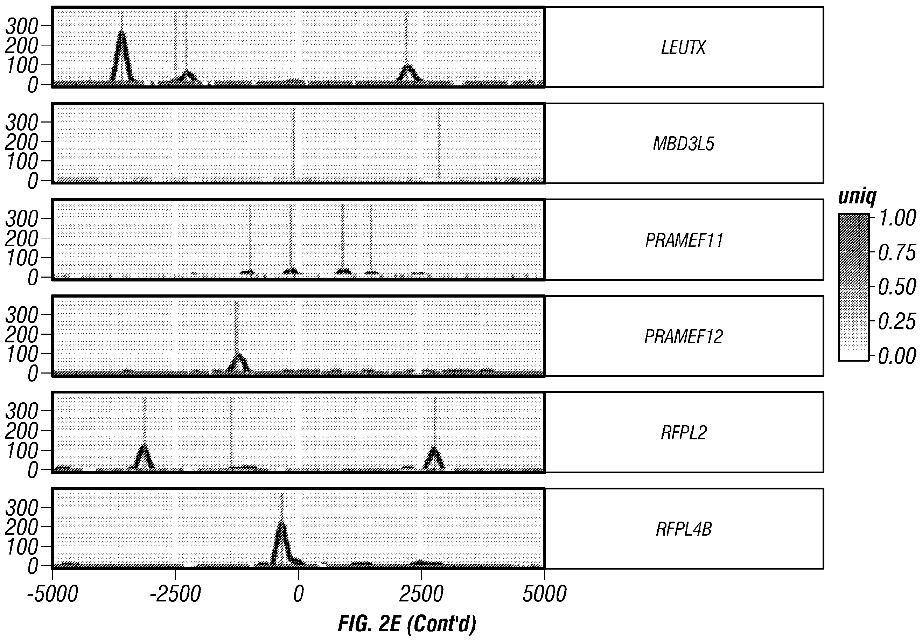

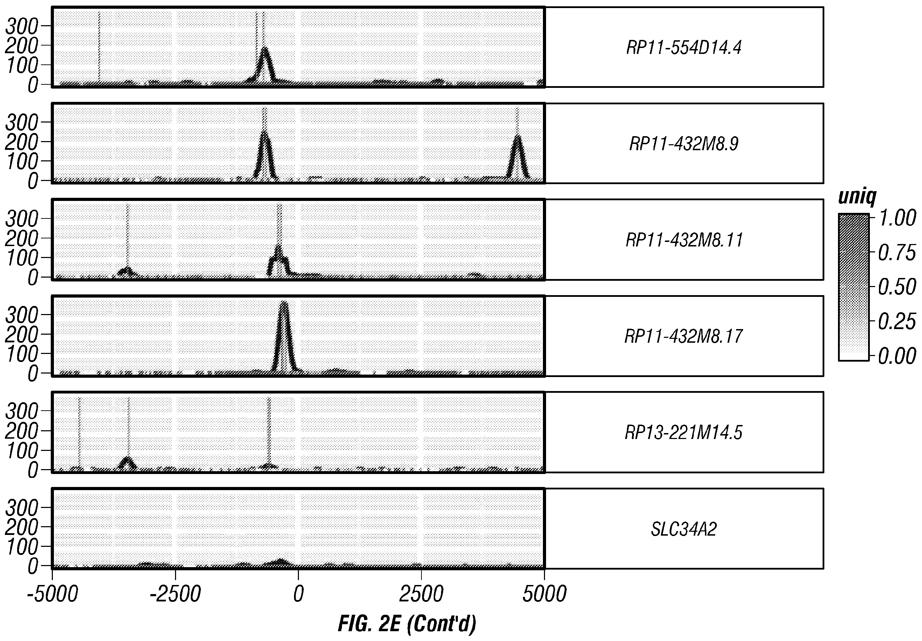

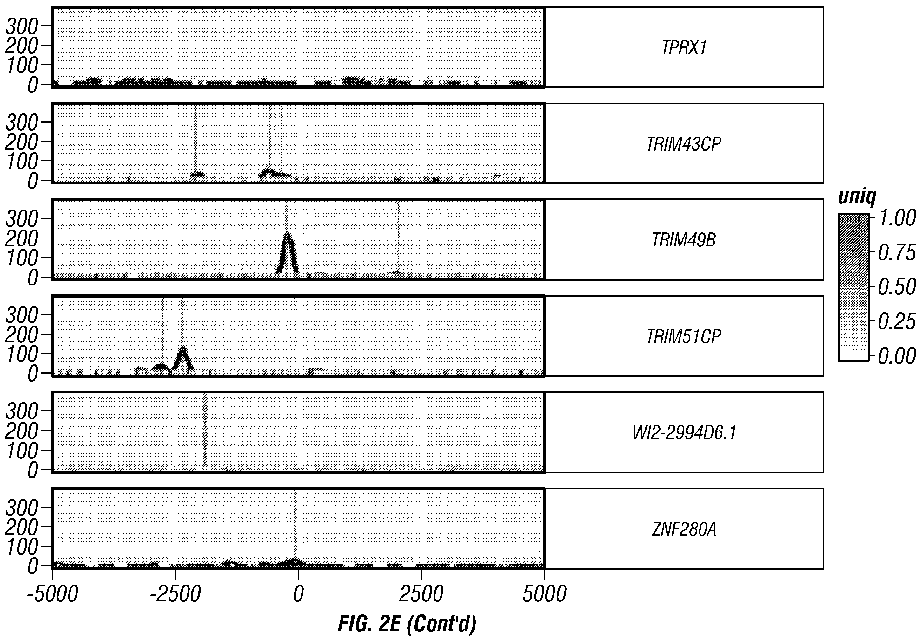

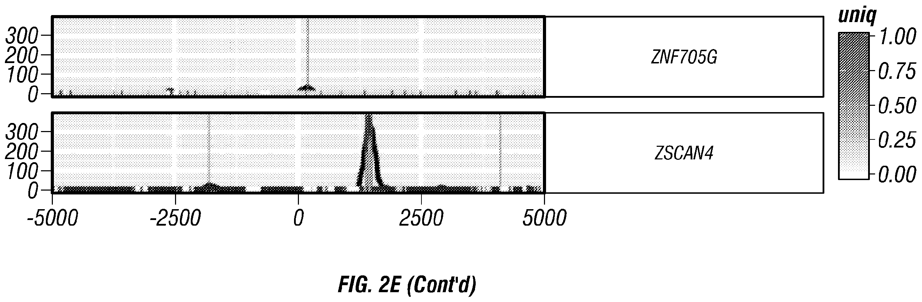

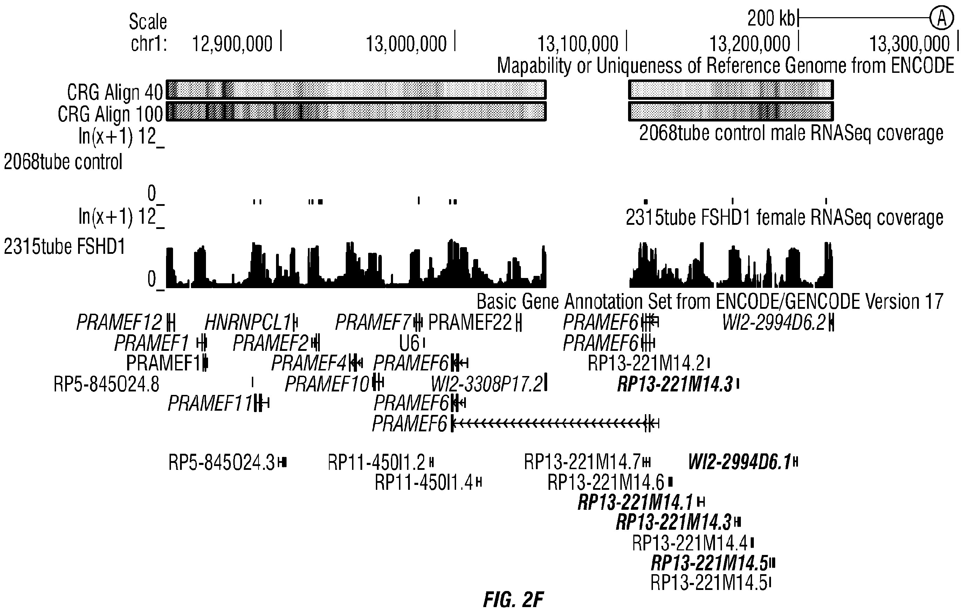

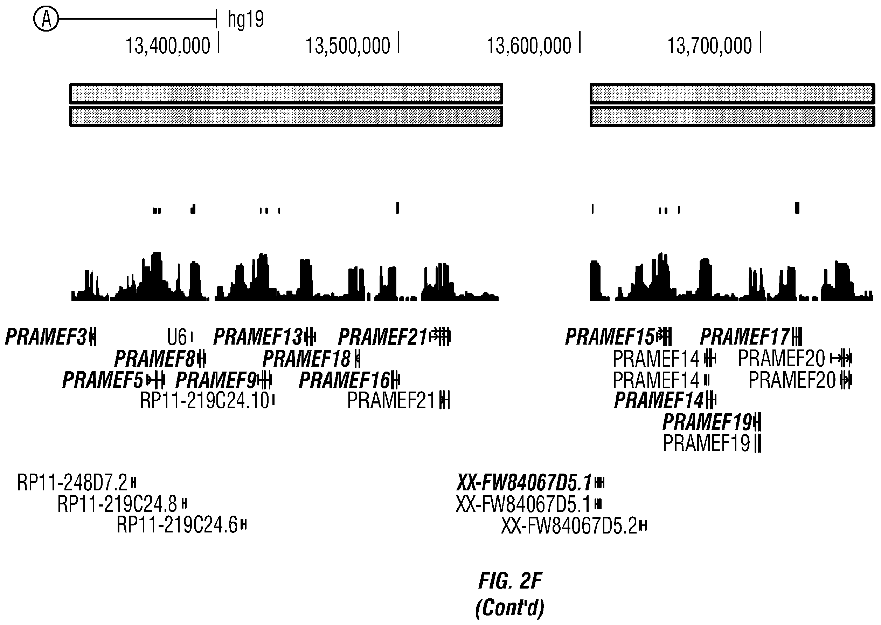

**FIGS. 2A-2F** Expression of Dux4 targets in FSHD biopsy samples. **FIG. 2A****.** Heatmap of expression of the most robust 114 DUX4 targets across all control and FSHD skeletal muscle biopsy samples. The 114 genes were selected as the subset of 228 robust DUX4 targets that met a threshold of average expression across all of the samples (transformed expression level > 0.5, see Methods). The expression levels were transformed and centered by subtracting the median for each gene. M=male, F=Female. **FIGS. 2B****,C,D.** Pairwise comparisons of gene expression levels in (**FIG. 2B**) FSHD and control biopsy samples, (**FIG. 2C**) FSHD and control cultured myotubes, and (**FIG. 2D**) MB541 transduced with DUX4 or GFP as a control. **FIG. 2E****.** DUX4 binding sites within 5Kb of a set of candidate biomarker genes. The vertical bar corresponds to the presence of a consensus motif for the DUX4 binding site. The black line indicates the DUX4 ChIP-Seq coverage in DUX4-transduced muscle cells. The gradient along the X-axis indicates the sequence uniqueness based on the ENCODE CRG Align 40 track. **FIG. 2F**. The *PRAMEF* locus. The gene names in bold type correspond to members of the 67 candidate biomarker genes expressed in FSHD biopsies. The uniqueness tracks and RNASeq tracks for one FSHD and one control myotube sample are also shown.

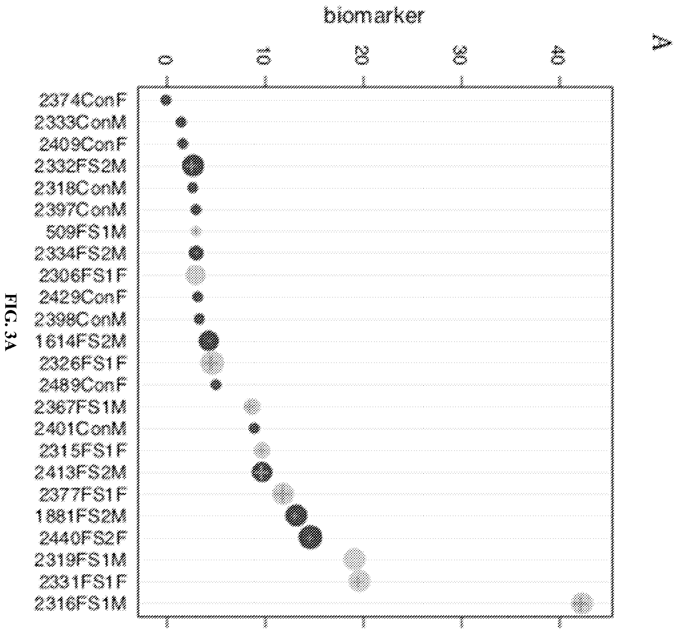

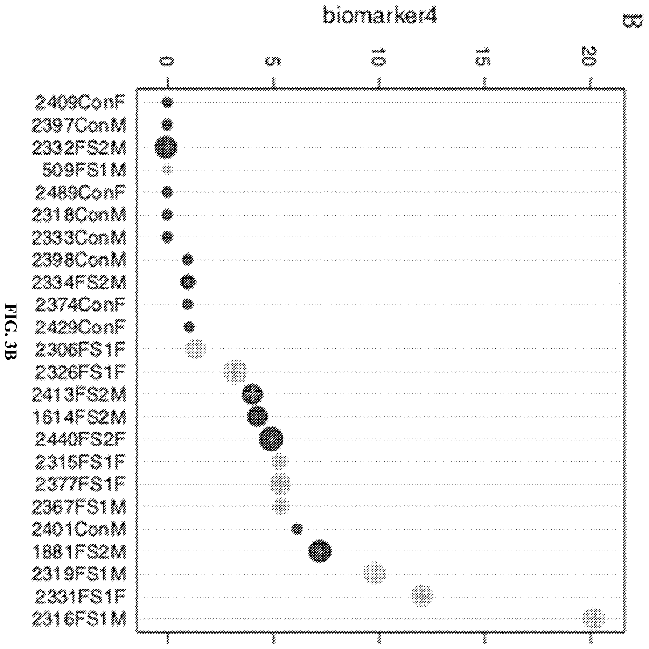

**FIGS. 3A-3B** - Association of DUX4-target gene expression with clinical severity. **FIG. 3A****.** The Y-axis is the total number of reads that mapped to any of the 67 candidate biomarker genes in each sample (scaled and square root transformed). The size of the circle corresponds to the clinical severity score (CSS) and the sizes of "+" within the circles correspond to the pathology score, with larger size indicating a higher severity. The samples with missing "+" did not have pathology scores. **FIG. 3B****.** Similar to A but using the total reads mapped to the selected four biomarkers: *LEUTX, PRAMEF2, TRIM43,* and *KHDC1L.*

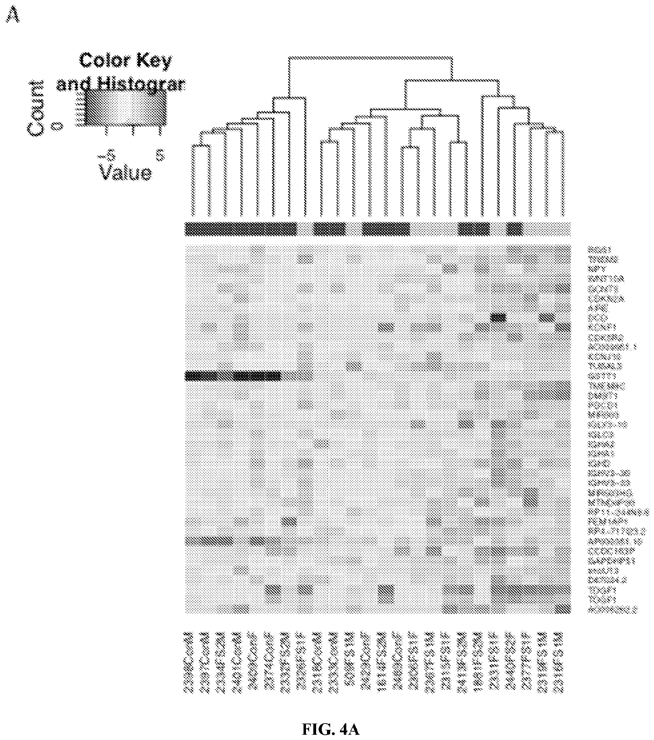

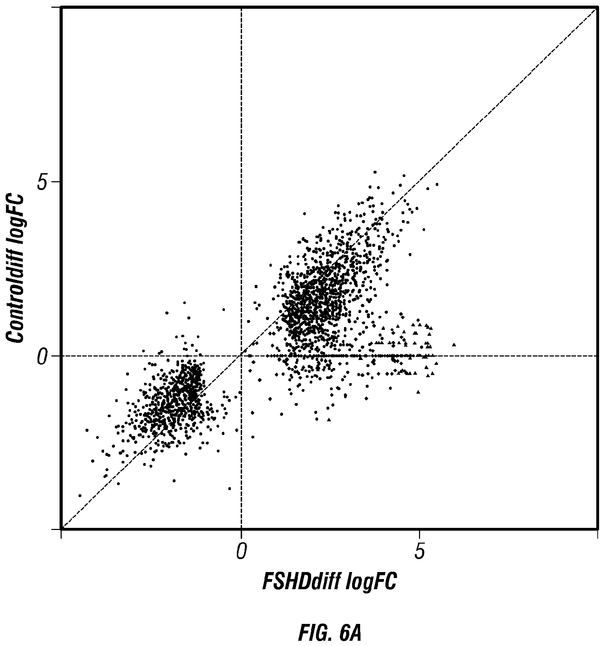

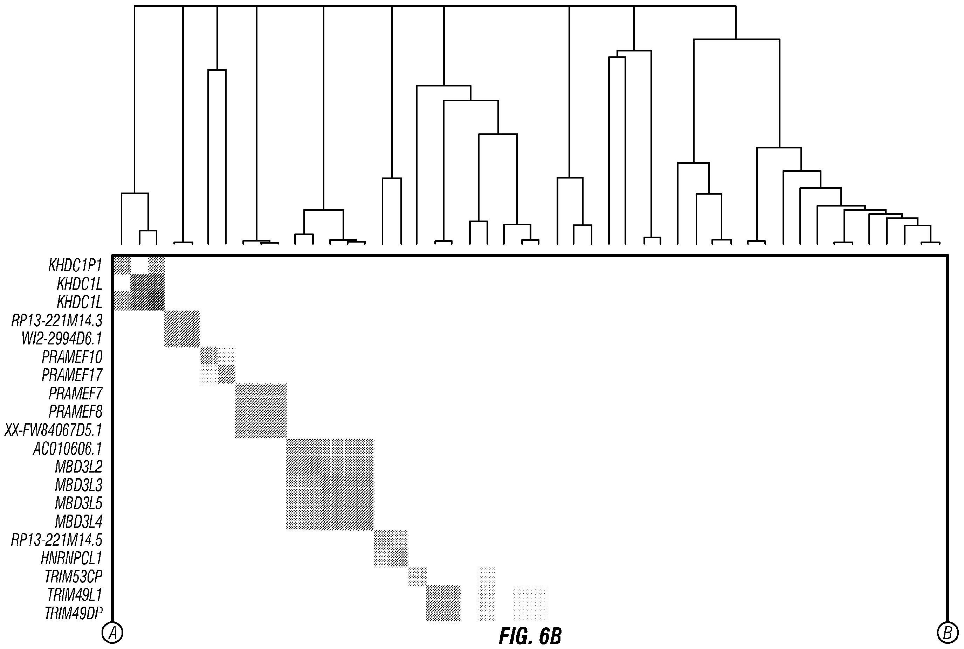

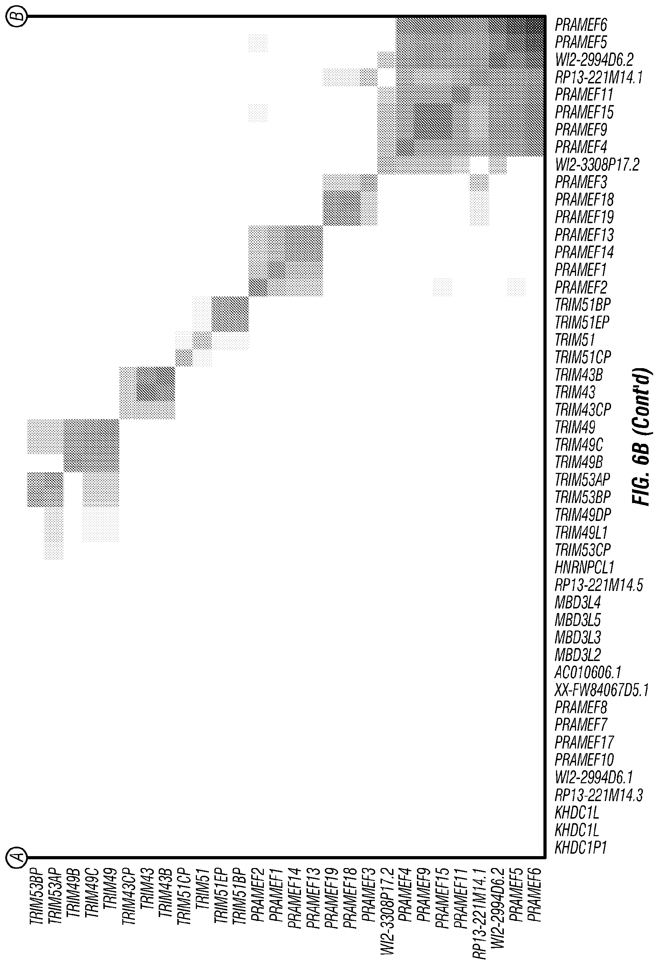

**FIGS. 4A-4B** - Genes expressed at higher levels in FSHD samples that are not DUX4 targets. **FIG. 6A****.** Heatmap of genes expressed more highly FSHD samples but that are not induced (logFC < 1) by DUX4-transduction of human muscle cells with presentation and coding as described in **FIG. 2A**. **FIG. 6B****.** Boxplot of the expression distribution for the genes in panel A based on RNA-seq reads from the FSHD samples that were DUX4-target-positive, stratified as immune and non-immune genes. The dots correspond to the expression of each gene in control sample 2401. Note that some loci have copy number variation in the human population, such as *GSTT1* that is not present in some individuals. Boxplot: vertical bounds of rectangle represent the 25^{th} and 75^{th} percentiles, the whisker extends to the extreme value (minimum or maximum) bounded by 1.5 times the IQR (25^{th} and 75^{th} interquartile range), and the filled circles are the median and the open circles are the outliers.

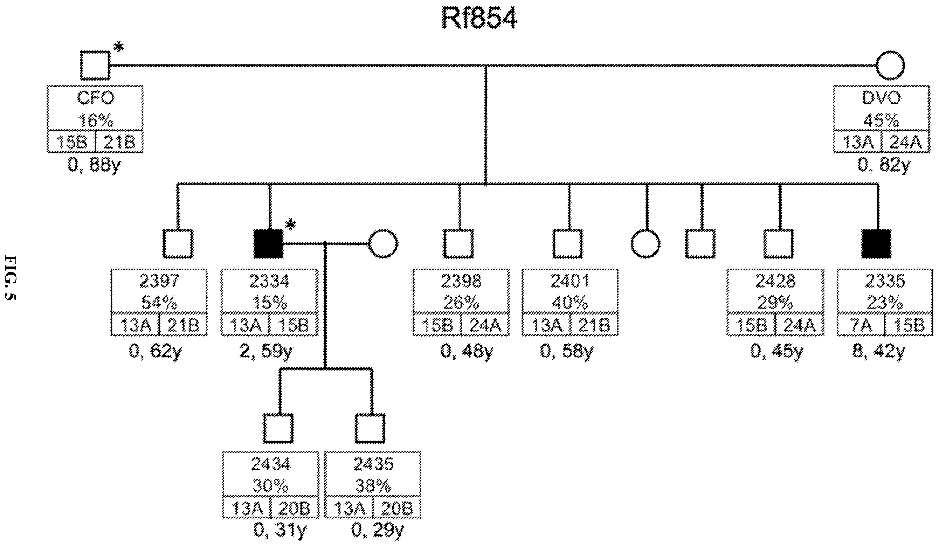

**FIG. 5** - Pedigree showing individual 2401. 2401 does not show clinical signs of FSHD despite having DUX4-target gene expression in a muscle biopsy. 2401 has one FSHD1 affected brother (2335) and one FSHD2 affected brother (2334). The father also has low D4Z4 methylation in the absence of an FSHD permissive chromosome 4 allele. SMCHD1 has a synonymous variant of unknown significance in 2334 and CFO (indicated by the *) but not in 2401 (Lemmers, Tawil et al. 2012), suggesting that another, yet unidentified, locus might modify DUX4 or DUX4-target gene expression in this family. %, degree of CpG methylation at the FseI site in the first D4Z4 repeat; number A/number B, the number of D4Z4 repeats on an FSHD-permissive 4qA allele or a non-permissive 4qB allele; number, the clinical severity score, age; filled circle/square, clinical diagnosis of FSHD.

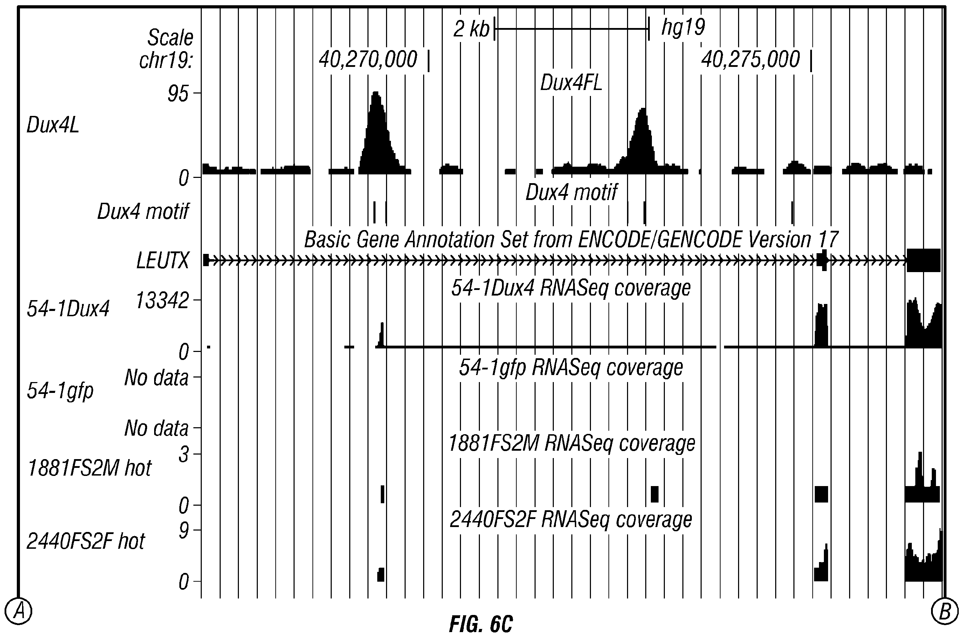

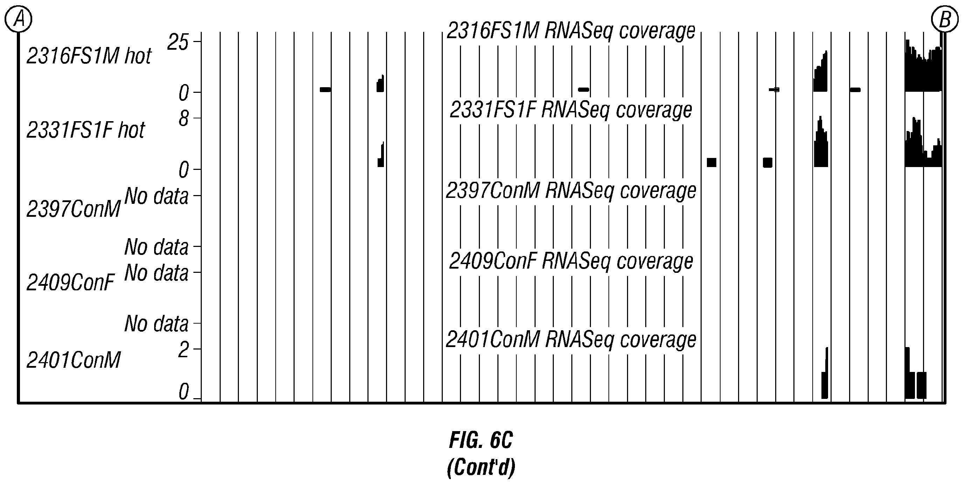

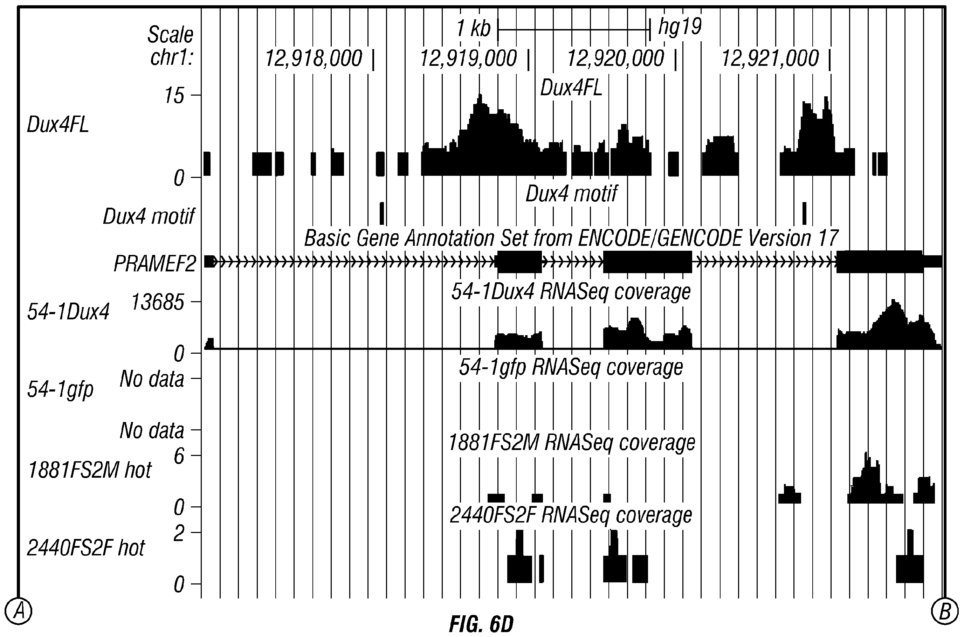

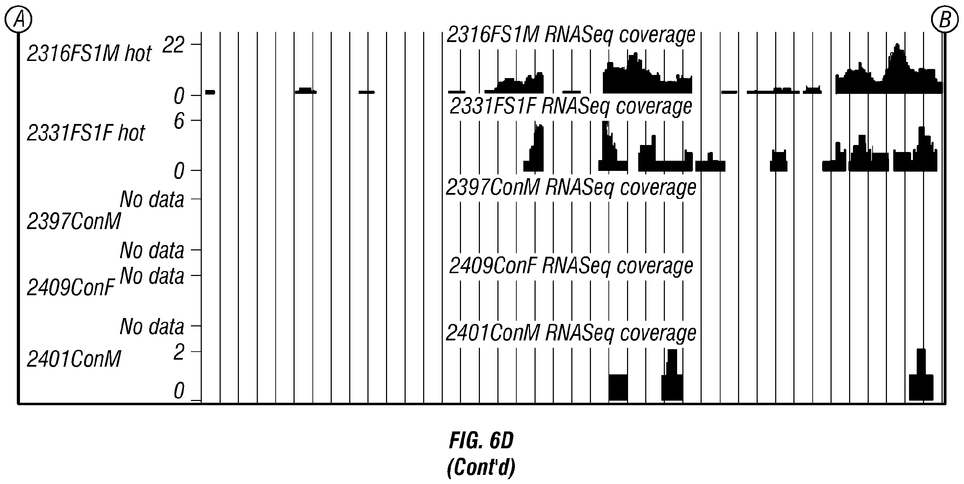

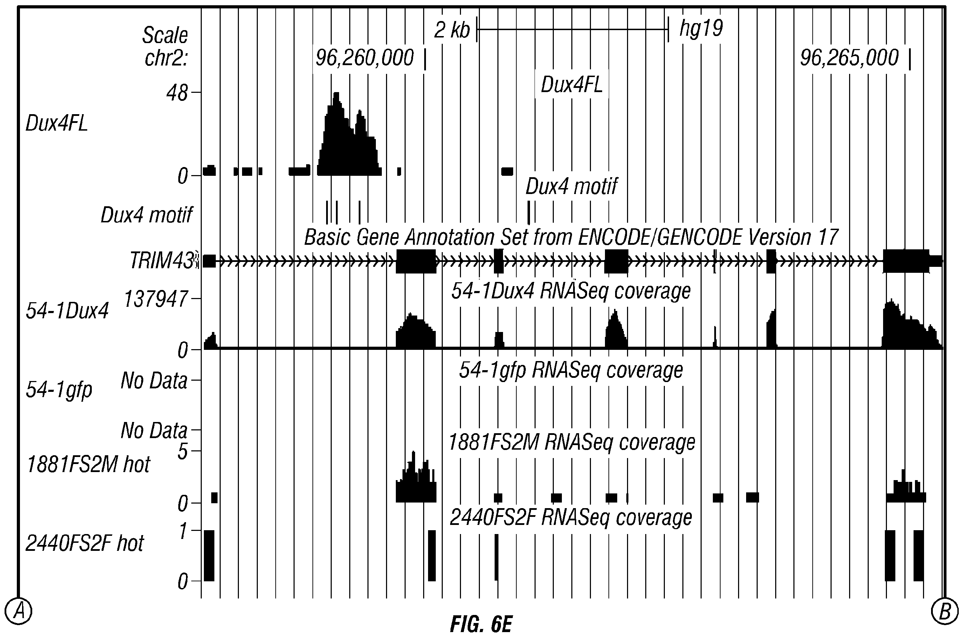

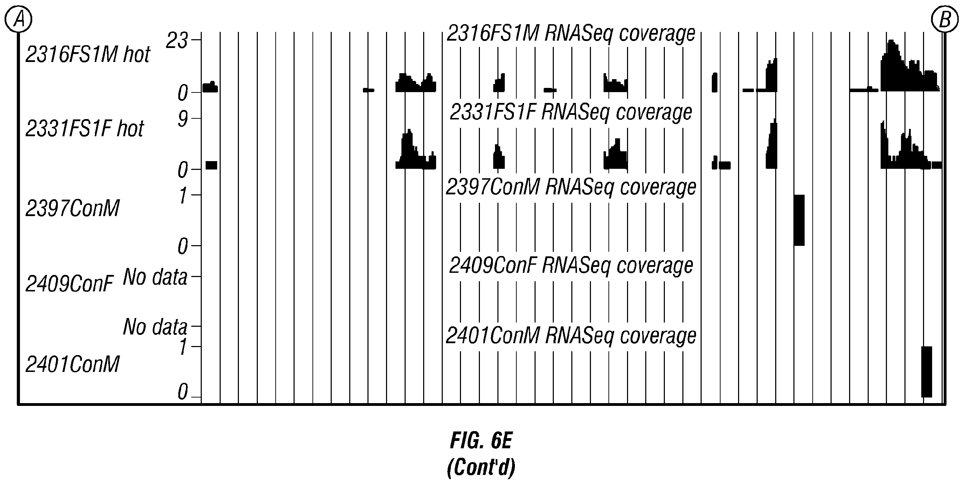

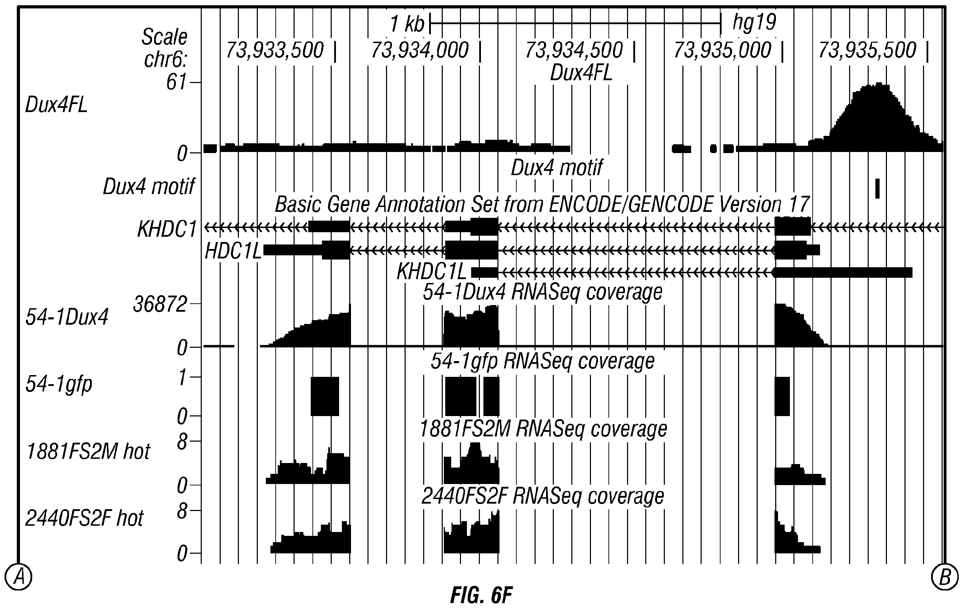

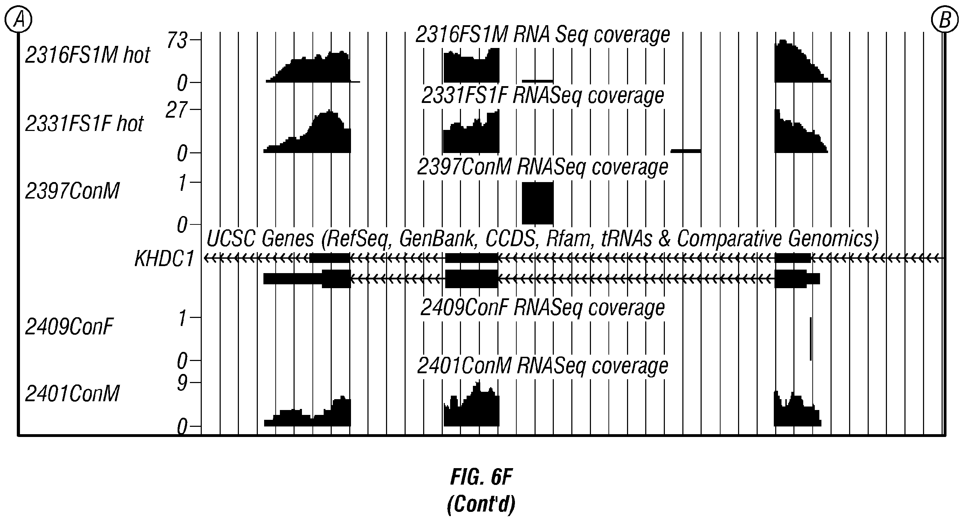

**FIGS. 6A-6F** - **FIG. 6A****.** Pairwise comparison of the log-fold change of gene expression during differentiation for FSHD and control samples. The X-axis is the average log-FC between myotubes and myoblasts for control samples, and Y-axis is average log-FC between myotubes and myoblasts for FSHD samples. **FIG. 6B****.** Clustering of biomarker candidate genes based on the number of shared reads. **FIGS. 6C****, D, E, F,** RNA-Seq and Dux4 ChIP-seq tracks at four selected biomarker candidates.

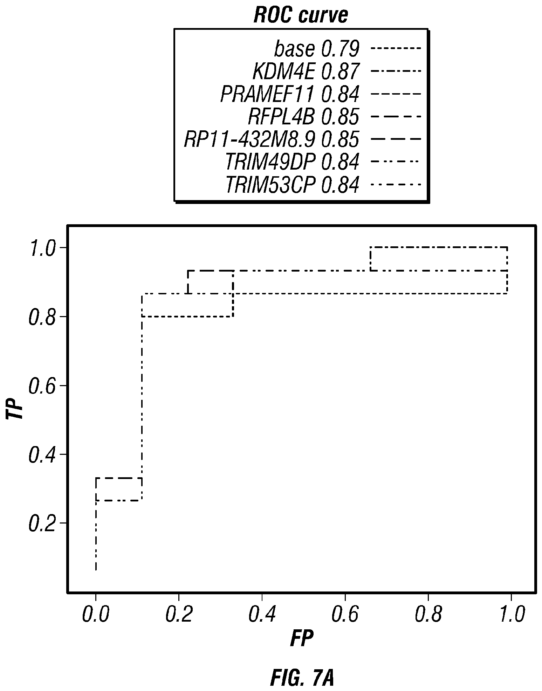

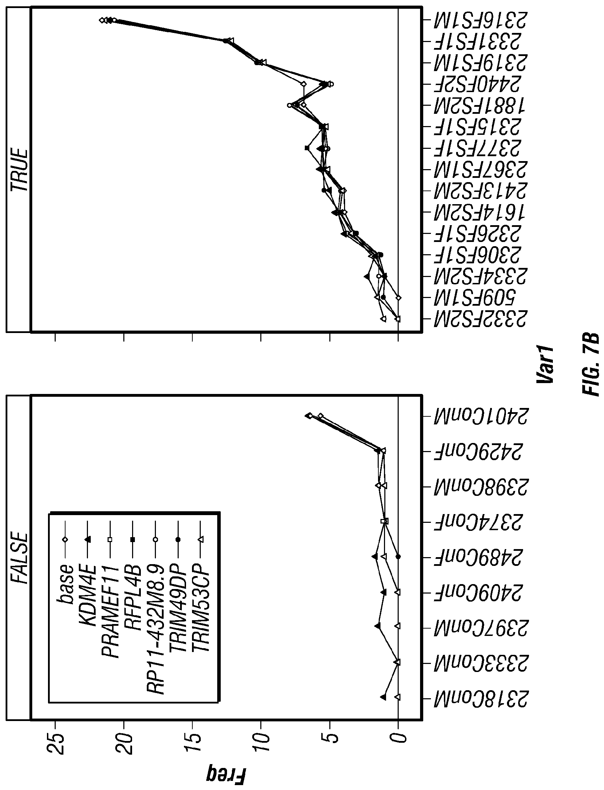

**FIGS. 7A-7B** - Adding additional DUX4 regulated genes to the four selected candidate biomarkers (*LEUTX, PRAMEF2, TRIM43, KHDC1L*) does not significantly improve discrimination between FSHD and control samples. **FIG. 7A****.** The AUROC (the area under the receiver operating characteristics) score was computed as a measurement of the discriminative power to segregate FSHD and control samples by a given gene set, using the normalized total number of reads mapped to the gene set. The base level AUROC score was computed using the four selected biomarkers. One or more genes from the pool of remaining 63 candidate biomarkers were then included and the AUROC score was then recomputed. The genes with the highest improvement of AUROC scores are shown. **FIG. 7B****.** The number of reads that mapped to the selected gene sets (normalized and square root transformed) were plotted for each sample of control (FALSE) and FSHD (TRUE) biopsies. The four FSHD samples with very low expression of Dux4 targets (509, 2332, 2334, 2306) remain as boundary cases that were not clearly separable from the control samples, whereas the remaining FSHD samples and the 2401 control sample show higher reads that the rest of the controls and adding an additional candidate biomarker gene to the selected four candidates does not improve separation.

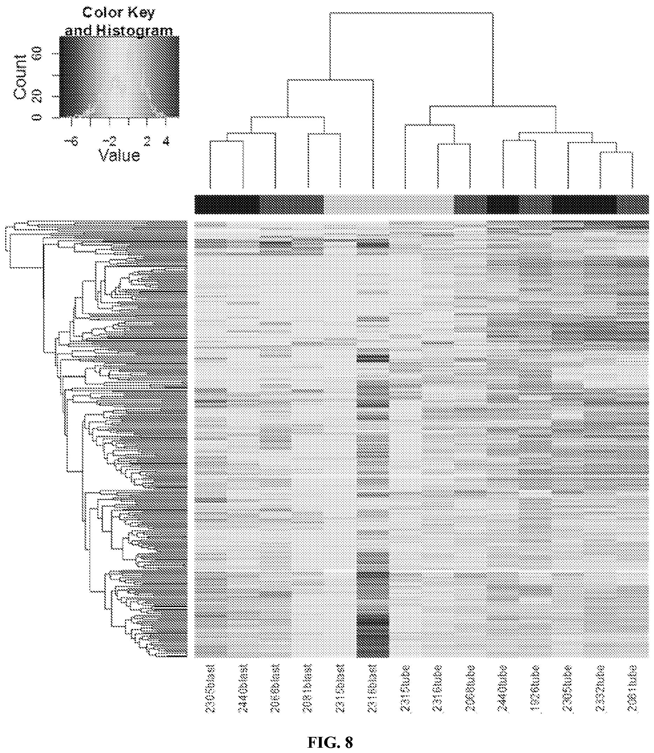

**FIG. 8** - Heatmap of expression of genes that increase during myogenesis (myotubes vs myoblasts of control samples, FDR < 0.01, adjusted log fold-change > 1) across myotubes/myoblasts samples. The expression levels are normalized, transformed and centered by subtracting the median for each gene. FSHD2 myotubes have higher expression of up-regulated genes during myogenesis than FSHD1 myotubes.

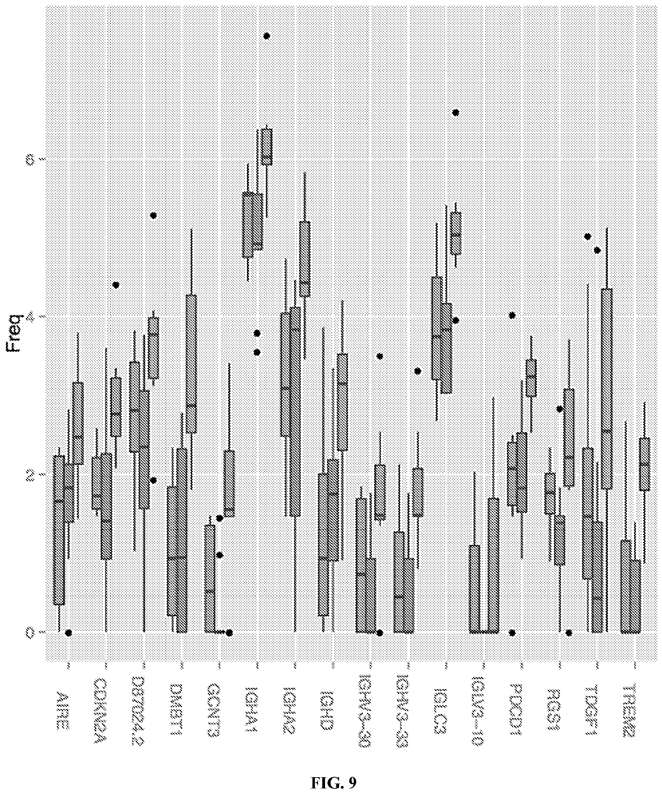

**FIG. 9** - Immune-associated non-DUX4 target genes are elevated in FSHD muscle biopsies that express DUX4 targets but not in controls or FSHD biopsies with low or absent expression of DUX4 targets. The box-plots represent the median and range of the normalized adjusted read counts for each of the immune-associated genes that were determined to be more highly expressed in DUX4-target-expressing FSHD biopsies compared to controls. This comparison shows that the FSHD biopsies with low or absent expression of DUX4-target genes also have low expression of the immune-associated genes that are not DUX4 targets. Box-plot: horizontal bar, median value; rectangle, ranges from the 25^{th} and 75^{th} percentiles; whiskers, extend to the extreme value (minimum or maximum) bounded by 1.5 times the IQR (25^{th} and 75^{th} interquartile range; filled dots, outliers.

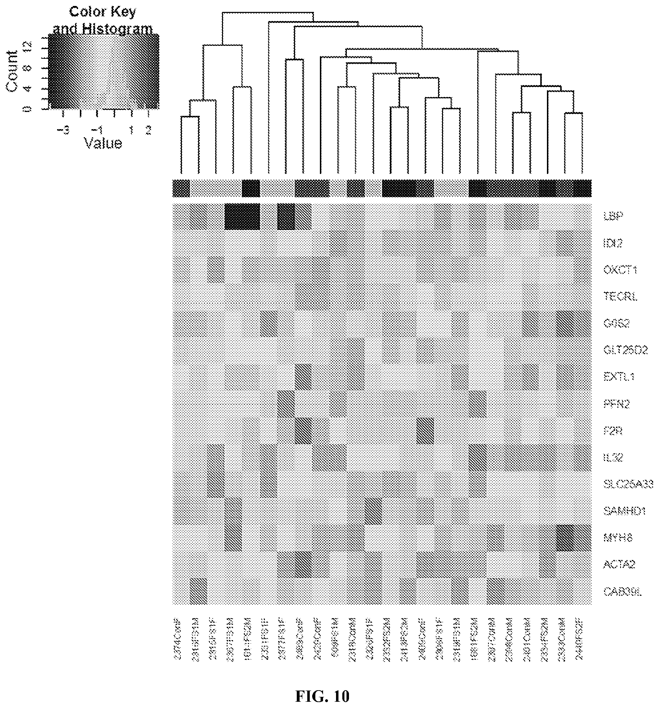

**FIG. 10** - Heatmap showing clustering of biopsy samples based on expression of candidate target genes identified from an expression array study. The RNA-seq data from the biopsy samples in the study were used to cluster the samples based on the expression of the fifteen candidate FSHD biomarkers from Rahimov et al (Rahimov, King et al. 2012) using the same approach as in **FIG. 2A** for the DUX4 target genes.

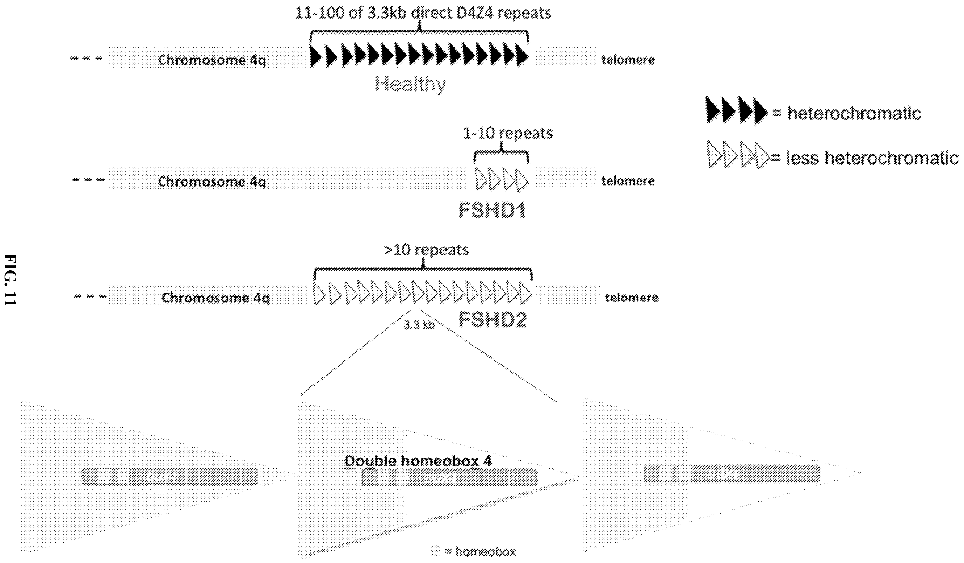

**FIG. 11** - Mis-expression of DUX4 in skeletal muscle causes Facioscapulohumeral muscular dystrophy (FSHD).

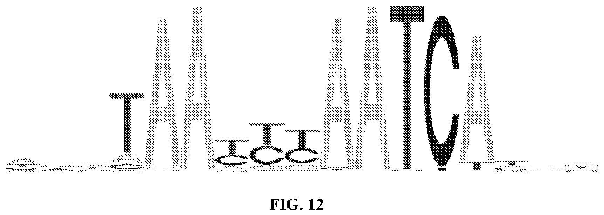

**FIG. 12** - DUX4 binding sites in the mappable genome

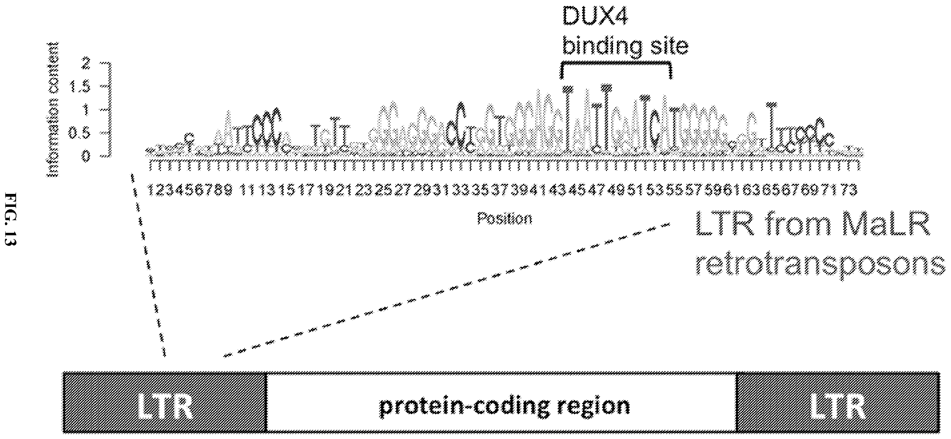

**FIG. 13** - DUX4 binding sites enriched in repeat

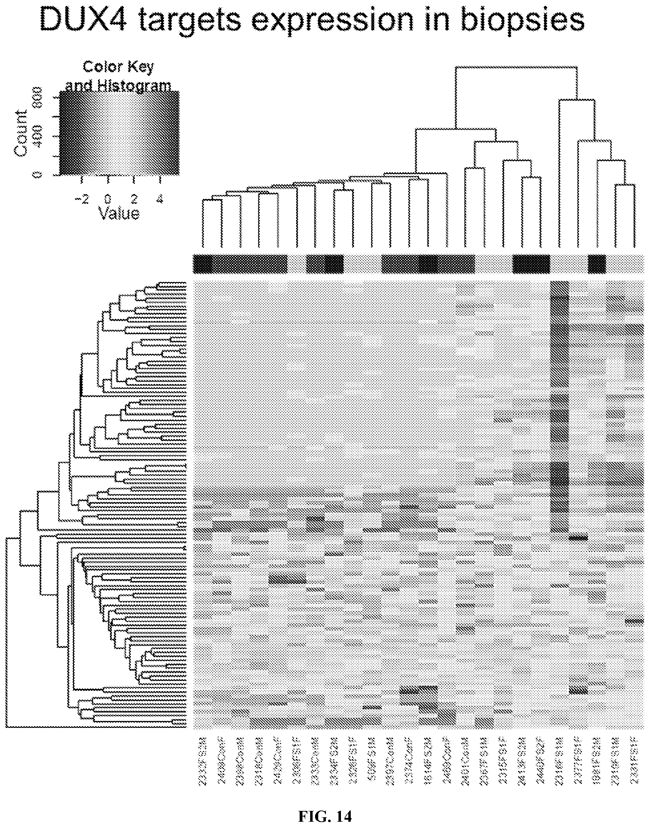

**FIG. 14** - DUX4 targets expression in biopsies

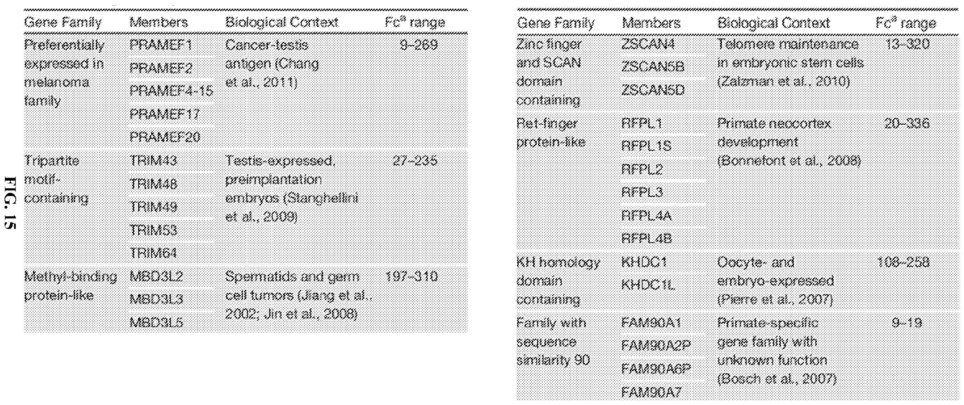

**FIG. 15** - DUX4 activates repetitive gene clusters

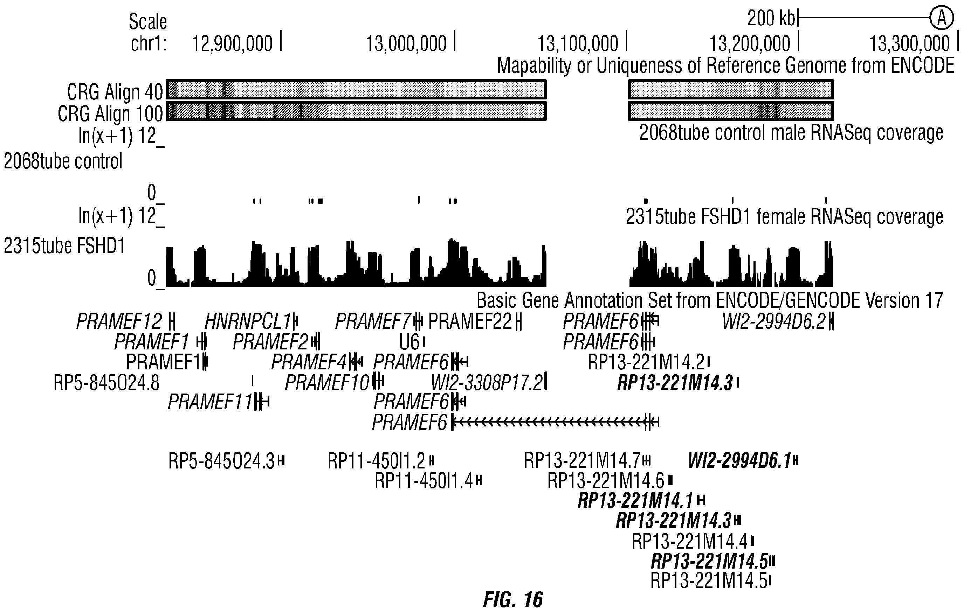

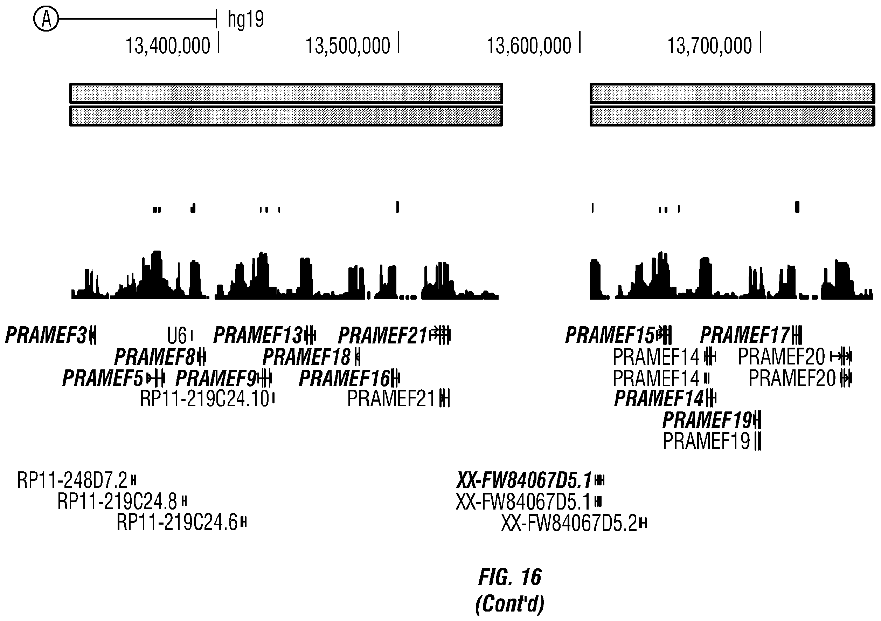

**FIG. 16** - DUX4 activates repetitive gene clusters (PAMEF gene cluster and TAF11-like macrosattllite repeats)

### DESCRIPTION OF ILLUSTRATIVE ASPECTS

### I. INTRODUCTION

Certain aspects provide a test that could assist testing services or physicians to determine the presence or risk of certain diseases such as DUX-4-related diseases. Here methods and compositions are disclosed to determine the risk or presence of DUX-4-related diseases such as FSHD.

### II. DEFINITIONS

By "subject" or "patient" is meant any single subject for which therapy is desired, including humans, cattle, dogs, guinea pigs, rabbits, chickens, and so on. Also intended to be included as a subject are any subjects involved in clinical research trials not showing any clinical sign of disease, or subjects involved in epidemiological studies, or subjects used as controls.

The term "primer," as used herein, is meant to encompass any nucleic acid that is capable of priming the synthesis of a nascent nucleic acid in a template-dependent process. Typically, primers are oligonucleotides from ten to twenty and/or thirty base pairs in length, but longer sequences can be employed. Primers may be provided in double-stranded and/or single-stranded form, although the single-stranded form is preferred.

As used herein, "increased expression" or "decreased expression" refers to an expression level of a biomarker in the subject's sample relative to a normal reference standard or normal control sample, including comparing an expression level of a biomarker in the subject's sample to a normal reference standard representing the same biomarker or a different biomarker, or an expression level of the same biomarker or a different biomarker in a normal control sample. Alternatively, "increased expression" or "decreased expression" of a biomarker in the subject's sample relative to a normal reference standard or normal control sample includes comparing an expression level of a biomarker in the subject's sample to a diseased or FSHD reference standard representing the same biomarker or a different biomarker, or an expression level of the same biomarker or a different biomarker in a diseased control sample or particularly, a FSHD control sample

In certain aspects, the expression level of a normal reference standard may be an expression level, such as a numerical value or a qualitative value, from a non-diseased, non-cancerous, or non-FSHD tissue from the same subject. Alternatively, the reference standard may be a reference standard of expression from a different human subject or group of human subjects or cohorts; in certain embodiments the reference standard may be further qualified by subject age, subject gender, severity of FSHD, previous treatment, familial history, and/or other biological or physiological feature associated with FSHD. For instance, in some embodiments, the reference standard may be the level of expression from a cohort of human patients within the same age range as the subject being tested. For example, the normal reference standard of expression may be an expression level obtained from a non-diseased, non-cancerous, or non-FSHD sample (e.g., a tissue, fluid or cell sample) of a subject or group of subjects without disease such as cancer or FSHD, or an expression level obtained from a non-diseased, non-cancerous, or non-FSHD tissue of a subject or a group of subjects, which may or may not include subjects with a disease such as cancer or FSHD.

The reference standard may be a single value or may be a range of values. The reference standard of expression can be determined using any method known to those of ordinary skill in the art. In some embodiments, the reference standard may be the level of expression determined from a cohort of subjects with FSHD or a cohort of subjects not having FSHD, lacking symptoms of FSHD, or not diagnosed with FSHD. The reference standard may also be depicted graphically as an area on a graph. In certain embodiments, a reference standard is a normalized level, while in other embodiments, it may be a level that is not stable with respect to the tissue or biological sample being tested.

"Control sample," as used herein, may refer to a normal control sample or diseased control sample that may be taken from the same individual, subject or patient or a different individual, subject or patient or a group of individuals, subjects or patients. The normal control sample may be a non-diseased, non-cancerous, or non-FSHD sample. In further aspects, the diseased control sample may refer to a sample that have symptoms of a disease. For example, a diseased control sample in the context of determining risk of developing or presence of FSHD may refer to a FSHD sample.

In the context of determining risk of developing or presence of FSHD, the normal reference standard or normal control sample may refer to a non-FSHD reference standard or non-FSHD control sample; in further aspects, the non-FSHD reference standard or non-FSHD control sample may not be known or determined to have FSHD, but may have or may not have one or more diseases or disorders that are not FSHD; while alternatively, the non-FSHD reference standard or non-FSHD control sample may not be known to have any diseases or disorders or may be determined not to have any diseases or disorders.

"Prognosis" refers to as a prediction of how a patient will progress, and whether there is a chance of recovery. Prognosis also includes prediction of favorable responses to particular treatments. A response may be either a therapeutic response (sensitivity or recurrence-free survival) or a lack of therapeutic response (residual disease, which may indicate resistance or recurrence).

"About" and "approximately" shall generally mean an acceptable degree of error for the quantity measured given the nature or precision of the measurements. Typically, exemplary degrees of error are within 20 percent (%), preferably within 10%, and more preferably within 5% of a given value or range of values. Alternatively, and particularly in biological systems, the terms "about" and "approximately" may mean values that are within an order of magnitude, preferably within 5-fold and more preferably within 2-fold of a given value. Numerical quantities given herein are approximate unless stated otherwise, meaning that the term "about" or "approximately" can be inferred when not expressly stated.

### III. DISEASES

Methods may involve the determination of altered expression of one or more biomarkers in certain DUX-4-regulated diseases relative to a normal reference standard or a normal control sample. Diseases to be prevented, treated, determined or diagnosed can be any disease that affects a subject through DUX-4 or any genes or pathways targeted by DUX-4. Non-limiting examples include muscular diseases, cancer, infections, diabetes, cardiovascular disease, neurological disease, neurodegenerative disease, genetic disease, liver disease, infection, trauma, toxicity, or immunological disease.

### A. Muscle Diseases

In certain aspects of the disclosure, subjects or patients are determined, identified or diagnosed whether they have or are at risk of a muscle disease like muscular dystrophy, or more particularly facioscapulohumeral dystrophy (FSHD).

Muscular dystrophy (MD) is a group of muscle diseases that weaken the musculoskeletal system and hamper locomotion. Muscular dystrophies are characterized by progressive skeletal muscle weakness, defects in muscle proteins, and the death of muscle cells and tissue.

It soon became evident that the disease had more than one form. In addition to Duchenne muscular dystrophy, the other major forms are Becker, limb-girdle, congenital, facioscapulohumeral, myotonic, oculopharyngeal, distal, and Emery-Dreifuss muscular dystrophy. These diseases predominately affect males, although females may be carriers of the disease gene. Most types of MD are multi-system disorders with manifestations in body systems including the heart, gastrointestinal system, nervous system, endocrine glands, eyes and brain.

Apart from the nine major types of muscular dystrophy listed above, several MD-like conditions have also been identified. Normal intellectual, behavioral, bowel and sexual function is noticed in individuals with other forms of MD and MD-like conditions. MD-affected individuals with susceptible intellectual impairment are diagnosed through molecular characteristics but not through problems associated with disability. However, a third of patients who are severely affected with MD may have cognitive impairment, behavioral, vision and speech problems.

Myotonic dystrophy (dystrophia myotonica, myotonia atrophica) is a chronic, slowly progressing, highly variable, inherited multisystemic disease. It is characterized by wasting of the muscles (muscular dystrophy), cataracts, heart conduction defects, endocrine changes, and myotonia. Two types of myotonic dystrophy exist.

Myotonic dystrophy type 1 (DM1), also called Steinert disease, has a severe congenital form and a milder childhood-onset form. Myotonic dystrophy type 2 (DM2), also called proximal myotonic myopathy (PROMM) or adult-onset form, is rarer than DM1 and generally manifests with milder signs and symptoms. Myotonic dystrophy can occur in patients of any age. Both forms of the disease display an autosomal dominant pattern of inheritance.

Facioscapulohumeral dystrophy (FSHD) is a human muscular dystrophy that initially affects the muscles of the face and upper extremities, but can progress to affect most skeletal muscles (Pandya, et al., 2008). The most common genetic cause of FSHD (FSHD1) is the deletion of a subset of D4Z4 macrosatellite repeats in the subtelomeric region of chromosome 4; whereas the less common form of FSHD (FSHD2) is caused, in the majority of cases, by a mutation in the *SMCHD1* gene on chromosome 18 (Lemmers, et al., 2012; Lemmers 2010). The mutations for FSHD1 and FSHD2 both result in decreased epigenetic repression of the D4Z4 repeat in somatic tissue and mis-expression of a retrogene, DUX4, contained within each D4Z4 repeat on chromosome 4, as well as within a nearly identical D4Z4 repeats in the subtelomeric region of chromosome 10 (van der Maarel, 2012). Genetics strongly implicates the expression of DUX4 as necessary for FSHD because decreased D4Z4 epigenetic repression, either due to contraction of the repeats or to a mutation in *SMCHD1*, results in FSHD only in individuals with a specific FSHD-permissive haplotype that contains a poly-adenylation site for the DUX4 mRNA in the region adjacent to the D4Z4 repeat (Lemmers, et al., 2010).

Despite the overwhelming genetic evidence that DUX4 mRNA expression is necessary for FSHD, its primary role has been questioned because of the extremely low abundance of the DUX4 mRNA in affected FSHD muscle cells and biopsies. However, the low mRNA abundance represents a variegated expression pattern with relatively high expression of DUX4 in a small number of nuclei at a single time point, possibly being expressed in short bursts in different muscle nuclei over time (Snider, et al., 2010).

### B. Cancer

Certain aspects involve a disease, such as cancer. For example, cancer prediction or prognosis may be based on determination of biomarkers. The cancer may be a solid tumor, metastatic cancer, or non-metastatic cancer. In certain aspects, the cancer may originate in the bladder, blood, bone, bone marrow, brain, breast, colon, esophagus, gastrointestine, gum, head, kidney, liver, lung, nasopharynx, neck, ovary, prostate, skin, stomach, testis, tongue, or uterus. In certain aspects, the cancer is human ovarian cancer. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; androblastoma, malignant; sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; hodgkin's disease; hodgkin's; paragranuloma; malignant lymphoma, small lymphocytic; malignant lymphoma, large cell, diffuse; malignant lymphoma, follicular; mycosis fungoides; other specified non-hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

### IV. DUX4

DUX4 belongs to the double-homeobox transcription factor family, and the biological role of this large class of DNA-binding proteins is largely unknown. The coding sequence of the DUX4 retrogene has been conserved in primates (Clapp *et al*., 2007), but whether this retrogene has a normal physiological function is unknown. Previously the inventors found that DUX4 is normally expressed at high levels in germ cells of human testes and is epigenetically repressed in somatic tissues (Snider *et al*., 2010), whereas the epigenetic repression of the DUX4 locus in somatic tissues is less efficient in both FSHD1 and FSHD2, resulting in DUX4 expression in FSHD muscle cell nuclei. The germline-specific expression pattern of DUX4 is similar to that of other double homeodomain proteins (Booth and Holland, 2007; Wu *et al*., 2010). The function of this distinct family of DNA-binding proteins is unknown, but their shared tissue expression pattern may indicate a possible role for double homeodomain transcription factors in reproductive biology.

DUX4 is normally expressed in the male testis. Antibody detection and in situ hybridization indicate that cells in the seminiferous tubules are expressing DUX4, most likely the spermatogonia and primary spermatocytes, although additional studies using dual detection of lineage markers remains to be performed (Snider, et al., 2010).

DUX4 is a double-homeobox transcription factor. When mis-expressed in primary human muscle cells in culture, DUX4 binds to a double-homeodomain motif and activates the expression of a broad set of genes, many involved in stem and germ cell biology (Geng, et al., 2012). Some of the DUX4 targets have been previously identified as Cancer Testis Antigens, genes whose expression is normally restricted to the immune-privileged germline that induce an immune response when mis-expressed in cancer cells. Initial studies showed that a selected set of six DUX4 targets were detected by RT-PCR in FSHD muscle cultures and biopsies, but not in control muscle, demonstrating that the low levels of DUX4 mRNA expression in FSHD muscle was sufficient to activate its downstream program of gene expression (Geng, et al., 2012).

In contrast, expression array studies of skeletal muscle biopsies did not identify DUX4 target genes as specifically mis-expressed in FSHD (Rahimov, et al., 2012). However, because of the low and variegated expression of DUX4 in FSHD muscle, it is perhaps understandable that the targets of DUX4, also expressed at low levels, might not be easily detected in discovery-oriented studies using expression arrays.

In particular aspects, to determine whether a broader set of DUX4 regulated genes could be identified as mis-expressed in FSHD muscle, and whether other gene signatures that were not related to DUX4 could be identified in FSHD, RNA-seq was performed on both cultured muscle and muscle biopsies from control, FSHD1 and FSHD2 individuals. These results identify DUX4 regulated genes as the major difference between control and FSHD, both muscle cells and biopsies, and that many of the genes not regulated by DUX4 that are associated with FSHD biopsies appear to be related to immune cell infiltration. These results suggest that DUX4 is the major determinant of abnormal gene expression in FSHD, together with an immune cell infiltrate. In addition, similar to other studies, it was found that some individuals in FSHD families can express DUX4 and DUX4 target genes without the clinical manifestation of FSHD.

This disclosure relates to U.S. Patent Application No., 13/817,531, filed on May 23, 2013, international application PCT/US12/48557, filed on July 27, 2012, U.S. Patent Application NO. 14/236,003, filed on July 27, 2012, international application NO. PCT/US12/48557, filed on July 27, 2012.

### V. SAMPLE PREPARATION

In certain aspects, methods involve obtaining a sample from a subject. The methods of obtaining provided herein may include methods of biopsy such as fine needle aspiration, core needle biopsy, vacuum assisted biopsy, incisional biopsy, excisional biopsy, punch biopsy, shave biopsy or skin biopsy. In certain aspects the sample is obtained from a biopsy from colorectal tissue by any of the biopsy methods previously mentioned. In other aspects the sample may be obtained from any of the tissues provided herein that include but are not limited to gall bladder, skin, heart, lung, breast, pancreas, liver, muscle, kidney, smooth muscle, bladder, colon, intestine, brain, prostate, esophagus, or thyroid tissue. Alternatively, the sample may be obtained from any other source including but not limited to blood, sweat, hair follicle, buccal tissue, tears, menses, feces, or saliva. In certain aspects the sample is obtained from cystic fluid or fluid derived from a tumor or neoplasm. In yet other aspects the cyst, tumor or neoplasm is colorectal. In certain aspects of the current methods, any medical professional such as a doctor, nurse or medical technician may obtain a biological sample for testing. Yet further, the biological sample can be obtained without the assistance of a medical professional.

A sample may include but is not limited to, tissue, cells, or biological material from cells or derived from cells of a subject. The biological sample may be a heterogeneous or homogeneous population of cells or tissues. The biological sample may be obtained using any method known to the art that can provide a sample suitable for the analytical methods described herein. The sample may be obtained by non-invasive methods including but not limited to: scraping of the skin or cervix, swabbing of the cheek, saliva collection, urine collection, feces collection, collection of menses, tears, or semen.

The sample may be obtained by methods known in the art. In certain aspects the samples are obtained by biopsy. In other aspects the sample is obtained by swabbing, scraping, phlebotomy, or any other methods known in the art. In some cases, the sample may be obtained, stored, or transported using components of a kit of the present methods. In some cases, multiple samples, such as multiple colorectal samples may be obtained for diagnosis by the methods described herein. In other cases, multiple samples, such as one or more samples from one tissue type (for example colon) and one or more samples from another tissue (for example buccal) may be obtained for diagnosis by the methods. In some cases, multiple samples such as one or more samples from one tissue type (e.g. rectal) and one or more samples from another tissue (e.g. cecum) may be obtained at the same or different times. Samples may be obtained at different times are stored and/or analyzed by different methods. For example, a sample may be obtained and analyzed by routine staining methods or any other cytological analysis methods.

In some aspects the biological sample may be obtained by a physician, nurse, or other medical professional such as a medical technician, endocrinologist, cytologist, phlebotomist, radiologist, or a pulmonologist. The medical professional may indicate the appropriate test or assay to perform on the sample. In certain aspects a molecular profiling business may consult on which assays or tests are most appropriately indicated. In further aspects of the current methods, the patient or subject may obtain a biological sample for testing without the assistance of a medical professional, such as obtaining a whole blood sample, a urine sample, a fecal sample, a buccal sample, or a saliva sample.

In other cases, the sample is obtained by an invasive procedure including but not limited to: biopsy, needle aspiration, or phlebotomy. The method of needle aspiration may further include fine needle aspiration, core needle biopsy, vacuum assisted biopsy, or large core biopsy. In some aspects, multiple samples may be obtained by the methods herein to ensure a sufficient amount of biological material.

General methods for obtaining biological samples are also known in the art. Publications such as Ramzy, Ibrahim Clinical Cytopathology and Aspiration Biopsy 2001 describes general methods for biopsy and cytological methods. In one aspect, the sample is a fine needle aspirate of a colorectal or a suspected colorectal tumor or neoplasm. In some cases, the fine needle aspirate sampling procedure may be guided by the use of an ultrasound, X-ray, or other imaging device.

In some aspects of the present methods, the molecular profiling business may obtain the biological sample from a subject directly, from a medical professional, from a third party, or from a kit provided by a molecular profiling business or a third party. In some cases, the biological sample may be obtained by the molecular profiling business after the subject, a medical professional, or a third party acquires and sends the biological sample to the molecular profiling business. In some cases, the molecular profiling business may provide suitable containers, and excipients for storage and transport of the biological sample to the molecular profiling business.

In some aspects of the methods described herein, a medical professional need not be involved in the initial diagnosis or sample acquisition. An individual may alternatively obtain a sample through the use of an over the counter (OTC) kit. An OTC kit may contain a means for obtaining said sample as described herein, a means for storing said sample for inspection, and instructions for proper use of the kit. In some cases, molecular profiling services are included in the price for purchase of the kit. In other cases, the molecular profiling services are billed separately. A sample suitable for use by the molecular profiling business may be any material containing tissues, cells, nucleic acids, genes, gene fragments, expression products, gene expression products, or gene expression product fragments of an individual to be tested. Methods for determining sample suitability and/or adequacy are provided.

In some aspects, the subject may be referred to a specialist such as an oncologist, surgeon, or endocrinologist. The specialist may likewise obtain a biological sample for testing or refer the individual to a testing center or laboratory for submission of the biological sample. In some cases the medical professional may refer the subject to a testing center or laboratory for submission of the biological sample. In other cases, the subject may provide the sample. In some cases, a molecular profiling business may obtain the sample.

### VI. NUCLEIC ACID ASSAYS

It is contemplated that a number of assays could be employed to analyze nucleic acids in biological samples. Such assays include, but are not limited to, array hybridization, solution hybridization, nucleic amplification, polymerase chain reaction, quantitative PCR, RT-PCR, *in situ* hybridization, Northern hybridization, hybridization protection assay (HPA) (GenProbe), digital PCR, ddPCR (digital droplet PCR), nCounter (nanoString), BEAMing (Beads, Emulsions, Amplifications, and Magnetics) (Inostics), ARMS (Amplification Refractory Mutation Systems), RNA-Seq, TAm-Seg (Tagged-Amplicon deep sequencing) PAP (Pyrophosphorolysis-activation polymerization, branched DNA (bDNA) assay (Chiron), rolling circle amplification (RCA), single molecule hybridization detection (US Genomics), invader assay (ThirdWave Technologies), and/or Oligo Ligation Assay (OLA), hybridization, and array analysis (e.g. U.S. Patent applications 11/141,707, filed May 31, 2005; 11/857,948, filed September 19, 2007; 11/273,640, filed November 14, 2005 and provisional patent application 60/869,295, filed December 8, 2006).

### A. Isolation of Nucleic Acids

Nucleic acids may be isolated using techniques well known to those of skill in the art, though in particular embodiments, methods for isolating small nucleic acid molecules, and/or isolating RNA molecules can be employed.

Chromatography is a process often used to separate or isolate nucleic acids from protein or from other nucleic acids. Such methods can involve electrophoresis with a gel matrix, filter columns, alcohol precipitation, and/or other chromatography.

If nucleic acids from cells is to be used or evaluated, methods generally involve lysing the cells with a chaotropic (*e.g*., guanidinium isothiocyanate) and/or detergent (*e.g*., N-lauroyl sarcosine) prior to implementing processes for isolating particular populations of RNA.

In particular methods for separating targeted nucleic acids from other nucleic acids, a gel matrix may be prepared using polyacrylamide, though agarose can also be used. The gels may be graded by concentration or they may be uniform. Plates or tubing can be used to hold the gel matrix for electrophoresis. For example, one-dimensional electrophoresis may be employed for the separation of nucleic acids. Plates may be used to prepare a slab gel, while the tubing (glass or rubber, typically) can be used to prepare a tube gel. The phrase "tube electrophoresis" refers to the use of a tube or tubing, instead of plates, to form the gel. Materials for implementing tube electrophoresis can be readily prepared by a person of skill in the art or purchased.

Methods may involve the use of organic solvents and/or alcohol to isolate nucleic acids used in methods and compositions disclosed herein. Some aspects are described in U.S. Patent Application Serial No. 10/667,126, which is hereby incorporated by reference.

In certain aspects, this disclosure provides methods for efficiently isolating small RNA molecules from cells comprising: adding an alcohol solution to a cell lysate and applying the alcohol/lysate mixture to a solid support before eluting the RNA molecules from the solid support. In some aspects, the amount of alcohol added to a cell lysate achieves an alcohol concentration of about 55% to 60%. While different alcohols can be employed, ethanol works well. A solid support may be any structure, and it includes beads, filters, and columns, which may include a mineral or polymer support with electronegative groups. A glass fiber filter or column may work particularly well for such isolation procedures.

### B. Amplification

Many methods exist for evaluating biomarker levels by amplifying all or part of nucleic acid sequences such as mature mRNA, precursor mRNAs, and/or primary mRNAs. Suitable nucleic acid polymerization and amplification techniques include reverse transcription (RT), polymerase chain reaction (PCR), real-time PCR (quantitative PCR (q-PCR)), digital PCR, ddPCR (digital droplet PCR), nucleic acid sequence-base amplification (NASBA), ligase chain reaction, multiplex ligatable probe amplification, invader technology (Third Wave), rolling circle amplification, in vitro transcription (IVT), strand displacement amplification, transcription-mediated amplification (TMA), RNA (Eberwine) amplification, and other methods that are known to persons skilled in the art. In certain embodiments, more than one amplification method may be used, such as reverse transcription followed by real time PCR (Chen *et al*., 2005 and/or U.S. Patent Application serial number 11/567,082, filed December 5, 2006).

An exemplary PCR reaction includes multiple amplification steps, or cycles that selectively amplify target nucleic acid species. An exemplary reaction includes three steps: a denaturing step in which a target nucleic acid is denatured; an annealing step in which a set of PCR primers (forward and reverse primers) anneal to complementary DNA strands; and an elongation step in which a thermostable DNA polymerase elongates the primers. By repeating these steps multiple times, a DNA fragment is amplified to produce an amplicon, corresponding to the target DNA sequence. Exemplary PCR reactions may include 20 or more cycles of denaturation, annealing, and elongation. In many cases, the annealing and elongation steps can be performed concurrently, in which case the cycle contains only two steps. Since mature mRNAs are single stranded, a reverse transcription reaction (which produces a complementary cDNA sequence) may be performed prior to PCR reactions. Reverse transcription reactions include the use of, *e.g.,* a RNA-based DNA polymerase (reverse transcriptase) and a primer.

In PCR and q-PCR methods, for example, a set of primers is used for each target sequence. In certain embodiments, the lengths of the primers depends on many factors, including, but not limited to, the desired hybridization temperature between the primers, the target nucleic acid sequence, and the complexity of the different target nucleic acid sequences to be amplified. In certain embodiments, a primer is about 15 to about 35 nucleotides in length. In other embodiments, a primer is equal to or fewer than 15, 20, 25, 30, or 35 nucleotides in length or any range derivable therein. In additional embodiments, a primer is at least 35 nucleotides in length.

In some embodiments, two or more nucleic acids are amplified in a single reaction volume or multiple reaction volumes. In certain aspects, one or more nucleic may be used as a normalization control or a reference nucleic acid for normalization. Normalization may be performed in separate or the same reaction volumes as other amplification reactions.

One aspect includes multiplex q-PCR, such as qRT-PCR, which enables simultaneous amplification and quantification of at least one nucleic acids of interest and at least one reference nucleic acid in one reaction volume by using more than one pair of primers and/or more than one probe. The primer pairs may comprise at least one amplification primer that uniquely binds each nucleic acid, and the probes are labeled such that they are distinguishable from one another, thus allowing simultaneous quantification of multiple nucleic acids. Multiplex qRT-PCR has research and diagnostic uses, including but not limited to detection of nucleic acids for diagnostic, prognostic, and therapeutic applications.

A single combined reaction for q-PCR, may be used to: (1) decrease risk of experimenter error, (2) reduce assay-to-assay variability, (3) decrease risk of target or product contamination, and (4) increase assay speed. The qRT-PCR reaction may further be combined with the reverse transcription reaction by including both a reverse transcriptase and a DNA-based thermostable DNA polymerase. When two polymerases are used, a "hot start" approach may be used to maximize assay performance (U.S. Patents 5,411,876 and 5,985,619). For example, the components for a reverse transcriptase reaction and a PCR reaction may be sequestered using one or more thermoactivation methods or chemical alteration to improve polymerization efficiency (U.S. Patents 5,550,044, 5,413,924, and 6,403,341).

To assess the expression of biomarkers, real-time RT-PCR detection can be used to screen nucleic acids or RNA isolated from samples of interest and a related reference such as, but not limited to a normal adjacent tissue (NAT) samples.

A panel of amplification targets may be chosen for real-time RT-PCR quantification. In one aspect, the panel of targets includes one or more nucleic acids described herein. The selection of the panel or targets can be based on the results of microarray expression analyses.

One example of a normalization target is 5S rRNA and others can be included. Reverse transcription (RT) reaction components may be assembled on ice prior to the addition of RNA template. Total RNA template may be added and mixed. RT reactions may be incubated in an appropriate PCR System at an appropriate temperature (such as 15-30°C, including all values and ranges there between) for an appropriate time, 15 to 30 minutes or longer, then at a temperature of 35 to 42 to 50°C for 10 to 30 to 60 minutes, and then at 80 to 85 to 95°C for 5 minutes, then placed on wet ice. Reverse Transcription reaction components may include nuclease-free water, reverse transcription buffer, dNTP mix, RT Primer, RNase Inhibitor, Reverse Transcriptase, and RNA.

Following assembly of the PCR reaction components a portion of the RT reaction is transferred to the PCR mix. PCR reactions may be incubated in an PCR system at an elevated temperature (*e.g*., 95°C) for 1 minute or so, then for a number of cycles of denaturing, annealing, and extension (*e.g*., 40 cycles of 95°C for 5 seconds and 60°C for 30 seconds). Results can be analyzed, for example, with SDS V2.3 (Applied Biosystems). Real-time PCR components may include Nuclease-free water, MgCl₂, PCR Buffer, dNTP mix, one or more primers, DNA Polymerase, cDNA from RT reaction and one or more detectable label.

Software tools such as NormFinder (Andersen *et al*., 2004) may be used to determine targets for normalization with the targets of interest and tissue sample set. For normalization of the real-time RT-PCR results, the cycle threshold (Cₜ) value (a log value) for the microRNA of interest is subtracted from the geometric mean Cₜ value of normalization targets. Fold change can be determined by subtracting the dCₜ normal reference (N) from the corresponding dCₜ sample being evaluated (T), producing a ddCₜ(T-N) value for each sample. The average ddCₜ(T-N) value across all samples is converted to fold change by 2^{ddCt}. The representative p-values are determined by a two-tailed paired Student's t-test from the dCₜ values of sample and normal reference.

There may be provided methods for using digital PCR. Digital polymerase chain reaction (digital PCR, DigitalPCR, dPCR, or dePCR) is a refinement of conventional polymerase chain reaction methods that can be used to directly quantify and clonally amplify nucleic acids including DNA, cDNA or RNA. The key difference between dPCR and traditional PCR lies in the method of measuring nucleic acids amounts, with the former being a more precise method than PCR. PCR carries out one reaction per single sample. dPCR also carries out a single reaction within a sample, however the sample is separated into a large number of partitions and the reaction is carried out in each partition individually. This separation allows a more reliable collection and sensitive measurement of nucleic acid amounts. The method has been demonstrated as useful for studying variations in gene sequences - such as copy number variants and point mutations - and it is routinely used for clonal amplification of samples for "next-generation sequencing."

For example in digital PCR, a sample is partitioned so that individual nucleic acid molecules within the sample are localized and concentrated within many separate regions. (The capture or isolation of individual nucleic acid molecules has been effected in micro well plates, capillaries, the dispersed phase of an emulsion, and arrays of miniaturized chambers, as well as on nucleic acid binding surfaces.) The partitioning of the sample allows one to estimate the number of different molecules by assuming that the molecule population follows the Poisson distribution. As a result, each part will contain "0" or "1" molecules, or a negative or positive reaction, respectively. After PCR amplification, nucleic acids may be quantified by counting the regions that contain PCR end-product, positive reactions. In conventional PCR, the number of PCR amplification cycles is proportional to the starting copy number. dPCR, however, is not dependent on the number of amplification cycles to determine the initial sample amount, eliminating the reliance on uncertain exponential data to quantify target nucleic acids and therefore provides absolute quantification.

### C. Nucleic Acid Arrays

Certain aspects concern the preparation and use of nucleic acid arrays or nucleic acid probe arrays, which are ordered macroarrays or microarrays of nucleic acid molecules (probes) that are fully or nearly complementary or identical to a plurality of nucleic acids molecules or precursor nucleic acids molecules and are positioned on a support or support material in a spatially separated organization. Macroarrays are typically sheets of nitrocellulose or nylon upon which probes have been spotted. Microarrays position the nucleic acid probes more densely such that up to 10,000 nucleic acid molecules can be fit into a region typically 1 to 4 square centimeters.

Representative methods and apparatus for preparing a microarray have been described, for example, in U.S. Patents 5,143,854; 5,202,231; 5,242,974; 5,288,644; 5,324,633; 5,384,261; 5,405,783; 5,412,087; 5,424,186; 5,429,807; 5,432,049; 5,436,327; 5,445,934; 5,468,613; 5,470,710; 5,472,672; 5,492,806; 5,503,980; 5,510,270; 5,525,464; 5,527,681; 5,529,756; 5,532,128; 5,545,531; 5,547,839; 5,554,501; 5,556,752; 5,561,071; 5,571,639; 5,580,726; 5,580,732; 5,593,839; 5,599,695; 5,599,672; 5,610;287; 5,624,711; 5,631,134; 5,639,603; 5,654,413; 5,658,734; 5,661,028; 5,665,547; 5,667,972; 5,695,940; 5,700,637; 5,744,305; 5,800,992; 5,807,522; 5,830,645; 5,837,196; 5,871,928; 5,847,219; 5,876,932; 5,919,626; 6,004,755; 6,087,102; 6,368,799; 6,383,749; 6,617,112; 6,638,717; 6,720,138, as well as WO 93/17126; WO 95/11995; WO 95/21265; WO 95/21944; WO 95/35505; WO 96/31622; WO 97/10365; WO 97/27317; WO 99/35505; WO 09923256; WO 09936760; WO 0138580; WO 0168255; WO 03020898; WO 03040410; WO 03053586; WO 03087297; WO 03091426; WO 03100012; WO 04020085; WO 04027093; EP 373 203; EP 785 280; EP 799 897 and UK 8 803 000. Moreover, a person of ordinary skill in the art could readily analyze data generated using an array. Such protocols are disclosed above, and include information found in WO 9743450; WO 03023058; WO 03022421; WO 03029485; WO 03067217; WO 03066906; WO 03076928; WO 03093810; WO 03100448A1.

Microarrays can be fabricated by spotting nucleic acid molecules, *e.g.,* genes, oligonucleotides, *etc.,* onto substrates or fabricating oligonucleotide sequences *in situ* on a substrate. Spotted or fabricated nucleic acid molecules can be applied in a high density matrix pattern of up to about 30 non-identical nucleic acid molecules per square centimeter or higher, *e.g.* up to about 100 or even 1000 per square centimeter. Microarrays typically use coated glass as the solid support, in contrast to the nitrocellulose-based material of filter arrays. A variety of different array devices in which a plurality of distinct nucleic acid probes are stably associated with the surface of a solid support are known to those of skill in the art. Useful substrates for arrays include nylon, glass, metal, plastic, and silicon. Such arrays may vary in a number of different ways, including average probe length, sequence or types of probes, nature of bond between the probe and the array surface, e.g. covalent or non-covalent, and the like. The labeling and screening methods are not limited by with respect to any parameter except that the probes detect nucleic acids; consequently, methods and compositions may be used with a variety of different types of nucleic acid arrays.

Representative methods and apparatuses for preparing a microarray have been described, for example, in U.S. Patents 5,143,854; 5,202,231; 5,242,974; 5,288,644; 5,324,633; 5,384,261; 5,405,783; 5,412,087; 5,424,186; 5,429,807; 5,432,049; 5,436,327; 5,445,934; 5,468,613; 5,470,710; 5,472,672; 5,492,806; 5,525,464; 5,503,980; 5,510,270; 5,525,464; 5,527,681; 5,529,756; 5,532,128; 5,545,531; 5,547,839; 5,554,501; 5,556,752; 5,561,071; 5,571,639; 5,580,726; 5,580,732; 5,593,839; 5,599,695; 5,599,672; 5,610;287; 5,624,711; 5,631,134; 5,639,603; 5,654,413; 5,658,734; 5,661,028; 5,665,547; 5,667,972; 5,695,940; 5,700,637; 5,744,305; 5,800,992; 5,807,522; 5,830,645; 5,837,196; 5,871,928; 5,847,219; 5,876,932; 5,919,626; 6,004,755; 6,087,102; 6,368,799; 6,383,749; 6,617,112; 6,638,717; 6,720,138, as well as WO 93/17126; WO 95/11995; WO 95/21265; WO 95/21944; WO 95/35505; WO 96/31622; WO 97/10365; WO 97/27317; WO 99/35505; WO 09923256; WO 09936760; WO0138580; WO 0168255; WO 03020898; WO 03040410; WO 03053586; WO 03087297; WO 03091426; WO03100012; WO 04020085; WO 04027093; EP 373 203; EP 785 280; EP 799 897 and UK 8 803 000.

It is contemplated that the arrays can be high density arrays, such that they contain 2, 20, 25, 50, 80, 100, or more, or any integer derivable therein, different probes. It is contemplated that they may contain 1000, 16,000, 65,000, 250,000 or 1,000,000 or more, or any integer or range derivable therein, different probes. The probes can be directed to targets in one or more different organisms or cell types. In some embodiments, the oligonucleotide probes may range from 5 to 50, 5 to 45, 10 to 40, 9 to 34, or 15 to 40 nucleotides in length. In certain embodiments, the oligonucleotide probes are 5, 10, 15, 20, 25, 30, 35, 40 nucleotides in length, including all integers and ranges there between.

Moreover, the large number of different probes can occupy a relatively small area providing a high density array having a probe density of generally greater than about 60, 100, 600, 1000, 5,000, 10,000, 40,000, 100,000, or 400,000 different oligonucleotide probes per cm². The surface area of the array can be about or less than about 1, 1.6, 2, 3, 4, 5, 6, 7, 8, 9, or 10 cm².

Moreover, a person of ordinary skill in the art could readily analyze data generated using an array. Such protocols are disclosed herein or may be found in, for example, WO 9743450; WO 03023058; WO 03022421; WO 03029485; WO 03067217; WO 03066906; WO 03076928; WO 03093810; WO 03100448A1.

### D. Hybridization

After an array or a set of probes is prepared and the nucleic acids in the sample is labeled, the population of target nucleic acids may be contacted with the array or probes under hybridization conditions, where such conditions can be adjusted, as desired, to provide for an optimum level of specificity in view of the particular assay being performed. Suitable hybridization conditions are well known to those of skill in the art and reviewed in Sambrook et al. (2001) and WO 95/21944. Of particular interest in many embodiments is the use of stringent conditions during hybridization. Stringent conditions are known to those of skill in the art.

### E. Labels and Labeling Techniques

In some embodiments, methods concern nucleic acids that are directly or indirectly labeled. It is contemplated that nucleic acids may first be isolated and/or purified prior to labeling. This may achieve a reaction that more efficiently labels the mRNA, as opposed to other RNA in a sample in which the nucleic acids is not isolated or purified prior to labeling. In many embodiments, the label is non-radioactive. Generally, nucleic acids may be labeled by adding labeled nucleotides (one-step process) or adding nucleotides and labeling the added nucleotides (two-step process).

In some embodiments, nucleic acids are labeled by catalytically adding to the nucleic acid an already labeled nucleotide or nucleotides. One or more labeled nucleotides can be added to nucleic acids molecules. See U.S Patent 6,723,509.

In other embodiments, an unlabeled nucleotide or nucleotides may be catalytically added to a nucleic acids, and the unlabeled nucleotide is modified with a chemical moiety that enables it to be subsequently labeled. In some embodiments, the chemical moiety is a reactive amine such that the nucleotide is an amine-modified nucleotide. Examples of amine-modified nucleotides are well known to those of skill in the art, many being commercially available such as from Ambion, Sigma, Jena Bioscience, and TriLink.

In contrast to labeling of cDNA during its synthesis, the issue for labeling at least one FSHD biomarker is how to label the already existing molecule. In some methods, embodiments concern the use of an enzyme capable of using a di- or tri-phosphate ribonucleotide or deoxyribonucleotide as a substrate for its addition to a FSHD biomarker. Moreover, in specific embodiments, it involves using a modified di- or tri-phosphate ribonucleotide, which is added to the 3' end of a nucleic acids. The source of the enzyme is not limiting. Examples of sources for the enzymes include yeast, gram-negative bacteria such as *E. coli, Lactococcus lactis,* and sheep pox virus.

Enzymes capable of adding such nucleotides include, but are not limited to, poly(A) polymerase, terminal transferase, and polynucleotide phosphorylase. In specific embodiments, a ligase is contemplated as not being the enzyme used to add the label, and instead, a non-ligase enzyme is employed. Terminal transferase catalyzes the addition of nucleotides to the 3' terminus of a nucleic acid. Polynucleotide phosphorylase can polymerize nucleotide diphosphates without the need for a primer.

Labels on nucleic acids or nucleic acid probes may be colorimetric (includes visible and UV spectrum, including fluorescent), luminescent, enzymatic, or positron emitting (including radioactive). The label may be detected directly or indirectly. Radioactive labels include ¹²⁵I, ³²P, ³³P, and ³⁵S. Examples of enzymatic labels include alkaline phosphatase, luciferase, horseradish peroxidase, and β-galactosidase. Labels can also be proteins with luminescent properties, *e.g.,* green fluorescent protein and phycoerythrin.

The colorimetric and fluorescent labels contemplated for use as conjugates include, but are not limited to, Alexa Fluor dyes, BODIPY dyes, such as BODIPY FL; Cascade Blue; Cascade Yellow; coumarin and its derivatives, such as 7-amino-4-methylcoumarin, aminocoumarin and hydroxycoumarin; cyanine dyes, such as Cy3 and Cy5; eosins and erythrosins; fluorescein and its derivatives, such as fluorescein isothiocyanate; macrocyclic chelates of lanthanide ions, such as Quantum Dye™; Marina Blue; Oregon Green; rhodamine dyes, such as rhodamine red, tetramethylrhodamine and rhodamine 6G; Texas Red; fluorescent energy transfer dyes, such as thiazole orange-ethidium heterodimer; and, TOTAB.

Specific examples of dyes include, but are not limited to, those identified above and the following: Alexa Fluor 350, Alexa Fluor 405, Alexa Fluor 430, Alexa Fluor 488, Alexa Fluor 500. Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, and, Alexa Fluor 750; amine-reactive BODIPY dyes, such as BODIPY 493/503, BODIPY 530/550, BODIPY 558/568, BODIPY 564/570, BODIPY 576/589, BODIPY 581/591, BODIPY 630/650, BODIPY 650/655, BODIPY FL, BODIPY R6G, BODIPY TMR, and, BODIPY-TR; Cy3, Cy5, 6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, SYPRO, TAMRA, 2',4',5',7'-Tetrabromosulfonefluorescein, and TET.

Specific examples of fluorescently labeled ribonucleotides are available from Molecular Probes, and these include, Alexa Fluor 488-5-UTP, Fluorescein-12-UTP, BODIPY FL-14-UTP, BODIPY TMR-14-UTP, Tetramethylrhodamine-6-UTP, Alexa Fluor 546-14-UTP, Texas Red-5-UTP, and BODIPY TR-14-UTP. Other fluorescent ribonucleotides are available from Amersham Biosciences, such as Cy3-UTP and Cy5-UTP.

Examples of fluorescently labeled deoxyribonucleotides include Dinitrophenyl (DNP)-11-dUTP, Cascade Blue-7-dUTP, Alexa Fluor 488-5-dUTP, Fluorescein-12-dUTP, Oregon Green 488-5-dUTP, BODIPY FL-14-dUTP, Rhodamine Green-5-dUTP, Alexa Fluor 532-5-dUTP, BODIPY TMR-14-dUTP, Tetramethylrhodamine-6-dUTP, Alexa Fluor 546-14-dUTP, Alexa Fluor 568-5-dUTP, Texas Red-12-dUTP, Texas Red-5-dUTP, BODIPY TR-14-dUTP, Alexa Fluor 594-5-dUTP, BODIPY 630/650-14-dUTP, BODIPY 650/665-14-dUTP; Alexa Fluor 488-7-OBEA-dCTP, Alexa Fluor 546-16-OBEA-dCTP, Alexa Fluor 594-7-OBEA-dCTP, Alexa Fluor 647-12-OBEA-dCTP.

It is contemplated that nucleic acids may be labeled with two different labels. Furthermore, fluorescence resonance energy transfer (FRET) may be employed in methods (*e.g.,* Klostermeier *et al*., 2002; Emptage, 2001; Didenko, 2001).

Alternatively, the label may not be detectable *per se*, but indirectly detectable or allowing for the isolation or separation of the targeted nucleic acid. For example, the label could be biotin, digoxigenin, polyvalent cations, chelator groups and the other ligands, include ligands for an antibody.

A number of techniques for visualizing or detecting labeled nucleic acids are readily available. Such techniques include, microscopy, arrays, Fluorometry, Light cyclers or other real time PCR machines, FACS analysis, scintillation counters, Phosphoimagers, Geiger counters, MRI, CAT, antibody-based detection methods (Westerns, immunofluorescence, immunohistochemistry), histochemical techniques, HPLC (Griffey *et al*., 1997), spectroscopy, capillary gel electrophoresis (Cummins *et al*., 1996), spectroscopy; mass spectroscopy; radiological techniques; and mass balance techniques.

When two or more differentially colored labels are employed, fluorescent resonance energy transfer (FRET) techniques may be employed to characterize association of one or more nucleic acid. Furthermore, a person of ordinary skill in the art is well aware of ways of visualizing, identifying, and characterizing labeled nucleic acids, and accordingly, such protocols may be used as part of some embodiments. Examples of tools that may be used also include fluorescent microscopy, a BioAnalyzer, a plate reader, Storm (Molecular Dynamics), Array Scanner, FACS (fluorescent activated cell sorter), or any instrument that has the ability to excite and detect a fluorescent molecule.

### VII. PROTEIN EXPRESSION ASSAYS

In some embodiments, the gene or protein expression of one or more biomarkers is compared to a control, for example, the expression in the DNA region and/or the expression of a nearby gene sequence from a sample from an individual known to have or not to have FSHD, or to an expression level that distinguishes between FSHD and non-FSHD states. Such methods, like the methods of detecting expression described herein, are useful in providing risk prediction, diagnosis, prognosis, *etc.,* of FSHD. Methods for measuring transcription and/or translation of a particular gene sequence or biomarker are well known in the art. See, for example, Ausubel, Current Protocols in Molecular Biology, 1987-2006, John Wiley & Sons; and Sambrook and Russell, Molecular Cloning: A Laboratory Manual, 3rd Edition, 2000.

Polypeptides encoded by the genes described herein can be detected and/or quantified by any methods known to those of skill in the art from samples as described herein. In some embodiments, antibodies can also be used to detect polypeptides encoded by the genes described herein. Antibodies to these polypeptides can be produced using well known techniques (see, *e.g.,* Harlow & Lane, 1988 and Harlow & Lane, 1999; Coligan, 1991; Goding, 1986; and Kohler & Milstein, 1975). Such techniques include antibody preparation by selection of antibodies from libraries of recombinant antibodies in phage or similar vectors, as well as preparation of polyclonal and monoclonal antibodies by immunizing rabbits or mice (see, *e.g.,* Huse *et al*., 1989; Ward *et al*., 1989).

Once specific antibodies are available, binding interactions with the proteins of interest can be detected by a variety of immunoassay methods. For a review of immunological and immunoassay procedures, see *Basic and Clinical Immunology* (1991). Moreover, the immunoassays of certain aspects can be performed in any of several configurations, which are reviewed extensively in Enzyme Immunoassay (1980); and Harlow & Lane, supra).

Immunoassays also often use a labeling agent to specifically bind to and label the complex formed by the antibody and antigen. The labeling agent may itself be one of the moieties comprising the antibody/antigen complex. Thus, the labeling agent may be a labeled polypeptide or a labeled antibody that binds the protein of interest. Alternatively, the labeling agent may be a third moiety, such as a secondary antibody, that specifically binds to the antibody/antigen complex (a secondary antibody is typically specific to antibodies of the species from which the first antibody is derived). Other proteins capable of specifically binding immunoglobulin constant regions, such as protein A or protein G may also be used as the labeling agent. These proteins exhibit a strong non-immunogenic reactivity with immunoglobulin constant regions from a variety of species (see, *e.g.,* Kronval *et al*., 1973; Akerstrom *et al*., 1985). The labeling agent can be modified with a detectable moiety, such as biotin, to which another molecule can specifically bind, such as streptavidin. A variety of detectable moieties are well known to those skilled in the art.

Commonly used assays include noncompetitive assays, e.g., sandwich assays, and competitive assays. In competitive assays, the amount of polypeptide present in the sample is measured indirectly by measuring the amount of a known, added (exogenous) polypeptide of interest displaced (competed away) from an antibody that binds by the unknown polypeptide present in a sample. Commonly used assay formats include immunoblots, which are used to detect and quantify the presence of protein in a sample. Other assay formats include liposome immunoassays (LIA), which use liposomes designed to bind specific molecules (*e.g*., antibodies) and release encapsulated reagents or markers. The released chemicals are then detected according to standard techniques (see Monroe *et al*., 1986).

Any suitable method can be used to detect one or more of the markers described herein. Successful practice can be achieved with one or a combination of methods that can detect and, preferably, quantify the markers. These methods include, without limitation, hybridization-based methods, including those employed in biochip arrays, mass spectrometry (e.g., laser desorption/ionization mass spectrometry), fluorescence (e.g. sandwich immunoassay), surface plasmon resonance, ellipsometry and atomic force microscopy. Expression levels of markers (e.g., polynucleotides or polypeptides) are compared by procedures well known in the art, such as RT-PCR, Northern blotting, Western blotting, flow cytometry, immunocytochemistry, binding to magnetic and/or antibody-coated beads, in situ hybridization, fluorescence in situ hybridization (FISH), flow chamber adhesion assay, ELISA, microarray analysis, or colorimetric assays. Methods may further include, one or more of electrospray ionization mass spectrometry (ESI-MS), ESI-MS/MS, ESI-MS/(MS)n, matrix-assisted laser desorption ionization time-of-flight mass spectrometry (MALDI-TOF-MS), surface-enhanced laser desorption/ionization time-of-flight mass spectrometry (SELDI-TOF-MS), desorption/ionization on silicon (DIOS), secondary ion mass spectrometry (SIMS), quadrupole time-of-flight (Q-TOF), atmospheric pressure chemical ionization mass spectrometry (APCI-MS), APCI-MS/MS, APCI-(MS)11, atmospheric pressure photoionization mass spectrometry (APPI-MS), APPI-MS/MS, and APPI-(MS)n, quadrupole mass spectrometry, fourier transform mass spectrometry (FTMS), and ion trap mass spectrometry, where n is an integer greater than zero.

Detection methods may include use of a biochip array. Biochip arrays include protein and polynucleotide arrays. One or more markers are captured on the biochip array and subjected to analysis to detect the level of the markers in a sample.

### VIII. Pharmaceutical Compositions

In certain aspects, the compositions or agents for use in the methods, such as known or test drugs for FSHD treatment, are suitably contained in a pharmaceutically acceptable carrier. The carrier is non-toxic, biocompatible and is selected so as not to detrimentally affect the biological activity of the agent. The agents in some aspects may be formulated into preparations for local delivery (i.e. to a specific location of the body, such as skeletal muscle or other tissue) or systemic delivery, in solid, semi-solid, gel, liquid or gaseous forms such as tablets, capsules, powders, granules, ointments, solutions, depositories, inhalants and injections allowing for oral, parenteral or surgical administration. Certain aspects also contemplate local administration of the compositions by coating medical devices and the like.

Suitable carriers for parenteral delivery via injectable, infusion or irrigation and topical delivery include distilled water, physiological phosphate-buffered saline, normal or lactated Ringer's solutions, dextrose solution, Hank's solution, or propanediol. In addition, sterile, fixed oils may be employed as a solvent or suspending medium. For this purpose any biocompatible oil may be employed including synthetic mono- or diglycerides. In addition, fatty acids such as oleic acid find use in the preparation of injectables. The carrier and agent may be compounded as a liquid, suspension, polymerizable or non-polymerizable gel, paste or salve.

The carrier may also comprise a delivery vehicle to sustain (i.e., extend, delay or regulate) the delivery of the agent(s) or to enhance the delivery, uptake, stability or pharmacokinetics of the therapeutic agent(s). Such a delivery vehicle may include, by way of non-limiting examples, microparticles, microspheres, nanospheres or nanoparticles composed of proteins, liposomes, carbohydrates, synthetic organic compounds, inorganic compounds, polymeric or copolymeric hydrogels and polymeric micelles.

In certain aspects, the actual dosage amount of a composition administered to a patient or subject can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject.

In certain aspects, pharmaceutical compositions may comprise, for example, at least about 0.1% of an active agent, such as an isolated exosome, a related lipid nanovesicle, or an exosome or nanovesicle loaded with therapeutic agents or diagnostic agents. In other aspects, the active agent may comprise between about 2% to about 75% of the weight of the unit, or between about 25% to about 60%, for example, and any range derivable therein. In other non-limiting examples, a dose may also comprise from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 5 microgram/kg/body weight to about 100 mg/kg/body weight, about 5 microgram/kg/body weight to about 500 milligram/kg/body weight, etc., can be administered.

Solutions of pharmaceutical compositions can be prepared in water suitably mixed with a surfactant, such as hydroxypropylcellulose. Dispersions also can be prepared in glycerol, liquid polyethylene glycols, mixtures thereof and in oils. Under ordinary conditions of storage and use, these preparations contain a preservative to prevent the growth of microorganisms.

In certain aspects, the pharmaceutical compositions are advantageously administered in the form of injectable compositions either as liquid solutions or suspensions; solid forms suitable for solution in, or suspension in, liquid prior to injection may also be prepared. These preparations also may be emulsified. A typical composition for such purpose comprises a pharmaceutically acceptable carrier. For instance, the composition may contain 10 mg or less, 25 mg, 50 mg or up to about 100 mg of human serum albumin per milliliter of phosphate buffered saline. Other pharmaceutically acceptable carriers include aqueous solutions, non-toxic excipients, including salts, preservatives, buffers and the like.

Examples of non-aqueous solvents are propylene glycol, polyethylene glycol, vegetable oil and injectable organic esters such as ethyloleate. Aqueous carriers include water, alcoholic/aqueous solutions, saline solutions, parenteral vehicles such as sodium chloride, Ringer's dextrose, etc. Intravenous vehicles include fluid and nutrient replenishers. Preservatives include antimicrobial agents, antifungal agents, anti-oxidants, chelating agents and inert gases. The pH and exact concentration of the various components the pharmaceutical composition are adjusted according to well-known parameters.

Additional formulations are suitable for oral administration. Oral formulations include such typical excipients as, for example, pharmaceutical grades of mannitol, lactose, starch, magnesium stearate, sodium saccharine, cellulose, magnesium carbonate and the like. The compositions take the form of solutions, suspensions, tablets, pills, capsules, sustained release formulations or powders.

In further aspects, the pharmaceutical compositions may include classic pharmaceutical preparations. Administration of pharmaceutical compositions according to certain aspects may be via any common route so long as the target tissue is available via that route. This may include oral, nasal, buccal, rectal, vaginal or topical. Alternatively, administration may be by orthotopic, intradermal, subcutaneous, intramuscular, intraperitoneal or intravenous injection. Such compositions would normally be administered as pharmaceutically acceptable compositions that include physiologically acceptable carriers, buffers or other excipients. Aerosol delivery can be used. Volume of the aerosol is between about 0.01 ml and 0.5 ml.

An effective amount of the pharmaceutical composition is determined based on the intended goal. The term "unit dose" or "dosage" refers to physically discrete units suitable for use in a subject, each unit containing a predetermined-quantity of the pharmaceutical composition calculated to produce the desired responses discussed above in association with its administration, *i.e*., the appropriate route and treatment regimen. The quantity to be administered, both according to number of treatments and unit dose, depends on the protection or effect desired.

Precise amounts of the pharmaceutical composition also depend on the judgment of the practitioner and are peculiar to each individual. Factors affecting the dose include the physical and clinical state of the patient, the route of administration, the intended goal of treatment (*e.g*., alleviation of symptoms versus cure) and the potency, stability and toxicity of the particular therapeutic substance.

### IX. KITS

Certain aspects also concern kits containing such as diagnostic and/or therapeutic kits, as well as kits for preparing and/or screening materials. In some aspects, kits can be used to evaluate one or more nucleic acids molecules or protein. In certain aspects, a kit contains, contains at least or contains at most 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 500, 1,000 or more nucleic acid probes, synthetic nucleic acid molecules, nucleic acid inhibitors, antibodies, or any value or range and combination derivable therein. In some aspects, there are kits for evaluating nucleic acid expression or protein expression, particular expression levels of one or more biomarkers in a cell.

Kits may comprise components, which may be individually packaged or placed in a container, such as a tube, bottle, vial, syringe, or other suitable container means.

Individual components may also be provided in a kit in concentrated amounts; in some aspects, a component is provided individually in the same concentration as it would be in a solution with other components. Concentrations of components may be provided as 1x, 2x, 5x, 10x, or 20x or more.

Kits for using nucleic acid probes, nucleic acids, nonsynthetic nucleic acids, and/or nucleic acid inhibitors for prognostic or diagnostic applications are provided. Specifically contemplated are any such molecules corresponding to any DUX4-related biomarker identified herein.

In certain aspects, negative and/or positive control synthetic nucleic acids and/or nucleic acid inhibitors are included in some kit aspects. The control molecules can be used to verify transfection efficiency and/or control for transfection-induced changes in cells.

It is contemplated that any method or composition described herein can be implemented with respect to any other method or composition described herein and that different aspects may be combined. The claims originally filed are contemplated to cover claims that are multiply dependent on any filed claim or combination of filed claims.

Certain aspects involving specific nucleic acids by name is contemplated also to cover aspects involving nucleic acids whose sequences are at least 80, 81, 82, 83, 84, 85, 86, 87, 88, 89, 90, 91, 92, 93, 94, 95, 96, 97, 98, 99% identical to the sequence of the specified nucleic acids (or any range or value derivable therein) listed in any of the tables.

Further aspects include kits for analysis of a biological or pathological sample by assessing biomarker profile for a sample comprising, in suitable container means, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 500, 1,000 nucleic acid probes or antibodies, wherein the nucleic acid probes or antibodies detect 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 500, 1,000 of the biomarker identified herein. The kit can further comprise reagents for labeling nucleic acids in the sample. The kit may also include labeling reagents, including at least one of amine-modified nucleotide, poly(A) polymerase, and poly(A) polymerase buffer. Labeling reagents can include an amine-reactive dye.

### X. EXAMPLES

### EXAMPLES 1- Materials and Methods

Transduction of muscle cells cultures with DUX4: The transduction of the 54-1 and MB135 human myoblast lines was previously described (Geng, et al., 2012). RNA was harvested after transduction by lenti-pgk-DUX4 or lenti-pgk-GFP and non-transduced cells as controls after 48 hrs (54-1) or 24 hrs (MB135).

Muscle cell cultures: Muscle cells derived from two FSHD1 and three FSHD2 were cultured and RNA was harvested from both myoblasts and myotubes. These cultures were described previously (Young, et al., 2013). As previously described, Primary myoblast cell lines were received from the University of Rochester biorepository (http://www.urmc.rochester.edu/fields-center) and were cultured in DMEM/F-10 media (Gibco) in the presence of 20% heat-inactivated fetal bovine serum (Gibco), 1% penicillin/streptomycin (Gibco). Media was supplemented with 10 ng/ml rhFGF (Promega) and 1 µM dexamethasone (SIGMA). Myoblasts were fused at 80% confluence in DMEM/F-12 Glutamax media containing 2% KnockOut serum replacement formulation (Gibco) for 36 hours.

Muscle Biopsy: All muscle biopsies were obtained at the University of Rochester Medical Center under an IRB-approved protocol. Muscle biopsy samples were obtained under local anesthesia by the minimally needle biopsy technique utilizing the modified Bergstrom needle as previously described (Welle, et al., 2004; Welle, et al., 2000). A muscle sample, adjacent to the sample used for RNA extraction, was oriented and mounted and then frozen in isopentane cooled in liquid nitrogen for histologic evaluation. A twelve point scale (0=normal muscle and 12=end stage muscle) was used for histopathologic grading of the sample based on: degree of muscle fiber variability, extent of central nucleation, presence of muscle fiber necrosis/regeneration and extent of interstitial fibrosis. (available through world wide web at http://www.urmc.rochester.edu/fields-center/protocols/PathologicSeverityScoring.cfm)

RNA-seq library preparation and sequencing: For all biological samples, RNA was extracted, poly(A) selected, and subjected to Illumina sequencing using standard protocols to generate 100bp single-end reads. Library preparation and sequencing was carried out by the FHCRC Genomics Shared Resource. Sequencing libraries were prepared from total RNA using the TruSeq RNA Sample Prep Kit (Illumina) according to the manufacturer's instructions. Library size distributions were validated using an Agilent 2100 Bioanalyzer. Additional library quality control, blending of pooled indexed libraries, and cluster optimization were performed using the QPCR NGS Library Quantization Kit (Agilent Technologies). Sequencing occurred on an Illumina HiSeq 2000 using a single-read, 100 base read length (SR100) sequencing strategy. RNA-seq datasets have been submitted to GEO **** [accession numbers to be provided].

RNA-seq data processing and analysis: Image analysis and base calling were performed with Illumina's Real Time Analysis v1.17.20 software. Files were demultiplexed of indexed reads and generated in FASTQ format using Illumina's CASAVA v1.8.2 software. Reads were removed that did not pass Illumina's base call quality threshold. Reads were aligned to human genome ensemble assembly GRCh37, using TopHat 2.0.8 (Trapnell et al., 2009). Read counts were collected for all genes using Bioconductor Rsamtools package. To include reads that mapped to multiple homologs in the genome, each hit of a read with multiple hits received a fractional count equal to one over the number of hits. Differential expressed genes were detected using Bioconductor package DESeq 1.14. Based on recommendation of DESeq package, to compare two groups of samples with fewer than six replicates for each condition, the "estimateDispersions" function and the "fit-only" sharing mode were used, so that dispersions were estimated based on local regression against mean values. For comparison of groups involving more than six replicates for each condition, the "maximum" sharing mode was used, which were the maximum of fitted values and per-gene estimates. To visualize gene expression clustering and to compute fold change differences, asinh transformation was applied to gene counts of each sample scaled by the corresponding size-factor. The adjusted log fold-change is computed as the difference of the average transformed values under the two conditions in comparison. The log fold-change computed this way has more regulated behavior at values close to or at zero, and approximates log fold-change for larger values. This approach is very similar to the variance-stabilization transformation (VST) method recommended by the most recent version of DESEQ. To make sure that differentially expressed genes were not driven by few extreme outliers, non-parametric Wilcoxon rank sum test was applied to samples in comparison as another filter. The p-value thresholds were selected based on the number of tested samples.

### EXAMPLES 2- DUX4 Targets as Biomarkers.

**DUX4 robustly induces a core set of genes in skeletal muscle cells.** To identify genes regulated by DUX4 in skeletal muscle cells, a muscle biopsy derived primary myoblast cell culture (MB135) from a control individual and an immortalized myoblast cell line (MB541) (Geng, et al., 2012; Krom, et al., 2012), were transduced with a D4Z4 repeat in the unaffected range, with a lentiviral vector expressing DUX4 or GFP as a control. RNA-seq identified 507 DUX4-up-regulated genes in MB135 and 643 in MB541 with 416 genes shared by the two cell types using stringent statistical criteria (FDR < 0.05, moderated log-fold-change > 1 (approximate linear fold change > 2.71 see Methods)). The fold-change of DUX4-regulated genes in the two cell types was highly concordant (FIG. 1A), including most genes that did not meet the statistical threshold in both populations, indicating that up-regulated genes specific to an individual cell type are mostly marginal cases near the cut-off for statistical significance rather than cell-type specific targets of DUX4. The most robust DUX4 targets, defined as the 228 up-regulated genes with a moderated log-fold-change greater than five (linear fold-change greater than 75) (FIG. 1A, purple data points and Table 3), were generally not expressed in the absence of DUX4 and were robustly induced by DUX4 in both muscle cell cultures (FIG. 1, B and C).

**Table 3**

| Ensembl | gene.name | X541Dux4_gfp.logFC | X541Dux4_NV.logFC | X135Dux4_gfp.logFC | X541Dux4_gfp.pval | X541Dux4_NV.pval | X135Dux4_gfp.pval | X54.1Dux4 | X54.1gfp | X54.1NV | MB135Dux4 | MB135gfp |
|---|---|---|---|---|---|---|---|---|---|---|---|---|
| ENSG00000144010 | TRIM43B | 14.4 | 14.4 | 14.3 | 1.90E-30 | 1.80E-30 | 4.20E-26 | 14.4 | 0 | 0 | 14.3 | 0 |
| ENSG00000257951 | RP11-554D14.4 | 14.3 | 14.3 | 14 | 2.50E-30 | 2.40E-30 | 2.30E-25 | 14.3 | 0 | 0 | 14 | 0 |
| ENSG00000144015 | TRIM43 | 14.3 | 14.3 | 14.3 | 3.40E-30 | 3.30E-30 | 5.40E-26 | 14.3 | 0 | 0 | 14.3 | 0 |
| ENSG00000157765 | SLC34A2 | 13.5 | 13.5 | 13.6 | 3.80E-28 | 3.70E-28 | 1.80E-24 | 13.5 | 0 | 0 | 13.6 | 0 |
| ENSG00000251258 | RFPL4B | 12.5 | 12.5 | 13.1 | 8.40E-28 | 8.00E-28 | 2.50E-24 | 13.9 | 1.4 | 1.4 | 13.8 | 0.7 |
| ENSG00000116726 | PRAMEF12 | 12.8 | 12.8 | 12.8 | 2.40E-26 | 2.30E-26 | 7.10E-23 | 12.8 | 0 | 0 | 12.8 | 0 |
| ENSG00000144188 | TRIM43CP | 12.8 | 12.8 | 12.8 | 2.40E-26 | 2.30E-26 | 7.90E-23 | 12.8 | 0 | 0 | 12.8 | 0 |
| ENSG00000204527 | DUXA | 12.6 | 12.6 | 11.6 | 7.70E-26 | 7.40E-26 | 4.90E-21 | 12.6 | 0 | 0 | 12.3 | 0.7 |
| ENSG00000180532 | ZSCAN4 | 11.5 | 12.5 | 12 | 9.80E-26 | 7.90E-27 | 1.80E-22 | 13.3 | 1.8 | 0.8 | 13.2 | 1.3 |
| ENSG00000248945 | RP11-432M8.17 | 12.3 | 12.3 | 12 | 4.20E-25 | 4.10E-25 | 3.40E-21 | 12.3 | 0 | 0 | 12 | 0 |
| ENSG00000120952 | PRAMEF2 | 12.3 | 12.3 | 12.6 | 5.20E-25 | 5.00E-25 | 2.50E-22 | 12.3 | 0 | 0 | 12.6 | 0 |
| ENSG00000230522 | MBD3L2 | 12.1 | 12.1 | 11.3 | 1.50E-24 | 1.50E-24 | 1.60E-19 | 12.1 | 0 | 0 | 11.3 | 0 |
| ENSG00000116721 | PRAMEF1 | 12.1 | 12.1 | 12.8 | 1.70E-24 | 1.60E-24 | 7.60E-23 | 12.1 | 0 | 0 | 12.8 | 0 |
| ENSG00000243501 | KHDC1L | 11 | 11.4 | 12.6 | 2.10E-24 | 6.80E-25 | 1.70E-22 | 12.8 | 1.8 | 1.4 | 12.6 | 0 |
| ENSG00000213921 | LEUTX | 12 | 12 | 12.1 | 2.20E-24 | 2.10E-24 | 3.40E-21 | 12 | 0 | 0 | 12.1 | 0 |
| ENSG00000249165 | RP11-321E2.13 | 12 | 12 | 11.6 | 3.50E-24 | 3.30E-24 | 3.40E-20 | 12 | 0 | 0 | 11.6 | 0 |
| ENSG00000182315 | MBD3L3 | 11.8 | 11.8 | 11.3 | 8.40E-24 | 8.10E-24 | 1.50E-19 | 11.8 | 0 | 0 | 11.3 | 0 |
| ENSG00000204495 | PRAMEF13 | 11.7 | 11.7 | 12.1 | 1.70E-23 | 1.60E-23 | 3.30E-21 | 11.7 | 0 | 0 | 12.1 | 0 |
| ENSG00000205718 | MBD3L4 | 11.7 | 11.7 | 11 | 1.70E-23 | 1.60E-23 | 5.10E-19 | 11.7 | 0 | 0 | 11 | 0 |
| ENSG00000223417 | TRIM49DP | 11.7 | 11.7 | 11.9 | 1.80E-23 | 1.80E-23 | 5.80E-21 | 11.7 | 0 | 0 | 11.9 | 0 |
| ENSG00000128253 | RFPL2 | 10.5 | 11.7 | 12 | 1.90E-23 | 6.20E-25 | 8.10E-22 | 12.6 | 2 | 0.8 | 12.7 | 0.7 |
| ENSG00000237247 | MBD3L5 | 11.7 | 11.7 | 10.9 | 1.90E-23 | 1.90E-23 | 1.10E-18 | 11.7 | 0 | 0 | 10.9 | 0 |
| ENSG00000204481 | PRAMEF14 | 11.6 | 11.6 | 12 | 2.50E-23 | 2.40E-23 | 3.90E-21 | 11.6 | 0 | 0 | 12 | 0 |
| ENSG00000196589 | AC010606.1 | 11.6 | 11.6 | 11.2 | 2.70E-23 | 2.60E-23 | 2.00E-19 | 11.6 | 0 | 0 | 11.2 | 0 |
| ENSG00000186223 | SSU72P4 | 11.6 | 11.6 | 11.3 | 2.90E-23 | 2.80E-23 | 1.20E-19 | 11.6 | 0 | 0 | 11.3 | 0 |
| ENSG00000255194 | SSU72P2 | 11.6 | 10.7 | 11.6 | 3.40E-23 | 2.30E-22 | 3.10E-20 | 11.6 | 0 | 0.8 | 11.6 | 0 |
| ENSG00000233802 | TRIM49L1 | 11.5 | 11.5 | 11.8 | 4.40E-23 | 4.20E-23 | 1.10E-20 | 11.5 | 0 | 0 | 11.8 | 0 |
| ENSG00000229542 | SSU72P7 | 11.4 | 11.4 | 11.1 | 1.30E-22 | 1.30E-22 | 3.40E-19 | 11.4 | 0 | 0 | 11.1 | 0 |
| ENSG00000237565 | SSU72P5 | 11.3 | 11.3 | 11.3 | 1.50E-22 | 1.40E-22 | 1.40E-19 | 11.3 | 0 | 0 | 11.3 | 0 |
| ENSG00000235268 | KDM4E | 11.3 | 11.3 | 12.2 | 1.90E-22 | 1.80E-22 | 2.10E-21 | 11.3 | 0 | 0 | 12.2 | 0 |
| ENSG00000236175 | SSU72P6 | 11.2 | 11.2 | 11 | 2.90E-22 | 2.80E-22 | 8.90E-19 | 11.2 | 0 | 0 | 11 | 0 |
| ENSG00000204510 | PRAMEF7 | 11.2 | 11.2 | 12 | 4.40E-22 | 4.20E-22 | 5.00E-21 | 11.2 | 0 | 0 | 12 | 0 |
| ENSG00000224199 | WI2-2994D6.1 | 11.1 | 11.1 | 12 | 5.50E-22 | 5.30E-22 | 5.20E-21 | 11.1 | 0 | 0 | 12 | 0 |
| ENSG00000133101 | CCNA1 | 9.6 | 7.9 | 7.2 | 6.20E-22 | 4.70E-18 | 7.10E-14 | 12.6 | 2.9 | 4.7 | 13 | 5.8 |
| ENSG00000229978 | RP13-221M14.3 | 11.1 | 11.1 | 11.8 | 7.90E-22 | 7.60E-22 | 1.10E-20 | 11.1 | 0 | 0 | 11.8 | 0 |
| ENSG00000204485 | XX-FW84067D5 .1 | 11 | 11 | 11.8 | 9.00E-22 | 8.70E-22 | 1.40E-20 | 11 | 0 | 0 | 11.8 | 0 |
| ENSG00000182330 | PRAMEF8 | 10.9 | 10.9 | 11.7 | 1.60E-21 | 1.50E-21 | 2.10E-20 | 10.9 | 0 | 0 | 11.7 | 0 |
| ENSG00000232423 | PRAMEF6 | 10.9 | 10.9 | 10.4 | 2.10E-21 | 2.10E-21 | 2.00E-18 | 10.9 | 0 | 0 | 11.1 | 0.7 |
| ENSG00000225581 | TRIM53AP | 10.9 | 10.9 | 11.4 | 2.30E-21 | 2.20E-21 | 8.10E-20 | 10.9 | 0 | 0 | 11.4 | 0 |
| ENSG00000168930 | TRIM49 | 10.9 | 10.9 | 11.3 | 2.60E-21 | 2.50E-21 | 1.30E-19 | 10.9 | 0 | 0 | 11.3 | 0 |
| ENSG00000204449 | TRIM49C | 10.9 | 10.9 | 11.3 | 2.70E-21 | 2.60E-21 | 1.40E-19 | 10.9 | 0 | 0 | 11.3 | 0 |
| ENSG00000251163 | CTD-2325A15.2 | 10.8 | 10.8 | 10.5 | 3.10E-21 | 2.90E-21 | 1.20E-17 | 10.8 | 0 | 0 | 10.5 | 0 |
| ENSG00000128250 | RFPL1 | 9.6 | 11.7 | 7.7 | 4.00E-21 | 1.90E-23 | 8.90E-15 | 11.7 | 2 | 0 | 12.4 | 4.7 |
| ENSG00000254104 | RP11-63E5.1 | 9.9 | 11.3 | 9.3 | 4.80E-21 | 1.70E-22 | 3.60E-17 | 11.3 | 1.4 | 0 | 11.2 | 1.9 |
| ENSG00000229571 | WI2-2994D6.2 | 10.7 | 10.7 | 10.7 | 6.60E-21 | 6.30E-21 | 2.40E-18 | 10.7 | 0 | 0 | 10.7 | 0 |
| ENSG00000223638 | RFPL4A | 9.2 | 9.7 | 11.4 | 1.30E-20 | 2.00E-21 | 6.60E-20 | 11.9 | 2.7 | 2.2 | 11.4 | 0 |
| ENSG00000229292 | CTD-2611012.2 | 9.4 | 9.5 | 10.8 | 1.30E-20 | 1.20E-20 | 1.80E-18 | 11.5 | 2 | 2 | 10.8 | 0 |
| ENSG00000166013 | TRIM53BP | 10.6 | 10.6 | 11 | 1.40E-20 | 1.30E-20 | 7.30E-19 | 10.6 | 0 | 0 | 11 | 0 |
| ENSG00000186232 | SSU72P3 | 10.5 | 10.5 | 10.6 | 1.80E-20 | 1.70E-20 | 5.50E-18 | 10.5 | 0 | 0 | 10.6 | 0 |
| ENSG00000225465 | RFPL1-AS1 | 9.2 | 11.6 | 6.8 | 2.90E-20 | 3.10E-23 | 6.50E-13 | 11.6 | 2.4 | 0 | 12.3 | 5.5 |
| ENSG00000249620 | RP11-321E2.10 | 10.5 | 10.5 | 10.9 | 3.10E-20 | 3.00E-20 | 1.10E-18 | 10.5 | 0 | 0 | 10.9 | 0 |
| ENSG00000204455 | TRIM51BP | 10.3 | 10.3 | 10.7 | 6.30E-20 | 6.10E-20 | 2.80E-18 | 10.3 | 0 | 0 | 10.7 | 0 |
| ENSG00000182053 | TRIM49B | 10.3 | 10.3 | 10.9 | 9.20E-20 | 8.90E-20 | 1.30E-18 | 10.3 | 0 | 0 | 10.9 | 0 |
| ENSG00000254764 | TRIM53CP | 10.2 | 10.2 | 10.7 | 1.50E-19 | 1.40E-19 | 4.10E-18 | 10.2 | 0 | 0 | 10.7 | 0 |
| ENSG00000237706 | TRIM51EP | 10.2 | 10.2 | 10.6 | 1.90E-19 | 1.80E-19 | 6.90E-18 | 10.2 | 0 | 0 | 10.6 | 0 |
| ENSG00000256779 | RP11-60C6.5 | 10.2 | 10.2 | 9.7 | 1.90E-19 | 1.90E-19 | 2.20E-17 | 10.2 | 0 | 0 | 10.9 | 1.3 |
| ENSG00000189348 | FAM90A27P | 10.1 | 10.1 | 11 | 2.60E-19 | 2.50E-19 | 6.20E-19 | 10.1 | 0 | 0 | 11 | 0 |
| ENSG00000204502 | PRAMEF5 | 10.1 | 10.1 | 10.4 | 2.80E-19 | 2.70E-19 | 1.60E-17 | 10.1 | 0 | 0 | 10.4 | 0 |
| ENSG00000250386 | RP11-432M8.11 | 10.1 | 10.1 | 10.7 | 3.10E-19 | 3.00E-19 | 3.80E-18 | 10.1 | 0 | 0 | 10.7 | 0 |
| ENSG00000249156 | RP11-432M8.9 | 10.1 | 10.1 | 11 | 3.20E-19 | 3.10E-19 | 7.10E-19 | 10.1 | 0 | 0 | 11 | 0 |
| ENSG00000157358 | PRAMEF15 | 10 | 10 | 10.4 | 4.50E-19 | 4.30E-19 | 1.50E-17 | 10 | 0 | 0 | 10.4 | 0 |
| ENSG00000179412 | RP13-221M14.5 | 10 | 10 | 10 | 4.80E-19 | 4.60E-19 | 1.10E-16 | 10 | 0 | 0 | 10 | 0 |
| ENSG00000204501 | PRAMEF9 | 10 | 10 | 10.4 | 5.60E-19 | 5.40E-19 | 1.60E-17 | 10 | 0 | 0 | 10.4 | 0 |
| ENSG00000237194 | SNAI1 P1 | 9.8 | 9.8 | 9.8 | 1.80E-18 | 1.80E-18 | 2.50E-16 | 9.8 | 0 | 0 | 9.8 | 0 |
| ENSG00000256980 | KHDC1L | 8.1 | 8.2 | 8.5 | 1.90E-18 | 9.10E-19 | 1.60E-16 | 12.8 | 4.7 | 4.6 | 12.7 | 4.2 |
| ENSG00000179172 | HNRNPCL1 | 9.8 | 9.8 | 10.2 | 2.30E-18 | 2.30E-18 | 5.50E-17 | 9.8 | 0 | 0 | 10.2 | 0 |
| ENSG00000226185 | TRIM64FP | 9.7 | 8.9 | 10 | 4.00E-18 | 2.70E-17 | 1.50E-16 | 9.7 | 0 | 0.8 | 10 | 0 |
| ENSG00000243073 | PRAMEF4 | 9.7 | 9.7 | 9.7 | 4.40E-18 | 4.20E-18 | 6.30E-16 | 9.7 | 0 | 0 | 9.7 | 0 |
| ENSG00000204513 | PRAMEF11 | 9.6 | 9.6 | 10 | 5.40E-18 | 5.20E-18 | 1.20E-16 | 9.6 | 0 | 0 | 10 | 0 |
| ENSG00000249357 | RP11-432M8.8 | 9.6 | 9.6 | 10.4 | 6.60E-18 | 6.30E-18 | 1.80E-17 | 9.6 | 0 | 0 | 10.4 | 0 |
| ENSG00000214534 | ZNF705E | 8.1 | 8.5 | 7.7 | 7.30E-18 | 1.50E-18 | 1.60E-14 | 11 | 2.9 | 2.6 | 11.2 | 3.5 |
| ENSG00000150244 | TRIM48 | 9.5 | 9.5 | 10.6 | 1.70E-17 | 1.60E-17 | 6.60E-18 | 9.5 | 0 | 0 | 10.6 | 0 |
| ENSG00000215372 | ZNF705G | 8 | 7.7 | 9 | 1.90E-17 | 8.20E-17 | 6.30E-16 | 10.6 | 2.6 | 2.9 | 10.3 | 1.3 |
| ENSG00000178928 | TPRX1 | 9.4 | 9.4 | 9.3 | 2.20E-17 | 2.10E-17 | 4.90E-15 | 9.4 | 0 | 0 | 9.3 | 0 |
| ENSG00000251360 | KHDC1P1 | 9.4 | 9.4 | 9.8 | 2.60E-17 | 2.50E-17 | 5.20E-16 | 9.4 | 0 | 0 | 9.8 | 0 |
| ENSG00000204505 | RP13-221M14.1 | 9.3 | 9.3 | 9.4 | 4.10E-17 | 3.90E-17 | 2.70E-15 | 9.3 | 0 | 0 | 9.4 | 0 |
| ENSG00000128276 | RFPL3 | 7.9 | 8.6 | 8.5 | 5.10E-17 | 4.60E-18 | 9.40E-15 | 10.4 | 2.4 | 1.7 | 9.7 | 1.3 |
| ENSG00000124900 | TRIM51 | 9.3 | 9.3 | 9.5 | 5.90E-17 | 5.70E-17 | 2.40E-15 | 9.3 | 0 | 0 | 9.5 | 0 |
| ENSG00000214325 | AC025287.1 | 8.3 | 9.6 | 9 | 1.50E-16 | 5.40E-18 | 4.30E-15 | 9.6 | 1.4 | 0 | 9.7 | 0.7 |
| ENSG00000141946 | ZIM3 | 9.1 | 9.1 | 8.6 | 1.70E-16 | 1.60E-16 | 1.50E-13 | 9.1 | 0 | 0 | 8.6 | 0 |
| ENSG00000176797 | DEFB103A | 9 | 9 | 7.1 | 2.80E-16 | 2.70E-16 | 5.20E-11 | 9 | 0 | 0 | 7.8 | 0.7 |
| ENSG00000177243 | DEFB103B | 9 | 9 | 7.1 | 2.80E-16 | 2.70E-16 | 5.20E-11 | 9 | 0 | 0 | 7.8 | 0.7 |
| ENSG00000204478 | PRAMEF20 | 9 | 9 | 9.3 | 2.80E-16 | 2.70E-16 | 5.80E-15 | 9 | 0 | 0 | 9.3 | 0 |
| ENSG00000239810 | WI2-3308P17.2 | 9 | 9 | 9.8 | 3.10E-16 | 3.00E-16 | 4.20E-16 | 9 | 0 | 0 | 9.8 | 0 |
| ENSG00000204486 | PRAMEF21 | 9 | 9 | 9.3 | 3.80E-16 | 3.70E-16 | 6.80E-15 | 9 | 0 | 0 | 9.3 | 0 |
| ENSG00000187545 | PRAMEF10 | 8.9 | 8.9 | 9.7 | 6.40E-16 | 6.20E-16 | 7.70E-16 | 8.9 | 0 | 0 | 9.7 | 0 |
| ENSG00000205853 | RFPL3-AS1 | 7.4 | 8.4 | 7.8 | 6.90E-16 | 1.90E-17 | 5.90E-14 | 10.1 | 2.7 | 1.7 | 9.7 | 1.9 |
| ENSG00000250782 | RP11-432M8.13 | 8.9 | 8.9 | 10 | 7.90E-16 | 7.60E-16 | 1.50E-16 | 8.9 | 0 | 0 | 10 | 0 |
| ENSG00000219061 | TRIM51FP | 8.9 | 8.9 | 8.9 | 8.50E-16 | 8.20E-16 | 7.00E-14 | 8.9 | 0 | 0 | 8.9 | 0 |
| ENSG00000131864 | USP29 | 8.8 | 8.8 | 8.8 | 1.00E-15 | 9.70E-16 | 6.70E-14 | 8.8 | 0 | 0 | 8.8 | 0 |
| ENSG00000104267 | CA2 | 8.8 | 8.8 | 10 | 1.30E-15 | 1.20E-15 | 9.30E-17 | 8.8 | 0 | 0 | 10 | 0 |
| ENSG00000204532 | ZSCAN5C | 7.4 | 6.6 | 8.4 | 4.00E-15 | 8.50E-14 | 2.90E-15 | 9.4 | 2 | 2.8 | 10.3 | 1.9 |
| ENSG00000255855 | RP11-735A19.2 | 8.6 | 8.6 | 10 | 4.10E-15 | 4.00E-15 | 1.80E-16 | 8.6 | 0 | 0 | 10 | 0 |
| ENSG00000237700 | RP11-219C24.6 | 8.6 | 8.6 | 9 | 4.50E-15 | 4.30E-15 | 2.00E-14 | 8.6 | 0 | 0 | 9 | 0 |
| ENSG00000224581 | XX-FW84067D5 .2 | 8.6 | 8.6 | 9.1 | 5.30E-15 | 5.20E-15 | 1.70E-14 | 8.6 | 0 | 0 | 9.1 | 0 |
| ENSG00000197213 | ZSCAN5B | 7.4 | 7.8 | 7.6 | 7.60E-15 | 2.50E-15 | 2.00E-13 | 9.2 | 1.8 | 1.4 | 9.5 | 1.9 |
| ENSG00000249666 | RP11-432M8.12 | 8.5 | 8.5 | 9.5 | 8.10E-15 | 7.80E-15 | 1.80E-15 | 8.5 | 0 | 0 | 9.5 | 0 |
| ENSG00000241576 | DUX4L11 | 8.4 | 8.4 | 7.1 | 2.20E-14 | 2.20E-14 | 3.10E-10 | 8.4 | 0 | 0 | 7.1 | 0 |
| ENSG00000236462 | DUX4L13 | 8.4 | 8.4 | 7.1 | 2.70E-14 | 2.60E-14 | 3.50E-10 | 8.4 | 0 | 0 | 7.1 | 0 |
| ENSG00000243653 | DUX4L14 | 8.4 | 8.4 | 7.1 | 3.00E-14 | 2.90E-14 | 4.00E-10 | 8.4 | 0 | 0 | 7.1 | 0 |
| ENSG00000249516 | CTD-2201E18.2 | 8.3 | 8.3 | 7.8 | 3.70E-14 | 3.50E-14 | 2.40E-11 | 8.3 | 0 | 0 | 7.8 | 0 |
| ENSG00000223913 | DUX4L12 | 8.3 | 8.3 | 6.9 | 4.90E-14 | 4.70E-14 | 1.00E-09 | 8.3 | 0 | 0 | 6.9 | 0 |
| ENSG00000196946 | ZNF705A | 6.6 | 6.8 | 7.2 | 5.40E-14 | 2.30E-14 | 2.30E-13 | 9.7 | 3.1 | 2.9 | 10.4 | 3.1 |
| ENSG00000124216 | SNAI1 | 6.9 | 8.1 | 6.6 | 7.90E-14 | 2.80E-15 | 4.70E-12 | 9 | 2 | 0.8 | 9.9 | 3.3 |
| ENSG00000170684 | ZNF296 | 6.4 | 6.5 | 5.2 | 1.60E-13 | 1.00E-13 | 2.90E-09 | 9.6 | 3.2 | 3.1 | 9.8 | 4.6 |
| ENSG00000239275 | RP4-675G8.2 | 8.1 | 8.1 | 8.5 | 1.70E-13 | 1.70E-13 | 9.70E-13 | 8.1 | 0 | 0 | 8.5 | 0 |
| ENSG00000187569 | DPPA3 | 8.1 | 8.1 | 6.9 | 1.80E-13 | 1.70E-13 | 1.30E-09 | 8.1 | 0 | 0 | 6.9 | 0 |
| ENSG00000169548 | ZNF280A | 6.3 | 5.8 | 5.5 | 1.90E-13 | 2.50E-12 | 1.70E-09 | 9.9 | 3.6 | 4.1 | 8.4 | 3 |
| ENSG00000189253 | TRIM64B | 8.1 | 8.1 | 9.1 | 2.00E-13 | 1.90E-13 | 2.70E-14 | 8.1 | 0 | 0 | 9.1 | 0 |
| ENSG00000214754 | AC004870.5 | 8.1 | 8.1 | 8.9 | 2.60E-13 | 2.50E-13 | 8.60E-15 | 8.1 | 0 | 0 | 9.6 | 0.7 |
| ENSG00000215356 | ZNF705B | 8.1 | 8.1 | 8.4 | 2.70E-13 | 2.60E-13 | 3.40E-12 | 8.1 | 0 | 0 | 8.4 | 0 |
| ENSG00000236217 | C1DP2 | 8.1 | 8.1 | 6.3 | 2.70E-13 | 2.60E-13 | 8.10E-10 | 8.1 | 0 | 0 | 7.9 | 1.6 |
| ENSG00000215343 | ZNF705D | 7.5 | 7.5 | 7.8 | 2.80E-13 | 2.70E-13 | 5.20E-12 | 8.3 | 0.8 | 0.8 | 8.6 | 0.7 |
| ENSG00000249910 | TRIM51CP | 8 | 8 | 8.4 | 3.10E-13 | 3.00E-13 | 1.30E-12 | 8 | 0 | 0 | 8.4 | 0 |
| ENSG00000250313 | RP11-5P22.3 | 8 | 8 | 8.6 | 3.30E-13 | 3.20E-13 | 5.50E-13 | 8 | 0 | 0 | 8.6 | 0 |
| ENSG00000166007 | TRIM51HP | 7.9 | 7.9 | 8.1 | 7.70E-13 | 7.50E-13 | 9.20E-12 | 7.9 | 0 | 0 | 8.1 | 0 |
| ENSG00000185041 | RP11-321E2.6 | 7.9 | 7.9 | 9.1 | 1.00E-12 | 1.00E-12 | 2.00E-14 | 7.9 | 0 | 0 | 9.1 | 0 |
| ENSG00000235800 | RP11-219C24.9 | 7.8 | 7.8 | 8 | 1.50E-12 | 1.50E-12 | 8.30E-12 | 7.8 | 0 | 0 | 8 | 0 |
| ENSG00000204450 | TRIM64 | 7.8 | 7.8 | 8.8 | 2.40E-12 | 2.30E-12 | 2.10E-13 | 7.8 | 0 | 0 | 8.8 | 0 |
| ENSG00000171872 | KLF17 | 6 | 5.1 | 6.1 | 2.80E-12 | 2.40E-10 | 9.40E-11 | 9.1 | 3.1 | 4 | 9.3 | 3.2 |
| ENSG00000135063 | FAM189A2 | 5.8 | 6.3 | 6.4 | 3.00E-12 | 3.60E-13 | 2.20E-11 | 9.5 | 3.6 | 3.2 | 9 | 2.6 |
| ENSG00000183508 | FAM46C | 6.1 | 5.1 | 5.6 | 5.50E-12 | 3.40E-10 | 7.40E-10 | 8.6 | 2.6 | 3.5 | 9 | 3.4 |
| ENSG00000230268 | SSU72P8 | 7.6 | 7.6 | 9.9 | 7.50E-12 | 7.20E-12 | 3.40E-16 | 7.6 | 0 | 0 | 9.9 | 0 |
| ENSG00000220948 | TRIM51GP | 7.6 | 7.6 | 7.5 | 9.00E-12 | 8.70E-12 | 1.90E-10 | 7.6 | 0 | 0 | 7.5 | 0 |
| ENSG00000145708 | CRHBP | 7.6 | 6.8 | 7.9 | 9.20E-12 | 5.50E-11 | 1.10E-11 | 7.6 | 0 | 0.8 | 7.9 | 0 |
| ENSG00000197123 | ZNF679 | 7.6 | 7.6 | 7.2 | 1.10E-11 | 1.10E-11 | 4.70E-09 | 7.6 | 0 | 0 | 7.2 | 0 |
| ENSG00000251402 | FAM90A25P | 7.5 | 6.7 | 6.1 | 1.60E-11 | 9.70E-11 | 1.10E-09 | 7.5 | 0 | 0.8 | 8.2 | 2.1 |
| ENSG00000205293 | RP11-1112C15.1 | 6.9 | 7.7 | 6.1 | 2.10E-11 | 3.30E-12 | 6.30E-09 | 7.7 | 0.8 | 0 | 7.8 | 1.6 |
| ENSG00000215156 | RP11-1023L17.2 | 6.3 | 7.2 | 5.3 | 2.40E-11 | 2.30E-12 | 3.50E-08 | 8 | 1.8 | 0.8 | 7.8 | 2.4 |
| ENSG00000218014 | KRT19P1 | 7.4 | 7.4 | 7.8 | 2.60E-11 | 2.50E-11 | 4.40E-11 | 7.4 | 0 | 0 | 7.8 | 0 |
| ENSG00000235950 | DUX4L15 | 7.4 | 7.4 | 7 | 2.80E-11 | 2.70E-11 | 6.60E-10 | 7.4 | 0 | 0 | 7 | 0 |
| ENSG00000226311 | DUX4L10 | 7.4 | 7.4 | 6.8 | 2.90E-11 | 2.80E-11 | 1.30E-09 | 7.4 | 0 | 0 | 6.8 | 0 |
| ENSG00000233834 | AC005083.1 | 6.2 | 6.2 | 5.9 | 3.00E-11 | 2.90E-11 | 3.80E-09 | 8 | 1.8 | 1.7 | 7.5 | 1.6 |
| ENSG00000255214 | RP11-163019.10 | 7.4 | 7.4 | 8 | 3.20E-11 | 3.10E-11 | 1.50E-11 | 7.4 | 0 | 0 | 8 | 0 |
| ENSG00000180828 | BHLHE22 | 7.4 | 7.4 | 6.9 | 3.90E-11 | 3.80E-11 | 1.90E-08 | 7.4 | 0 | 0 | 6.9 | 0 |
| ENSG00000163286 | ALPPL2 | 7.4 | 7.4 | 8.3 | 4.20E-11 | 4.00E-11 | 6.30E-12 | 7.4 | 0 | 0 | 8.3 | 0 |
| ENSG00000204480 | PRAMEF19 | 7.4 | 7.4 | 5.7 | 4.90E-11 | 4.80E-11 | 3.10E-05 | 7.4 | 0 | 0 | 5.7 | 0 |
| ENSG00000173110 | HSPA6 | 6.4 | 5.1 | 6.8 | 5.10E-11 | 3.60E-09 | 9.90E-10 | 7.8 | 1.4 | 2.7 | 7.5 | 0.7 |
| ENSG00000227476 | DUX4L5 | 7.3 | 7.3 | 7 | 6.60E-11 | 6.40E-11 | 4.80E-10 | 7.3 | 0 | 0 | 7 | 0 |
| ENSG00000227904 | DUX4L2 | 7.3 | 7.3 | 7 | 6.60E-11 | 6.40E-11 | 4.80E-10 | 7.3 | 0 | 0 | 7 | 0 |
| ENSG00000228188 | DUX4L6 | 7.3 | 7.3 | 7 | 6.90E-11 | 6.70E-11 | 4.80E-10 | 7.3 | 0 | 0 | 7 | 0 |
| ENSG00000256130 | DUX4 | 7.3 | 7.3 | 7 | 6.90E-11 | 6.70E-11 | 4.80E-10 | 7.3 | 0 | 0 | 7 | 0 |
| ENSG00000228114 | DUX4L3 | 7.3 | 7.3 | 7 | 7.00E-11 | 6.80E-11 | 4.80E-10 | 7.3 | 0 | 0 | 7 | 0 |
| ENSG00000163508 | EOMES | 5.7 | 5.7 | 8.1 | 1.20E-10 | 1.10E-10 | 1.40E-11 | 8.1 | 2.4 | 2.4 | 8.1 | 0 |
| ENSG00000225899 | FRG2B | 7.2 | 7.2 | 9.2 | 1.20E-10 | 1.20E-10 | 9.20E-15 | 7.2 | 0 | 0 | 9.2 | 0 |
| ENSG00000256477 | DUX4L4 | 7.2 | 7.2 | 6.8 | 1.30E-10 | 1.20E-10 | 1.60E-09 | 7.2 | 0 | 0 | 6.8 | 0 |
| ENSG00000172969 | FRG2C | 7.2 | 7.2 | 9.4 | 1.60E-10 | 1.50E-10 | 3.00E-15 | 7.2 | 0 | 0 | 9.4 | 0 |
| ENSG00000250537 | DUX4L8 | 7.2 | 7.2 | 6.9 | 1.70E-10 | 1.70E-10 | 7.00E-10 | 7.2 | 0 | 0 | 6.9 | 0 |
| ENSG00000251586 | TET2-AS1 | 6.6 | 7.4 | 5.9 | 1.70E-10 | 2.60E-11 | 2.70E-08 | 7.4 | 0.8 | 0 | 7.5 | 1.6 |
| ENSG00000205035 | RP11-707M1.1 | 7.2 | 7.2 | 7.4 | 2.00E-10 | 1.90E-10 | 8.00E-10 | 7.2 | 0 | 0 | 7.4 | 0 |
| ENSG00000248645 | RP11-366M4.6 | 6.6 | 6.6 | 6.9 | 2.00E-10 | 1.90E-10 | 7.00E-10 | 7.4 | 0.8 | 0.8 | 7.6 | 0.7 |
| ENSG00000238162 | AC009237.4 | 7.2 | 7.2 | 7.4 | 2.30E-10 | 2.20E-10 | 2.10E-09 | 7.2 | 0 | 0 | 7.4 | 0 |
| ENSG00000251076 | RP11-526F3.1 | 7.1 | 7.1 | 7.5 | 2.60E-10 | 2.60E-10 | 5.40E-11 | 7.1 | 0 | 0 | 7.5 | 0 |
| ENSG00000256830 | DUX2 | 6.5 | 7.3 | 7 | 4.90E-10 | 8.00E-11 | 6.40E-10 | 7.3 | 0.8 | 0 | 7 | 0 |
| ENSG00000163624 | CDS1 | 5.5 | 5.3 | 5.1 | 5.70E-10 | 9.70E-10 | 4.50E-07 | 7.9 | 2.4 | 2.6 | 7.2 | 2.1 |
| ENSG00000250891 | CTD-2281M20.1 | 7.1 | 7.1 | 7.8 | 5.70E-10 | 5.50E-10 | 1.90E-10 | 7.1 | 0 | 0 | 7.8 | 0 |
| ENSG00000254751 | TRIM64DP | 7 | 7 | 7 | 6.80E-10 | 6.50E-10 | 2.10E-08 | 7 | 0 | 0 | 7 | 0 |
| ENSG00000121966 | CXCR4 | 7 | 7 | 6.6 | 7.60E-10 | 7.40E-10 | 8.30E-11 | 7 | 0 | 0 | 8.5 | 1.9 |
| ENSG00000250946 | RP11-358N4.6 | 7 | 7 | 7.3 | 8.90E-10 | 8.60E-10 | 6.50E-10 | 7 | 0 | 0 | 7.3 | 0 |
| ENSG00000187912 | CLEC17A | 5.8 | 6.7 | 5.3 | 9.40E-10 | 9.40E-11 | 3.20E-07 | 7.5 | 1.8 | 0.8 | 6.9 | 1.6 |
| ENSG00000249092 | CTD-2325A15.3 | 7 | 7 | 5.4 | 9.70E-10 | 9.40E-10 | 6.80E-07 | 7 | 0 | 0 | 6.1 | 0.7 |
| ENSG00000144406 | UNC80 | 5.7 | 5.2 | 6.8 | 1.40E-09 | 6.20E-09 | 1.30E-08 | 7.5 | 1.8 | 2.2 | 6.8 | 0 |
| ENSG00000205097 | FRG2 | 6.9 | 6.9 | 9.1 | 1.70E-09 | 1.60E-09 | 1.70E-14 | 6.9 | 0 | 0 | 9.1 | 0 |
| ENSG00000206172 | HBA1 | 5.5 | 6.7 | 7.6 | 2.00E-09 | 8.70E-11 | 6.20E-13 | 7.5 | 2 | 0.8 | 8.9 | 1.3 |
| ENSG00000255184 | RP11-31312.7 | 6.8 | 6.8 | 7.1 | 2.90E-09 | 2.80E-09 | 3.50E-09 | 6.8 | 0 | 0 | 7.1 | 0 |
| ENSG00000257012 | RP11-60C6.6 | 6.8 | 6.8 | 6.8 | 3.10E-09 | 3.00E-09 | 1.20E-08 | 6.8 | 0 | 0 | 6.8 | 0 |
| ENSG00000249068 | CTC-28708.1 | 5.4 | 6 | 5.7 | 4.10E-09 | 6.60E-10 | 8.20E-07 | 7.4 | 2 | 1.4 | 6.9 | 1.3 |
| ENSG00000232783 | AC073135.3 | 6.8 | 6.8 | 6.8 | 4.50E-09 | 4.30E-09 | 1.70E-08 | 6.8 | 0 | 0 | 6.8 | 0 |
| ENSG00000205622 | AF064858.6 | 6.2 | 7 | 8.5 | 5.10E-09 | 8.60E-10 | 2.90E-12 | 7 | 0.8 | 0 | 8.5 | 0 |
| ENSG00000224807 | DUX4L9 | 6.1 | 7 | 6.6 | 5.80E-09 | 9.90E-10 | 6.10E-09 | 7 | 0.8 | 0 | 6.6 | 0 |
| ENSG00000205879 | FAM90A2P | 5.1 | 6.3 | 5.6 | 6.00E-09 | 1.40E-10 | 4.30E-09 | 7.6 | 2.6 | 1.4 | 8.3 | 2.7 |
| ENSG00000188536 | HBA2 | 5.2 | 6.6 | 7.6 | 7.00E-09 | 1.50E-10 | 6.00E-13 | 7.4 | 2.2 | 0.8 | 8.9 | 1.3 |
| ENSG00000256703 | RP11-266K4.7 | 5 | 6.2 | 6.7 | 7.00E-09 | 1.60E-10 | 2.50E-10 | 7.6 | 2.6 | 1.4 | 8.3 | 1.6 |
| ENSG00000160505 | NLRP4 | 6.6 | 6.6 | 6.8 | 1.50E-08 | 1.40E-08 | 1.00E-08 | 6.6 | 0 | 0 | 6.8 | 0 |
| ENSG00000250807 | RP11-432M8.2 | 6.6 | 6.6 | 7.7 | 2.00E-08 | 2.00E-08 | 1.40E-10 | 6.6 | 0 | 0 | 7.7 | 0 |
| ENSG00000215339 | ZNF705C | 6 | 6.8 | 6.3 | 2.30E-08 | 4.00E-09 | 2.40E-07 | 6.8 | 0.8 | 0 | 7 | 0.7 |
| ENSG00000233518 | RP11-73H14.1 | 6.6 | 6.6 | 7.5 | 2.30E-08 | 2.20E-08 | 1.70E-10 | 6.6 | 0 | 0 | 8.2 | 0.7 |
| ENSG00000234064 | RP11-45011.2 | 6.6 | 6.6 | 6.7 | 2.30E-08 | 2.20E-08 | 1.80E-08 | 6.6 | 0 | 0 | 6.7 | 0 |
| ENSG00000188460 | ACTBP11 | 6 | 5.4 | 6.1 | 2.50E-08 | 7.80E-08 | 4.10E-07 | 6.8 | 0.8 | 1.4 | 6.1 | 0 |
| ENSG00000245870 | RP11-457P14.4 | 6.5 | 6.5 | 6.1 | 3.40E-08 | 3.30E-08 | 3.10E-06 | 6.5 | 0 | 0 | 6.1 | 0 |
| ENSG00000237635 | XX-2136C48.8 | 6.5 | 6.5 | 6 | 3.50E-08 | 3.40E-08 | 1.20E-07 | 6.5 | 0 | 0 | 6 | 0 |
| ENSG00000231219 | RP11-219C24.8 | 6.5 | 6.5 | 6.8 | 5.90E-08 | 5.70E-08 | 5.50E-09 | 6.5 | 0 | 0 | 6.8 | 0 |
| ENSG00000204491 | PRAMEF18 | 6.4 | 6.4 | 5.8 | 1.50E-07 | 1.40E-07 | 2.30E-05 | 6.4 | 0 | 0 | 5.8 | 0 |
| ENSG00000224760 | C1DP3 | 6.3 | 6.3 | 5.9 | 2.30E-07 | 2.20E-07 | 1.60E-05 | 6.3 | 0 | 0 | 5.9 | 0 |
| ENSG00000163283 | ALPP | 6.2 | 6.2 | 5.7 | 3.70E-07 | 3.60E-07 | 1.20E-05 | 6.2 | 0 | 0 | 6.4 | 0.7 |
| ENSG00000250558 | RP11-432M8.3 | 6.2 | 6.2 | 7.3 | 3.70E-07 | 3.60E-07 | 1.90E-09 | 6.2 | 0 | 0 | 7.3 | 0 |
| ENSG00000253496 | RP11-13N12.1 | 5.6 | 6.4 | 5.7 | 4.80E-07 | 9.20E-08 | 6.00E-07 | 6.4 | 0.8 | 0 | 7 | 1.3 |
| ENSG00000205044 | RP11-56P9.11 | 6.2 | 6.2 | 7.5 | 5.40E-07 | 5.30E-07 | 3.70E-10 | 6.2 | 0 | 0 | 7.5 | 0 |
| ENSG00000214244 | SETP21 | 6.2 | 6.2 | 6.8 | 5.40E-07 | 5.30E-07 | 1.70E-07 | 6.2 | 0 | 0 | 6.8 | 0 |
| ENSG00000229361 | UBTFL7 | 6.2 | 6.2 | 6.7 | 5.80E-07 | 5.70E-07 | 3.90E-07 | 6.2 | 0 | 0 | 6.7 | 0 |
| ENSG00000254828 | RP11-72M10.5 | 6.2 | 6.2 | 6.2 | 6.00E-07 | 5.90E-07 | 3.60E-06 | 6.2 | 0 | 0 | 6.2 | 0 |
| ENSG00000215354 | FAM90A24P | 5.5 | 6.3 | 5.4 | 7.90E-07 | 1.50E-07 | 1.70E-06 | 6.3 | 0.8 | 0 | 6.1 | 0.7 |
| ENSG00000216316 | RP3-354N19.3 | 6.1 | 6.1 | 5.8 | 9.40E-07 | 9.10E-07 | 1.30E-05 | 6.1 | 0 | 0 | 5.8 | 0 |
| ENSG00000172482 | AGXT | 6.1 | 6.1 | 5.5 | 1.20E-06 | 1.20E-06 | 2.90E-06 | 6.1 | 0 | 0 | 6.7 | 1.3 |
| ENSG00000231103 | RP5-845024.3 | 6.1 | 6.1 | 5.5 | 1.20E-06 | 1.20E-06 | 1.40E-06 | 6.1 | 0 | 0 | 6.3 | 0.7 |
| ENSG00000258084 | RP11-754N21.1 | 6.1 | 6.1 | 5.5 | 1.30E-06 | 1.30E-06 | 2.30E-05 | 6.1 | 0 | 0 | 5.5 | 0 |
| ENSG00000107447 | DNTT | 6.1 | 6.1 | 6 | 1.40E-06 | 1.40E-06 | 6.50E-06 | 6.1 | 0 | 0 | 6 | 0 |
| ENSG00000188831 | DPPA3P2 | 6 | 6 | 5.3 | 1.80E-06 | 1.80E-06 | 2.20E-05 | 6 | 0 | 0 | 5.3 | 0 |
| ENSG00000255065 | RP11-680E19.2 | 5.4 | 6.2 | 6.5 | 1.80E-06 | 3.70E-07 | 1.50E-06 | 6.2 | 0.8 | 0 | 6.5 | 0 |
| ENSG00000249329 | RP11-432M8.5 | 6 | 6 | 7 | 1.90E-06 | 1.90E-06 | 1.10E-08 | 6 | 0 | 0 | 7 | 0 |
| ENSG00000226968 | LINC00423 | 6 | 6 | 5.9 | 2.20E-06 | 2.10E-06 | 6.90E-05 | 6 | 0 | 0 | 5.9 | 0 |
| ENSG00000218725 | RP11-174C7.2 | 6 | 6 | 8.4 | 2.90E-06 | 2.80E-06 | 1.30E-11 | 6 | 0 | 0 | 8.4 | 0 |
| ENSG00000248205 | RP11-321E2.11 | 5.9 | 5.9 | 5.3 | 3.60E-06 | 3.60E-06 | 0.00015 | 5.9 | 0 | 0 | 5.3 | 0 |
| ENSG00000250055 | RP11-321E2.8 | 5.9 | 5.9 | 6.9 | 3.60E-06 | 3.60E-06 | 2.50E-08 | 5.9 | 0 | 0 | 6.9 | 0 |
| ENSG00000211530 | AL354933.1 | 5.9 | 5.9 | 6.2 | 4.20E-06 | 4.10E-06 | 4.70E-06 | 5.9 | 0 | 0 | 6.2 | 0 |
| ENSG00000168126 | OR2W6P | 5.3 | 6.1 | 6.3 | 5.00E-06 | 1.10E-06 | 4.20E-07 | 6.1 | 0.8 | 0 | 7.1 | 0.7 |
| ENSG00000254229 | FAM90A12P | 5.2 | 6.1 | 5.2 | 5.20E-06 | 1.10E-06 | 1.20E-05 | 6.1 | 0.8 | 0 | 5.9 | 0.7 |
| ENSG00000248861 | RP11-321E2.2 | 5.8 | 5.8 | 6.9 | 7.20E-06 | 7.10E-06 | 3.40E-08 | 5.8 | 0 | 0 | 6.9 | 0 |
| ENSG00000257954 | RP11-161H23.10 | 5.2 | 6 | 5.6 | 8.20E-06 | 1.80E-06 | 8.00E-06 | 6 | 0.8 | 0 | 6.4 | 0.7 |
| ENSG00000249427 | RP11-432M8.4 | 5.8 | 5.8 | 6.8 | 1.10E-05 | 1.10E-05 | 7.30E-08 | 5.8 | 0 | 0 | 6.8 | 0 |
| ENSG00000219492 | RP11-1396013.13 | 5.8 | 5.8 | 5.8 | 1.30E-05 | 1.30E-05 | 0.00015 | 5.8 | 0 | 0 | 5.8 | 0 |
| ENSG00000249339 | RP11-321E2.7 | 5.7 | 5.7 | 6.7 | 1.50E-05 | 1.50E-05 | 1.00E-07 | 5.7 | 0 | 0 | 6.7 | 0 |
| ENSG00000253134 | CTC-436K13.2 | 5.7 | 5.7 | 6.1 | 1.50E-05 | 1.50E-05 | 7.30E-06 | 5.7 | 0 | 0 | 6.1 | 0 |
| ENSG00000237417 | RP11-280G19.2 | 5.7 | 5.7 | 6.1 | 1.70E-05 | 1.60E-05 | 8.00E-06 | 5.7 | 0 | 0 | 6.1 | 0 |
| ENSG00000165929 | TC2N | 5.7 | 5.7 | 7.6 | 1.80E-05 | 1.70E-05 | 1.70E-12 | 5.7 | 0 | 0 | 9.2 | 1.6 |
| ENSG00000234749 | FAM90A21P | 5.1 | 5.9 | 5.7 | 1.80E-05 | 4.00E-06 | 7.20E-06 | 5.9 | 0.8 | 0 | 5.7 | 0 |
| ENSG00000241048 | RP11-167H9.5 | 5.7 | 5.7 | 5.8 | 1.90E-05 | 1.80E-05 | 4.70E-05 | 5.7 | 0 | 0 | 5.8 | 0 |
| ENSG00000253619 | RP11-369K17.1 | 5.7 | 5.7 | 5.4 | 2.20E-05 | 2.20E-05 | 4.10E-05 | 5.7 | 0 | 0 | 6.2 | 0.7 |
| ENSG00000236179 | RP13-221M14.4 | 5.6 | 5.6 | 6.3 | 3.00E-05 | 2.90E-05 | 6.90E-07 | 5.6 | 0 | 0 | 6.3 | 0 |
| ENSG00000224516 | AC068134.8 | 5.6 | 5.6 | 6 | 3.60E-05 | 3.50E-05 | 8.70E-05 | 5.6 | 0 | 0 | 6 | 0 |
| ENSG00000250296 | RP11-321E2.9 | 5.6 | 5.6 | 6.6 | 4.30E-05 | 4.20E-05 | 2.40E-07 | 5.6 | 0 | 0 | 6.6 | 0 |
| ENSG00000223912 | EEF1A1P36 | 5.5 | 5.5 | 5.5 | 5.80E-05 | 5.70E-05 | 3.90E-05 | 5.5 | 0 | 0 | 5.5 | 0 |
| ENSG00000248542 | RP11-432M8.16 | 5.5 | 5.5 | 6.3 | 6.20E-05 | 6.10E-05 | 4.50E-07 | 5.5 | 0 | 0 | 6.3 | 0 |
| ENSG00000204479 | PRAMEF17 | 5.5 | 5.5 | 5.8 | 7.50E-05 | 7.40E-05 | 1.30E-05 | 5.5 | 0 | 0 | 5.8 | 0 |
| ENSG00000235665 | AC007464.1 | 5.5 | 5.5 | 5.1 | 7.50E-05 | 7.40E-05 | 4.40E-06 | 5.5 | 0 | 0 | 6.4 | 1.3 |
| ENSG00000104413 | ESRP1 | 5.4 | 5.4 | 6.8 | 0.00011 | 0.00011 | 1.50E-07 | 5.4 | 0 | 0 | 6.8 | 0 |
| ENSG00000258220 | RP11-38F22.1 | 5.4 | 5.4 | 6 | 0.00015 | 0.00014 | 8.40E-05 | 5.4 | 0 | 0 | 6 | 0 |
| ENSG00000204503 | PRAMEF3 | 5.4 | 5.4 | 6 | 0.00016 | 0.00015 | 1.50E-05 | 5.4 | 0 | 0 | 6 | 0 |

In contrast to DUX4 up-regulated genes, there were very few genes down-regulated by DUX4. Compared to the lenti-GFP control, 28 down-regulated genes were common to both cell types. However, only one gene (CSF3) was also down-regulated relative to a no-virus control. Most of remaining 27 genes were involved in the innate immune response, consistent with the prior demonstration that DUX4 represses the innate immune response induced by lenti-viral transduction (Geng, et al., 2012; Semple, et al., 2010), a process partly mediated by up-regulation of DEFB103, a defensin peptide previously shown to block the innate immune response (Semple, et al., 2010).

**DUX4 regulated genes are mis-expressed in FSHD muscle cells.** To determine whether DUX4 targets, or other genes, are mis-expressed in FSHD muscle cells, gene expression in primary muscle cultures from five FSHD individuals (three FSHD2 and two FSHD1) and three unaffected control individuals were compared. In undifferentiated myoblasts, 90 genes showed increased expression in FSHD cells compared to controls and over one-half of these (51 genes) were among the subset of 228 robust DUX4 targets identified in the transduction experiments. In differentiated myotube cultures, 348 genes were differentially up-regulated in FSHD cells compared to controls, 158 of which were among the 228 most robust DUX4 targets (**FIG. 1D**, purple dots) and an additional 118 were induced 2.71- fold or more by DUX4 transduction in myoblasts but did not meet the more rigorous criteria for a DUX4 target (**FIG. 1D**, blue dots); whereas only 72 (∼20%) differentially expressed genes were not identified as regulated by DUX4 (**FIG. 1D**, olive dots and Table 4), and only 18 of these met the statistical threshold in both FSHD myoblasts and myotubes. Therefore, the vast majority of gene expression changes in cultured FSHD muscle cells can be attributed to the expression of DUX4 and genes regulated by DUX4. The increase in the number of DUX4 target genes detected in differentiated muscle compared to myoblasts likely represents the increase in DUX4 mRNA and protein that occurs during FSHD muscle differentiation (Krom, et al., 2012; Block, et al., 2013).

**Table 4**

| **Genes up regulated in both FSHD cultured myotubes and myoblasts relative to controls, but not up-regulated by Dux4** | | | | | | | | | | | | |
|---|---|---|---|---|---|---|---|---|---|---|---|---|
| gene. name | FSHDtube. logFC | FSHDbla st.logFC | X541Dux4_g fp.logFC | FSHDtube. pval | FSHDblast. pval | X541 Dux4_gfp. pval | control blasts | control tubes | FSHD blasts | FSHD tubes | 54-1Dux4 | 54-1gfp |
| APBB1IP | 3.3 | 3.2 | 0 | 1.60E-09 | 8.00E-07 | 1 | 0 | 0 | 3.16 | 3.34 | 0 | 0 |
| TMEM176B | 1.9 | 3.8 | 0 | 2.30E-06 | 1.80E-07 | 1 | 1.43 | 1.63 | 5.26 | 3.51 | 0 | 0 |
| NRK | 3 | 3.3 | -0.8 | 3.90E-08 | 1.60E-06 | 0.88 | 1.41 | 1.18 | 4.7 | 4.22 | 1.62 | 2.42 |
| MMP16 | 2.5 | 2 | 0.7 | 5.70E-07 | 2.50E-05 | 0.32 | 3.81 | 2.92 | 5.84 | 5.42 | 5.93 | 5.24 |
| PRRX2 | 2.4 | 3.3 | 0.1 | 3.00E-06 | 2.00E-06 | 1 | 2.65 | 1.47 | 5.92 | 3.87 | 3.45 | 3.4 |
| CTRB2 | 2.1 | 2.3 | -0.6 | 1.50E-05 | 1.50E-05 | 0.83 | 2.54 | 3.48 | 4.81 | 5.59 | 2.28 | 2.93 |
| SYCE1 | 1.9 | 2.6 | -1.2 | 4.90E-06 | 7.70E-07 | 0.84 | 2.38 | 2.74 | 5 | 4.6 | 1.02 | 2.24 |
| NKX2-6 | 1.7 | 1.6 | -0.4 | 1.50E-06 | 1.30E-05 | 0.56 | 1.25 | 0.31 | 2.85 | 2.06 | 5.47 | 5.88 |
| POU3F3 | 3.7 | 3.8 | 0 | 2.20E-08 | 1.20E-07 | 1 | 0.71 | 0.57 | 4.54 | 4.29 | 0 | 0 |
| ENSG00000206197 | 2.4 | 3.1 | -1 | 3.90E-06 | 2.00E-07 | 0.1 | 4.03 | 4.58 | 7.09 | 7.01 | 6.77 | 7.8 |
| AP000525.1 | 2.5 | 3.1 | -1 | 8.30E-05 | 6.90E-06 | 0.19 | 1.86 | 2.32 | 4.92 | 4.86 | 4.62 | 5.66 |
| LINC00516 | 2.5 | 3.5 | -0.9 | 6.00E-05 | 2.70E-06 | 0.29 | 1.23 | 2.19 | 4.77 | 4.64 | 4.29 | 5.21 |
| AL589743.1 | 2.4 | 3 | -1 | 3.70E-06 | 2.70E-07 | 0.11 | 5.01 | 5.51 | 8.02 | 7.93 | 7.77 | 8.76 |
| AL589743.2 | 2.4 | 3 | -0.9 | 2.20E-05 | 2.60E-06 | 0.19 | 2.46 | 3.04 | 5.47 | 5.41 | 5.2 | 6.14 |
| RP11-146E13.1 | 2.8 | 3.2 | -1 | 5.00E-05 | 7.50E-06 | 0.2 | 1.53 | 1.77 | 4.75 | 4.53 | 4.55 | 5.56 |
| RP11-49612.3 | 2.5 | 3 | -1.1 | 5.90E-06 | 4.70E-06 | 0.13 | 2.32 | 2.61 | 5.35 | 5.15 | 4.82 | 5.97 |
| RP11-146E13.2 | 3 | 3.7 | -0.9 | 7.30E-05 | 1.70E-05 | 0.57 | 0.43 | 0.72 | 4.13 | 3.71 | 2.97 | 3.84 |
| RP11-49612.6 | 2.7 | 3.2 | -1 | 5.40E-05 | 8.20E-06 | 0.21 | 1.53 | 1.77 | 4.72 | 4.52 | 4.52 | 5.53 |
| | | | | | | | | | | | | |

| **Genes up regulated only in FSHD cultured myoblasts and not in myotubes, and not by Dux4** | | | | | | | | | | | | |
|---|---|---|---|---|---|---|---|---|---|---|---|---|
| gene.name | FSHDtube. lfc | FSHDbla st.lfc | X541Dux4_g fp.lfc | FSHDtube. pval | FSHDblast. pval | X541Dux4_gfp. pval | control blasts | control tubes | FSHD blasts | FSHD tubes | 54-1Dux4 | 54-1gfp |
| TMEM176A | 0.60 | 4.10 | 0.00 | 5.10E-04 | 4.20E-08 | 1.00E+00 | 1.01 | 2.26 | 5.10 | 2.88 | 0.00 | 0.00 |
| SYNDIG1 | 1.90 | 2.30 | 0.20 | 1.80E-04 | 2.10E-05 | 1.00E+00 | 2.68 | 1.09 | 4.95 | 2.94 | 1.02 | 0.83 |
| SFRP1 | -0.60 | 2.50 | -0.40 | 6.80E-01 | 1.60E-08 | 1.00E+00 | 4.40 | 5.92 | 6.91 | 5.33 | 1.02 | 1.38 |
| STMN2 | 0.90 | 1.30 | 0.00 | 7.20E-04 | 6.00E-07 | 1.00E+00 | 6.85 | 6.77 | 8.11 | 7.67 | 0.00 | 0.00 |
| PTN | 0.50 | 3.30 | -1.00 | 2.80E-01 | 4.10E-07 | 2.40E-01 | 2.44 | 2.56 | 5.75 | 3.04 | 4.67 | 5.62 |
| CA9 | 0.10 | 1.80 | -2.20 | 4.50E-01 | 1.40E-05 | 1.10E-03 | 5.17 | 2.82 | 6.95 | 2.94 | 6.50 | 8.65 |
| BCHE | 1.30 | 2.90 | 0.50 | 3.00E-03 | 5.50E-06 | 5.10E-01 | 1.75 | 2.62 | 4.61 | 3.95 | 5.21 | 4.67 |
| RBP4 | 0.40 | 1.90 | 0.20 | 3.70E-01 | 4.60E-05 | 1.00E+00 | 3.07 | 2.38 | 4.95 | 2.81 | 1.02 | 0.83 |
| GABRQ | 0.70 | 2.90 | 0.00 | 3.30E-01 | 1.20E-05 | 1.00E+00 | 1.25 | 0.99 | 4.19 | 1.72 | 0.00 | 0.00 |
| CDC42BPG | 1.40 | 2.10 | 0.30 | 8.20E-04 | 2.90E-05 | 1.00E+00 | 2.72 | 3.13 | 4.82 | 4.58 | 2.28 | 2.03 |
| ADH1B | -0.80 | 2.50 | 0.00 | 2.60E-07 | 8.70E-07 | 1.00E+00 | 2.08 | 3.80 | 4.56 | 3.03 | 0.00 | 0.00 |
| AP000525.8 | 1.80 | 2.30 | -1.00 | 3.90E-04 | 4.40E-05 | 1.30E-01 | 4.93 | 5.24 | 7.20 | 7.00 | 6.57 | 7.53 |
| RP11-343H5.4 | 1.60 | 3.40 | 0.70 | 1.80E-01 | 4.30E-08 | 4.20E-01 | 1.86 | 3.18 | 5.23 | 4.77 | 5.09 | 4.41 |
| RP11-114H7.3 | 0.90 | 1.30 | 0.00 | 2.40E-05 | 3.80E-05 | 1.00E+00 | 0.00 | 0.00 | 1.29 | 0.90 | 0.00 | 0.00 |
| | | | | | | | | | | | | |

| **Genes up regulated only in FSHD cultured myotubes and not in myoblasts, and not by Dux4** | | | | | | | | | | | | |
|---|---|---|---|---|---|---|---|---|---|---|---|---|
| gene. name | FSHDtube. lfc | FSHDbla st.lfc | 541Dux4.lfc | FSHDtube. pval | FSHDblast. pval | 541Dux4.pval | control blasts | control tubes | FSHD blasts | FSHD tubes | 54-1Dux4 | 54-1gfp |
| KCNF1 | 3.30 | 0.90 | 0.90 | 1.40E-10 | 1.00E-01 | 7.90E-01 | 3.12 | 4.22 | 3.97 | 7.55 | 2.68 | 1.75 |
| HDC | 3.40 | 1.20 | -0.40 | 2.10E-09 | 6.80E-02 | 1.00E+00 | 0.43 | 1.71 | 1.62 | 5.14 | 1.02 | 1.38 |
| SLC16A6 | 2.90 | -0.60 | 0.40 | 1.30E-08 | 3.00E-01 | 8.10E-01 | 3.26 | 2.31 | 2.69 | 5.24 | 3.72 | 3.33 |
| ARGFX | 3.80 | 0.00 | 0.00 | 1.50E-08 | 1.00E+00 | 1.00E+00 | 0.00 | 0.00 | 0.00 | 3.80 | 0.00 | 0.00 |
| KISS1 | 1.90 | 1.30 | -1.20 | 3.80E-08 | 2.50E-02 | 7.90E-01 | 5.86 | 5.10 | 7.13 | 7.02 | 1.02 | 2.24 |
| SALL4 | 3.50 | 0.30 | 0.60 | 5.00E-08 | 7.10E-01 | 8.50E-01 | 0.95 | 0.31 | 1.28 | 3.78 | 2.83 | 2.24 |
| RP11-385N17.1 | 3.20 | 3.20 | 0.00 | 2.00E-07 | 9.90E-05 | 1.00E+00 | 0.43 | 0.00 | 3.60 | 3.24 | 0.00 | 0.00 |
| RP11-533F5.2 | 3.70 | 0.00 | 0.00 | 3.80E-07 | 1.00E+00 | 1.00E+00 | 0.00 | 0.00 | 0.00 | 3.71 | 0.00 | 0.00 |
| KIF5A | 2.90 | 1.00 | 0.60 | 4.50E-07 | 1.50E-01 | 5.60E-01 | 1.23 | 1.91 | 2.23 | 4.84 | 4.32 | 3.67 |
| FA2H | 2.00 | 0.40 | 0.20 | 5.70E-07 | 7.70E-01 | 1.00E+00 | 0.43 | 1.78 | 0.81 | 3.78 | 1.02 | 0.83 |
| KRT7 | 1.90 | 1.80 | -1.70 | 1.30E-06 | 3.00E-03 | 1.60E-02 | 6.56 | 5.01 | 8.37 | 6.90 | 5.29 | 6.96 |
| CKMT1 B | 3.60 | -0.50 | 0.00 | 1.30E-06 | 6.60E-01 | 1.00E+00 | 0.52 | 0.00 | 0.00 | 3.57 | 0.00 | 0.00 |
| AC093850.2 | 2.40 | 1.20 | -1.00 | 2.40E-06 | 6.80E-02 | 9.00E-01 | 2.49 | 3.31 | 3.66 | 5.70 | 1.02 | 2.03 |
| POSTN | 2.10 | 0.20 | 0.60 | 3.10E-06 | 5.40E-01 | 3.10E-01 | 8.14 | 8.14 | 8.38 | 10.20 | 10.75 | 10.14 |
| KIF14 | 1.70 | 1.20 | 0.90 | 3.40E-06 | 1.30E-02 | 1.60E-01 | 6.18 | 4.47 | 7.41 | 6.20 | 7.80 | 6.93 |
| ARSI | 2.00 | 0.60 | 0.90 | 3.50E-06 | 2.50E-01 | 2.40E-01 | 6.34 | 5.19 | 6.91 | 7.17 | 5.95 | 5.10 |
| RP11-807H22.5 | 3.10 | -0.40 | 0.00 | 4.00E-06 | 6.60E-01 | 1.00E+00 | 0.43 | 0.57 | 0.00 | 3.64 | 0.00 | 0.00 |
| RP11-73B2.6 | 3.00 | 1.60 | 0.00 | 4.70E-06 | 4.00E-03 | 1.00E+00 | 0.00 | 0.72 | 1.64 | 3.76 | 0.00 | 0.00 |
| AQP5 | 1.70 | 1.70 | -0.80 | 4.90E-06 | 9.20E-04 | 1.00E+00 | 1.41 | 3.67 | 3.14 | 5.36 | 0.00 | 0.83 |
| LDB2 | 2.90 | 2.50 | 0.00 | 8.70E-06 | 5.70E-03 | 1.00E+00 | 0.90 | 0.37 | 3.35 | 3.24 | 0.00 | 0.00 |
| C1QL4 | 1.60 | 0.40 | 0.60 | 1.00E-05 | 5.00E-01 | 7.20E-01 | 4.90 | 4.64 | 5.27 | 6.25 | 3.19 | 2.57 |
| SPP1 | 2.70 | 0.70 | 0.70 | 1.10E-05 | 9.20E-01 | 4.60E-01 | 1.62 | 2.31 | 2.33 | 5.04 | 4.98 | 4.29 |
| GUCA1A | 3.10 | -0.10 | 0.00 | 1.20E-05 | 1.00E+00 | 1.00E+00 | 0.43 | 0.68 | 0.38 | 3.80 | 0.00 | 0.00 |
| CTRB1 | 1.70 | 1.80 | -0.40 | 1.30E-05 | 5.30E-04 | 9.90E-01 | 2.49 | 2.79 | 4.28 | 4.49 | 1.62 | 2.03 |
| GPR126 | 1.60 | 1.40 | 0.80 | 1.40E-05 | 9.30E-01 | 2.00E-01 | 3.81 | 3.29 | 5.19 | 4.87 | 6.94 | 6.14 |
| CKMT1A | 3.30 | 0.00 | 0.00 | 1.40E-05 | 1.00E+00 | 1.00E+00 | 0.00 | 0.00 | 0.00 | 3.28 | 0.00 | 0.00 |
| GRIA3 | 1.90 | 1.60 | -0.60 | 2.00E-05 | 1.50E-03 | 3.70E-01 | 4.46 | 4.17 | 6.03 | 6.05 | 6.03 | 6.62 |
| PGA4 | 1.50 | 0.20 | -3.00 | 2.20E-05 | 8.70E-01 | 5.00E-02 | 3.09 | 3.90 | 3.25 | 5.38 | 1.62 | 4.57 |
| SERPINA9 | 1.40 | 0.30 | 0.40 | 2.90E-05 | 1.90E-01 | 6.70E-01 | 2.64 | 1.06 | 2.93 | 2.48 | 4.44 | 3.99 |
| RP11-472G21.2 | 2.80 | -0.60 | 0.00 | 2.90E-05 | 4.60E-01 | 1.00E+00 | 1.01 | 0.92 | 0.40 | 3.73 | 0.00 | 0.00 |
| PGA3 | 1.60 | 0.40 | -2.30 | 3.50E-05 | 6.90E-01 | 2.20E-01 | 2.57 | 3.44 | 2.97 | 5.02 | 1.62 | 3.92 |
| PI16 | 1.10 | -0.10 | 0.60 | 3.90E-05 | 1.00E+00 | 9.50E-01 | 0.71 | 1.17 | 0.64 | 2.30 | 2.00 | 1.38 |
| RP11-221J22.1 | 1.60 | 0.50 | 0.20 | 4.20E-05 | 5.20E-01 | 8.30E-01 | 5.63 | 4.10 | 6.09 | 5.66 | 5.84 | 5.69 |
| PGA5 | 1.50 | -0.40 | -3.20 | 4.20E-05 | 5.00E-01 | 8.70E-02 | 3.00 | 3.44 | 2.56 | 4.97 | 1.02 | 4.21 |
| RP11-371I1.2 | 2.80 | 3.50 | -0.60 | 4.40E-05 | 4.90E-05 | 9.10E-01 | 0.00 | 0.37 | 3.53 | 3.19 | 1.62 | 2.24 |
| AP000459.7 | 2.30 | 0.00 | 0.00 | 4.80E-05 | 1.00E+00 | 1.00E+00 | 0.00 | 0.00 | 0.00 | 2.31 | 0.00 | 0.00 |
| TBX5 | 2.80 | 1.70 | 0.00 | 5.40E-05 | 2.00E-02 | 1.00E+00 | 1.14 | 0.00 | 2.80 | 2.81 | 0.00 | 0.00 |
| ACE | 2.00 | 0.70 | -0.10 | 5.50E-05 | 2.00E-02 | 9.80E-01 | 3.58 | 2.82 | 4.31 | 4.83 | 2.97 | 3.11 |
| RP3-416H24.1 | 1.70 | 1.50 | -1.50 | 5.50E-05 | 3.00E-02 | 4.00E-02 | 5.83 | 4.11 | 7.31 | 5.77 | 4.65 | 6.18 |
| KCNN4 | 1.90 | 0.60 | 0.00 | 6.10E-05 | 8.10E-01 | 1.00E+00 | 4.94 | 2.11 | 5.56 | 4.03 | 3.93 | 3.92 |
| BFSP2 | 2.50 | 0.80 | 0.00 | 6.40E-05 | 6.40E-01 | 1.00E+00 | 0.43 | 0.31 | 1.18 | 2.77 | 0.00 | 0.00 |
| FAM43B | 2.00 | 0.90 | 0.70 | 7.00E-05 | 9.30E-02 | 6.40E-01 | 3.71 | 3.76 | 4.59 | 5.73 | 3.77 | 3.11 |
| POU3F2 | 2.30 | -0.50 | 0.60 | 7.70E-05 | 8.10E-03 | 3.50E-01 | 2.00 | 0.31 | 1.49 | 2.63 | 6.27 | 5.62 |
| AC138517.5 | 3.00 | 0.00 | 0.90 | 7.70E-05 | 1.00E+00 | 8.80E-01 | 0.00 | 0.00 | 0.00 | 3.01 | 2.28 | 1.38 |
| SOAT2 | 1.90 | 0.20 | -0.40 | 7.80E-05 | 9.30E-01 | 9.80E-01 | 1.66 | 3.38 | 1.90 | 5.26 | 1.62 | 2.03 |
| CTD-2314B22.3 | 1.80 | 2.20 | -0.90 | 7.80E-05 | 8.80E-05 | 1.50E-01 | 5.14 | 5.44 | 7.36 | 7.24 | 7.19 | 8.08 |
| RP11-312J18.6 | 2.70 | 0.00 | 0.00 | 8.00E-05 | 1.00E+00 | 1.00E+00 | 0.00 | 0.31 | 0.00 | 3.06 | 0.00 | 0.00 |
| LDHC | 1.40 | 1.10 | -1.70 | 8.20E-05 | 3.50E-03 | 3.90E-02 | 1.75 | 1.37 | 2.84 | 2.72 | 2.68 | 4.41 |
| AC018804.7 | 2.40 | 1.50 | -0.80 | 0.00011 | 1.00E-02 | 1.00E+00 | 2.30 | 2.53 | 3.81 | 4.91 | 0.00 | 0.83 |
| CTD-2331C18.5 | 2.00 | 0.60 | -3.30 | 0.00011 | 1.00E+00 | 2.80E-01 | 1.31 | 1.97 | 1.93 | 3.98 | 0.00 | 3.33 |
| LRRC15 | 2.70 | 0.80 | -0.70 | 0.00012 | 1.70E-01 | 5.70E-01 | 1.37 | 0.57 | 2.17 | 3.29 | 3.45 | 4.12 |
| RP5-1172A22.1 | 2.60 | -0.20 | 0.90 | 0.00012 | 6.40E-01 | 4.10E-01 | 2.35 | 0.87 | 2.19 | 3.50 | 4.57 | 3.67 |
| TNC | 1.30 | 0.30 | 0.10 | 0.00013 | 8.00E-01 | 8.40E-01 | 8.73 | 6.94 | 9.03 | 8.23 | 9.06 | 8.94 |
| ANO2 | 1.20 | 0.70 | -2.80 | 0.00013 | 6.90E-02 | 5.10E-01 | 4.28 | 4.36 | 4.99 | 5.60 | 0.00 | 2.82 |

**DUX4 target genes are expressed in FSHD biopsy samples.** To determine whether DUX4 target genes distinguish control from FSHD muscle biopsies, RNA-Seq was performed on 24 quadriceps needle biopsy samples (nine control, nine FSHD1 and six FSHD2) (see Methods and Table 5 for the complete sample list and associated clinical data). Only 38 genes were consistently up-regulated across all FSHD samples compared to all of the control samples (p-value < 0.05, moderated log fold-change > 1 and Wilcoxon rank sum p-value < 0.05 (see Methods)), and 28 of these (74%) were among the robust 228 DUX4 targets, indicating that most of the FSHD-specific gene expression in muscle biopsies was determined by DUX4.

**Table 5**

| FSHD1 | | | | | | | | | |
|---|---|---|---|---|---|---|---|---|---|
| NMD # | Gender | Age | D4Z4:Kb¹ | CSS² | Corr. CSS⁵ | Path. Score³ | Mucle Bx'd | | |
| 509 | M | 47 | 26 | 0/10 | 0 | 2 | L Quad | | |
| 2306 | F | 45 | 18 | 5/10 | 222 | NHS⁴ | L Quad | | |
| 2331 | F | 55 | 15 | 6/10 | 218 | 4 | R Quad | | |
| 2316 | M | 33 | 24 | 6/10 | 363 | 5 | R Quad | | |
| 2319 | M | 52 | 22 | 6/10 | 231 | NHS | R Quad | | |
| 2315 | F | 30 | 23 | 3/10 | 200 | 3 | L Quad | | |
| 2377 | F | 61 | 16 | 6/10 | 197 | 6 | R Quad | | |
| 2326 | F | 26 | 15 | 7/10 | 538 | 5 | R Quad | | |
| 2367 | M | 48 | 17 | 3/10 | 125 | 4 | L Quad | | |

| Control | | | | | | | | | |
|---|---|---|---|---|---|---|---|---|---|
| NMD # | | Gender | | | Age | | Muscle Bx'd | | |
| 2318 | | M | | | 37 | | L Quad | | |
| 2333 | | M | | | 44 | | L Quad | | |
| 2397* | | M | | | 62 | | L Quad | | |
| 2398 | | M | | | 48 | | L Quad | | |
| 2401 | | M | | | 58 | | L Quad | | |
| 2489 | | F | | | 69 | | R ANT TIB | | |
| 2429 | | F | | | 41 | | L Quad | | |
| 2409 | | F | | | 53 | | L Quad | | |
| 2374 | | F | | | 32 | | R Quad | | |

| FSHD2 | | | Fsel % | | | | | | |
|---|---|---|---|---|---|---|---|---|---|
| NMD # | Gender | Age | Blood | Blasts | SMCHD1 mutation | CSS | Corr.CSS⁵ | Path. Score | Muscle Bx'd |
| 1614 | M | 42 | 12 | ** | Yes | 5/10 | 238 | 2 | R Quad |
| 1881 | M | 34 | 16 | 2 | Yes | 6/10 | 352 | 3 | L Quad |
| 2332 | M | 26 | 7 | 3 | Yes | 6/10 | 461 | 4 | R Quad |
| 2413 | M | 50 | 13 | ** | No | 5/10 | 200 | 5 | L Quad |
| 2440 | F | 56 | 25 | 7 | Yes | 7/10 | 250 | 3 | R Quad |
| 2334 | M | 59 | 15 | -3 | No | 2/10 | 68 | 2 | L Quad |
| | | | | | | | | | |
| | | | | | | | | | |
| | | | | | | | | | |
| | | | | | | | | | |
| ** Blast Fsel same as blood | | | | | | | | | |

| | | | | | | | | | |
|---|---|---|---|---|---|---|---|---|---|
| 1. All refer to the size of the FSHD-permissive 4qA161 allele 2. CSS: clinical severity score: 0 asymptomatic-10 wheelchairbound 3. Path score: 0 = no pathology - 12 = severe pathology (none had inflammation) 4. NHS: No histological sample collected. 5. Age corrected CSS = ((CSSx2)/age at examination) x 1000 6. Fsel %: percentage CpG methylation of D4Z4 as measured by the Fsel restriction endonuclease | | | | | | | | | |

The expression of the robust DUX4 targets was examined across all biopsy samples (**FIG. 2A**), and noticed that nine of the fifteen FSHD samples had elevated expression levels of DUX4 targets compared to controls, whereas the other six FSHD biopsy samples had comparable expression of DUX4 targets as the controls. It was also noted that one control sample, 2401, had lower but detectable expression of DUX4 targets and this control clustered with the FSHD biopsy samples that also expressed DUX4 targets.

The segregation of FSHD samples into DUX4-target-positive and DUX4-target-negative samples likely reflects the biopsy of a muscle that is affected late in FSHD (the quadriceps) and a limited amount of sampled tissue with a small needle biopsy (see Discussion). The control sample 2401 is a member of a complex FSHD family (**FIG. 5**). He is a clinically unaffected individual with thirteen D4Z4 repeats on an FSHD-permissive haplotype, which is more than the standard threshold of ten repeats for FSHD and would not be expected to express DUX4. His FSHD2-affected sibling (2334, also included in this study) has the same thirteen repeat FSHD-permissive allele that is hypomethylated but without an identified pathological mutation in *SMCHD1*, suggesting that an as yet unknown modifier locus is segregating in this family and might be present in 2401 (see Discussion).

To determine the most robust FSHD-DUX4 biomarker genes, 2401 was removed from subsequent analyses based on the assumption that 2401 might represent a DUX4-expressing unaffected individual in a family with an unknown FSHD causing mutation, and restricted the comparison to the remaining control samples. Of the 118 genes expressed significantly higher in DUX4-target-positive FSHD-vs-control samples, 67 were among the tissue-culture identified 228 robust DUX4 targets (Table 6) and an additional thirteen were boundary cases of DUX4 regulated genes that did not make the strict cut-off for the robust DUX4 targets. Therefore approximately 69% of the genes associated with FSHD are regulated by DUX4. The 67 robust DUX4 target genes were among the most up-regulated genes in DUX4-target-positive FSHD biopsy samples, FSHD myotubes, and DUX4 transduced cells (FIG.s 2B-D, green dots) relative to their respective controls, and expression of all these genes increased during myogenesis in cultured FSHD muscle cells but not in control cells (**FIG. 6A**). Most of these genes were not expressed in the corresponding control samples, making them good candidates for FSHD biomarkers.

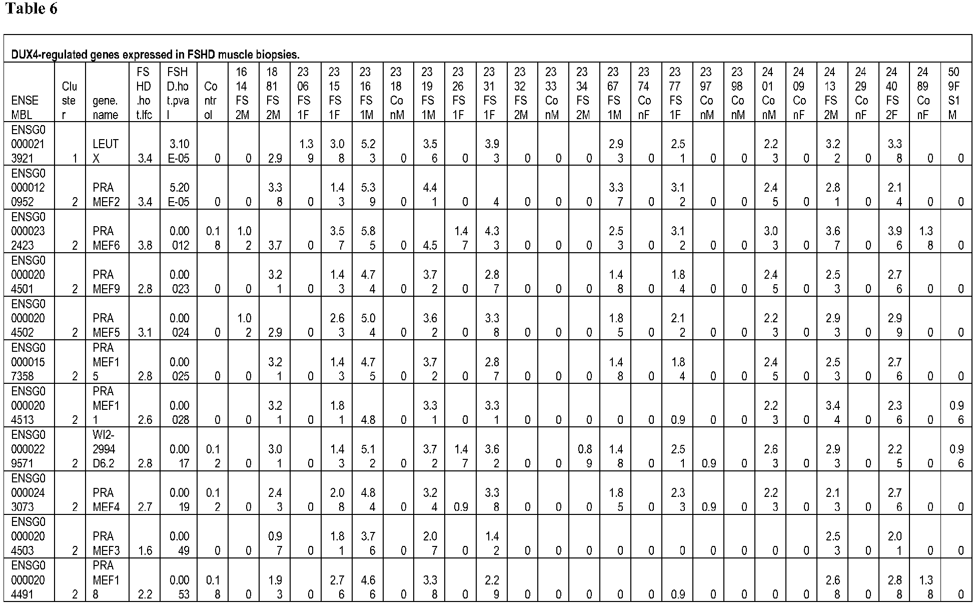

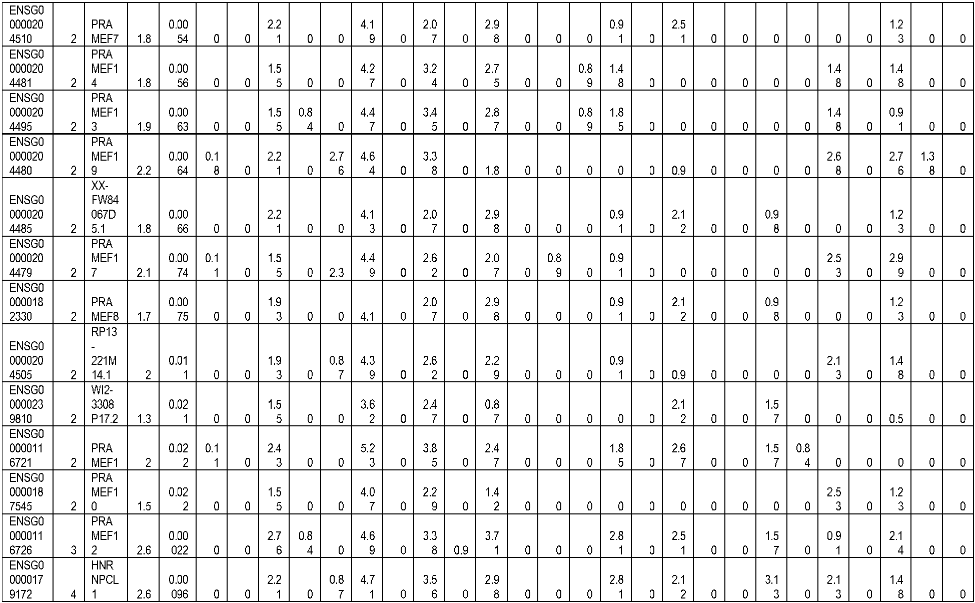

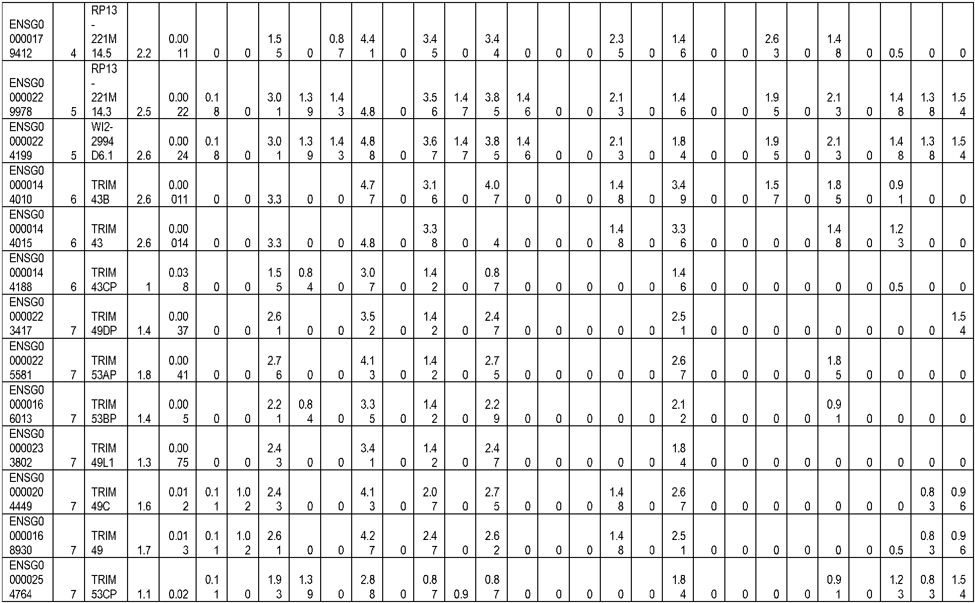

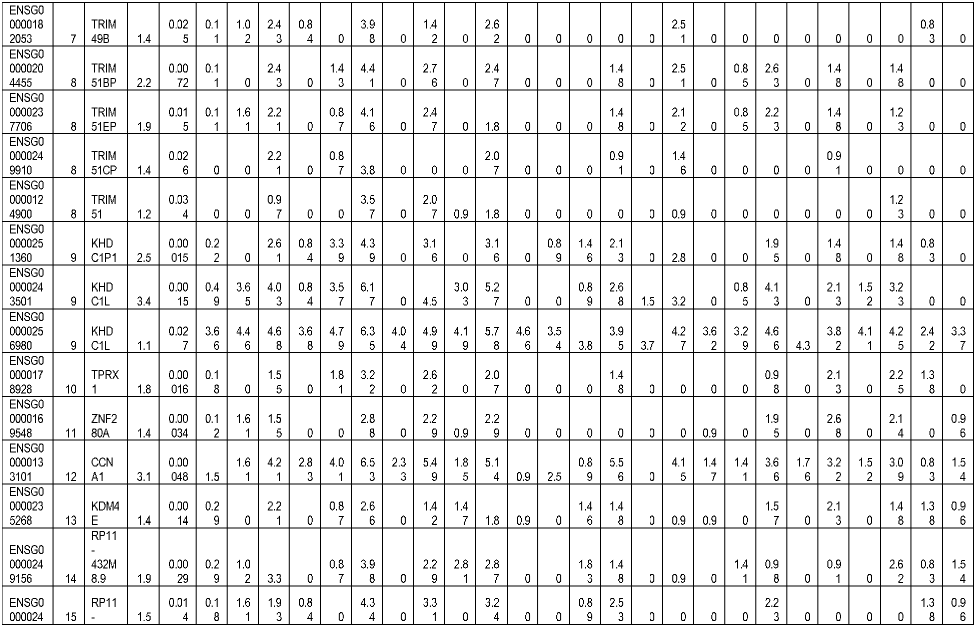

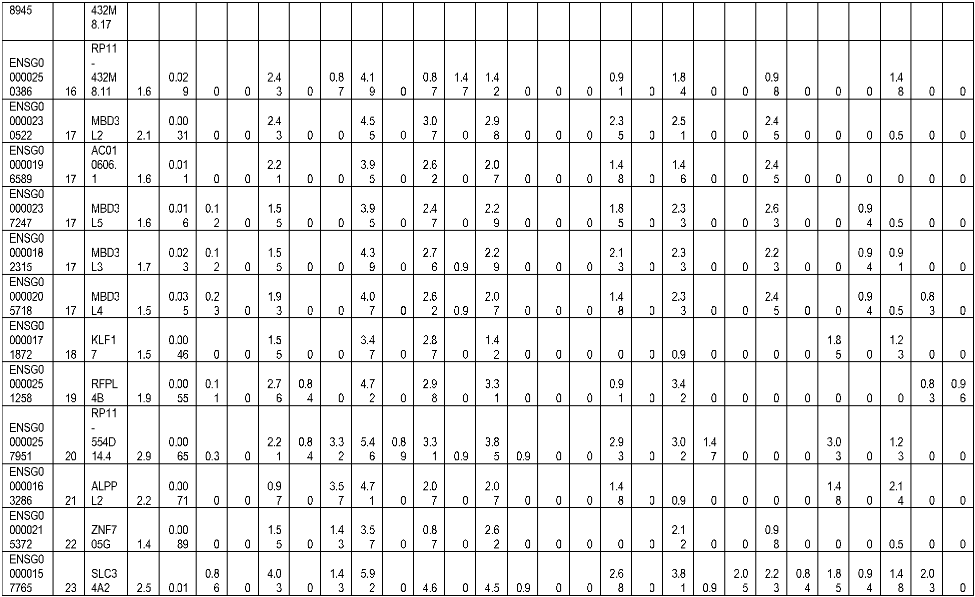

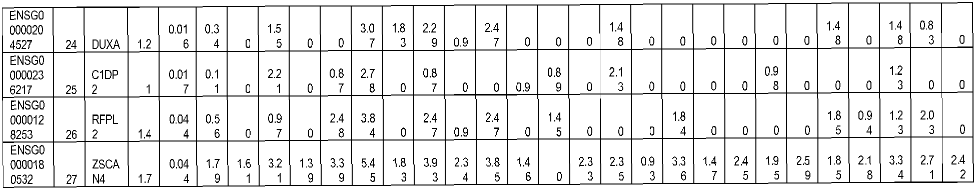

Gene expression between the DUX4-target-negative FSHD samples and the controls (excluding sample 2401) and did not find any differentially expressed genes using similar statistical thresholds. Therefore, the DUX4-target-positive FSHD samples show gene expression differences compared to the controls, whereas the DUX4-target-negative FSHD samples have similar gene expression to controls, again indicating that the DUX4 target genes are the main discriminator between FSHD and control samples.

**DUX4 target genes and candidate biomarkers.** To determine whether these candidate FSHD biomarkers might be direct targets of DUX4, the presence of DUX4 binding sites was examined based on motif analysis and DUX4 binding was examined from the prior ChIP-seq study of primary myoblasts transduced with DUX4 (**FIG. 2E**). This analysis was complicated by the fact that many of these DUX4 target genes are members of large gene families that share significant sequence homology. Therefore, these genes were clustered based on the number of shared RNA-seq reads (**FIG. 6B** and Table 6), and one member of each cluster was selected to examine the DUX4 binding in shown in **FIG. 3E**, which also includes the UCSC mapability tracks (CRG Align 40) to indicate potential loss of DUX4 peaks due to mapping ambiguity. Most of these candidate biomarker genes had very strong DUX4 ChIP-seq peaks and multiple DUX4 motifs within the five kilobases of the transcription start site (TSS). In several remaining cases, e.g., *PRAMEF11*, weak DUX4 peaks were centered over DUX4 motifs in repetitive regions that would diminish accurate mapping of reads. In a few cases (*C1DP2*, *KLF17*, *SLC34A2* and *TPRX1*), DUX4 binding was not observed in the promoter regions and it is possible that these genes were regulated by DUX4 indirectly, or by enhancers distant from the promoter regions.

It was striking that most biomarker candidates are members of highly homologous gene families that are clustered spatially on the chromosomes, including *PRAMEF* (preferentially expressed in melanoma), *TRIM* (tripartite motif-containing), *MBDL* (Methyl-CpG binding protein-like), *ZSCAN* (Zinc finger and SCAN domain containing) and *RFPL* (Ret-finger protein-like) families. The most dramatic example is the *PRAMEF* locus (**FIG. 2F**), which spans an approximately one megabase region and includes 28 biomarker candidates grouped into several different RNA-Seq clusters (see **FIG. 6B**).

From this pool of candidate biomarkers, four genes were identified as a core set of candidate biomarkers (the genome structure and RNA-seq reads for these genes are shown in **FIG. 6C-6D**). The candidate with the strongest statistical support is *LEUTX*, (leucine twenty homeobox). DUX4 activates a novel 5' UTR of *LEUTX* that is spliced into the second annotated exon that contains the beginning of the open reading frame. In the *PRAMEF* locus, *PRAMEF2* has the strongest statistical support with almost no expression in the control samples (except 2401). *TRIM43* is the strongest candidate among *TRIM* family members. *KHDC1L*, an isoform of *KHDC1*, and a nearby but non-overlapping pseudo gene *KHDC1P1*, are also good biomarkers with DUX4 binding sites within their promoters, whereas the overlapping *KHDC1* is not discriminative.

**Association of DUX4-target gene expression with clinical severity.** To associate expression of the candidate biomarkers with clinical data, the total number of reads that mapped to any of the 67 candidate biomarker genes in each sample (scaled and transformed by the square-root) was taken. This measure of biomarker gene expression with representation of the pathology score and the clinical severity score (CSS) (Balog, et al., 2012; Ricci, et al., 1999; van Overveld, et al., 2005) were plotted. Individuals with high pathology and CSS scores generally had higher biomarker expression (**FIG. 3A**). The discrimination between FSHD and control was slightly improved using only reads over the four selected biomarkers (*LEUTX*, *PRAMEF2*, *TRIM43*, and *KHDC1L*)(**FIG. 3B**).

It was assessed whether the discrimination of FSHD samples from controls might be improved by the addition of additional biomarker candidate genes to the four selected biomarkers (see **FIG. 7** for details). None of the other candidate biomarker genes improved the discrimination of FSHD and control samples and the four FSHD samples with very low expression of DUX4 targets (509, 2332, 2334, 2306) remained boundary cases that were not clearly separable from the control samples.

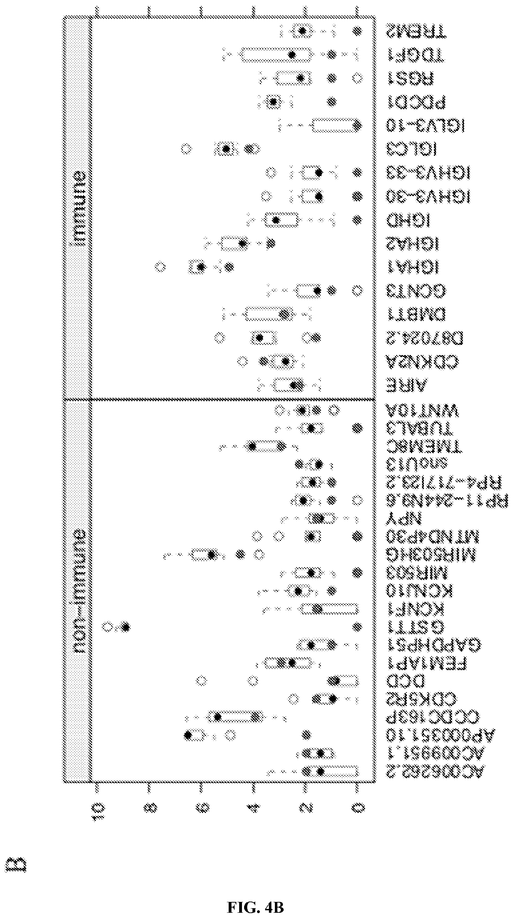

**Immune genes expressed in FSHD biopsies.** Although 80 of the 118 genes associated with the DUX4-target-positive FSHD samples were DUX4 targets (67 robust and 13 boundary cases), 38 genes were not identified as DUX4-regulated. The expression of these 38 genes across all samples was plotted in a clustered heat map (FIG. 4A). As anticipated, the DUX4-target positive FSHD samples cluster together, however, the control 2401 sample that had DUX4 target expression does not show a higher elevation of the 38 non-DUX4-target genes and no longer clusters with the FSHD samples. Sixteen of these FSHD-associated non-DUX4-target genes are expressed in cells of the immune system (Based on Gene Ontology annotation of "immune system process" and HUGO definitions of immunoglobulins gene family), including members of immunoglobulin clusters *IGHA*, *IGHD*, *IGLC* and *IGLV*, and plotting their distribution in DUX4-target-positive FSHD biopsies showed that the 2401 sample from the asymptomatic family member had lower expression of immune-associated genes compared to the FSHD-affected samples (FIG. 4B). Therefore, a major component of the non-DUX4-target gene changes likely represents the presence of immune cells in FSHD muscle biopsies, and the asymptomatic control individual 2401 that expresses some DUX4 target genes does not demonstrate expression changes indicating an immune infiltration. In addition, the FSHD biopsy samples with absent or low expression of DUX4 target genes also showed lower expression of the sixteen immune-associated genes (**FIG. 9**), indicating that the immune genes were mostly associated with muscle biopsies expressing DUX4 target genes.

**FSHD1 and FSHD2 have similar gene expression profiles.** Comparing gene expression between the FSHD1 and FSHD2 biopsy samples, only three genes expressed more highly in FSHD1 than FSHD2 were detected, and six genes expressed more highly in FSHD2 than FSHD1 were detected (Table 7). Interestingly, five of the six FSHD2-specific genes were members of homologous gene clusters in repeat arrays. *PCDHB2* is a member of the protocadherin cluster and several other members (*PCDHA2*, *PCDHA3*, and *PCDHA8*) were also preferentially expressed in FSHD2 patients, although they did not meet the statistical stringency. *TP53TG3C* is a member of *TP53TG3* gene family that have two adjacent copies and two more copies within 600 kb. Similarly, *RP11-3N2.13* and *RP11-3N2.1* are adjacent and homologous noncoding genes, while *RP11-760D2.5* is nearby.

**Table 7**

| ENSEMBL | gene.name | FSHD biopsy samples | | | | | | FSHD cultured myotubes | | | | | |
|---|---|---|---|---|---|---|---|---|---|---|---|---|---|
| #FSHD1 specfiic | | FSHD1 .FSHD 2.logFC | FSHD 1.logF C | FSHD 2.logF C | FSHD1.FS HD2.pval | FSHD1.p val | FSHD2.p val | FSHD1 2.logFC | FSHD1. logFC | FSHD2. logFC | FSHD12.p val | FSHD1.pv al | FSHD2.pv al |
| ENSG00000006071 | ABCC8 | 1.3 | 0.9 | -0.4 | 0.0063 | 1.70E-03 | 8.80E-01 | -0.1 | 0.1 | 0.2 | 9.90E-01 | 1.00E+00 | 9.40E-01 |
| ENSG00000172247 | C1QTNF4 | 1.2 | 0.9 | -0.3 | 0.0023 | 5.90E-03 | 4.40E-01 | 0.3 | 0.2 | -0.1 | 5.20E-01 | 7.50E-01 | 7.60E-01 |
| ENSG00000226005 | RP11-464C19.3 | 1.3 | 1.2 | -0.1 | 0.0087 | 2.70E-03 | 9.70E-01 | -0.2 | 0.5 | 0.6 | 8.30E-01 | 9.30E-01 | 4.50E-01 |
| #FSHD2 specific | | | | | | | | | | | | | |
| ENSG00000112852 | PCDHB2 | -0.7 | 0 | 0.7 | 3.70E-05 | 9.10E-01 | 6.20E-05 | -0.1 | 0.3 | 0.4 | 7.30E-01 | 4.90E-01 | 2.50E-01 |
| ENSG00000164778 | EN2 | -1 | 0.4 | 1.3 | 0.0083 | 2.10E-01 | 1.60E-04 | -0.7 | -0.3 | 0.5 | 1.50E-01 | 7.30E-01 | 1.90E-01 |
| ENSG00000205457 | TP53TG3C | -1.2 | -0.5 | 0.7 | 7.50E-05 | 1.70E-01 | 7.50E-03 | -1.8 | 0.8 | 2.6 | 4.20E-03 | 1.30E-01 | 3.50E-07 |
| ENSG00000214652 | RP11-3N2.13 | -0.9 | -0.3 | 0.6 | 1.80E-05 | 1.40E-01 | 3.00E-03 | -2.3 | 0.2 | 2.5 | 2.90E-07 | 6.30E-01 | 1.00E-08 |
| ENSG00000233288 | RP11-760D2.5 | -1.7 | -0.1 | 1.6 | 5.30E-05 | 9.90E-01 | 4.00E-04 | 0 | 0 | 0 | 1.00E+00 | 1.00E+00 | 1.00E+00 |
| ENSG00000244117 | RP11-3N2.1 | -0.8 | -0.3 | 0.5 | 5.30E-05 | 1.80E-01 | 4.50E-03 | -2.4 | 0.6 | 3 | 1.20E-06 | 9.80E-01 | 1.10E-08 |

FSHD1 and FSHD2 differentiated muscle cells in tissue culture were compared, although this analysis had even less power because of the sample size. The largest category of differentially expressed genes were genes that are increased during control muscle differentiation, suggesting that the FSHD2 cultures had better muscle differentiation compared to FSHD1 cultures (**FIG. 8**). It was then examined if the genes differentially expressed between FSHD1 and FSHD2 biopsy samples showed similar changes between cultured FSHD1 and FSHD2 myotubes. FSHD1 biopsy-specific genes did not show higher expression in FSHD1 cultured myotubes, while FSHD2 biopsy-specific genes also tended to be expressed at higher levels in cultured FSHD2 myotubes (Table 7), suggesting that a set of genes enriched in gene clusters or repetitive regions might be dysregulated in FSHD2 but not in FSHD1.

**Table 8**

| | **start** | **end** | **strand** | **gene.name** | **diff** |
|---|---|---|---|---|---|
| chr12 | 108274461 | 108274500 | + | NA | 15.76783055 |
| chr6 | 64260277 | 64260398 | + | PTP4A1 | 13.66001548 |
| chr19 | 40269508 | 40269567 | + | LEUTX | 13.219714 |
| chr4 | 25649655 | 25649978 | + | SLC34A2 | 12.99665174 |
| chr8 | 211074 | 211155 | + | NA | 12.97702544 |
| chr1 | 12833906 | 12833959 | + | PRAMEF1 | 11.48418972 |
| chr5 | 17626249 | 17626359 | - | NA | 10.82825385 |
| chr19 | 53809472 | 53809549 | - | FAM90A28P | 10.64027107 |
| chr4 | 165867089 | 165867298 | + | RP11-366M4.8 | 10.46307189 |
| chr11 | 94752052 | 94752121 | + | NA | 10.23871745 |
| chr11 | 49016024 | 49016159 | - | TRIM51GP | 9.974732112 |
| chr19 | 56284626 | 56284765 | - | NA | 9.837017677 |
| chr2 | 96178433 | 96178535 | + | TRIM64FP | 9.669275341 |
| chr5 | 78112492 | 78112713 | + | NA | 9.548754457 |
| chr5 | 178124587 | 178124747 | - | NA | 9.489541237 |
| chr12 | 7965382 | 7965403 | + | NA | 9.466694343 |
| chr19 | 53780734 | 53780825 | + | FAM90A27P | 9.417240056 |
| chr8 | 7225284 | 7225573 | - | FAM66B | 9.401264025 |
| chr11 | 89773055 | 89773130 | - | NA | 9.245933427 |
| chr12 | 7966594 | 7966677 | + | NA | 9.221879327 |
| chr11 | 94711164 | 94711233 | - | CWC15 | 9.111654989 |
| chr11 | 71537346 | 71537638 | - | CTD-2313N18.5 | 9.030891296 |
| chr1 | 13644854 | 13645092 | - | NA | 9.016979924 |
| chr2 | 97699243 | 97699383 | - | FAM178B | 8.930571185 |
| chr22 | 29833684 | 29833719 | + | RFPL1 | 8.802619068 |
| chr10 | 42383521 | 42383690 | + | NA | 8.76981309 |
| chr11 | 94770530 | 94771725 | + | NA | 8.697520333 |
| chr1 | 13167116 | 13167177 | - | HNRNPCL1 | 8.621413035 |
| chr5 | 31356594 | 31356697 | - | NA | 8.574693771 |
| chr5 | 99844875 | 99844963 | + | FAM174A | 8.560377788 |
| chr1 | 13194534 | 13194595 | + | PRAMEF1 | 8.541066114 |
| chr10 | 62677216 | 62677320 | - | RHOBTB1 | 8.521492427 |
| chr2 | 96260002 | 96260182 | - | NA | 8.511605087 |
| chr11 | 108537249 | 108537424 | - | NA | 8.471363849 |
| chr5 | 17502661 | 17502750 | - | NA | 8.440428754 |
| chr11 | 49862897 | 49863032 | - | TRIM51FP | 8.387348922 |
| chr15 | 82821162 | 82821216 | + | NA | 8.371036534 |
| chr2 | 18776283 | 18776466 | - | RDH14 | 8.251373573 |
| chr5 | 65827029 | 65827146 | - | RP11-5P22.3 | 8.171993318 |
| chr6 | 73918595 | 73919709 | + | NA | 8.159383803 |
| chr15 | 82664406 | 82664461 | + | NA | 8.133829247 |
| chr17 | 48272928 | 48272965 | + | NA | 8.042637807 |
| chr5 | 17626085 | 17626158 | - | NA | 8.019870165 |
| chr6 | 115319341 | 115319583 | + | NA | 7.955685251 |
| chr17 | 74706705 | 74707081 | - | NA | 7.903711454 |
| chr12 | 8320120 | 8320229 | + | ZNF705A | 7.865406455 |
| chrX | 148697580 | 148697680 | - | TMEM185A | 7.785603333 |
| chr13 | 107227140 | 107227194 | + | NA | 7.752403624 |
| chr12 | 92455635 | 92455662 | - | C12orf79 | 7.656957003 |
| chr1 | 13379280 | 13379382 | + | NA | 7.629791555 |
| chr1 | 93080157 | 93080303 | + | NA | 7.602104759 |
| chr1 | 149782985 | 149783197 | - | NA | 7.564342605 |
| chr2 | 96182437 | 96182490 | - | NA | 7.564342605 |
| chr1 | 78120652 | 78120772 | - | ZZZ3 | 7.55474558 |
| chr10 | 65773722 | 65773915 | - | NA | 7.545084287 |
| chr11 | 4301136 | 4301236 | - | NA | 7.535357858 |
| chr1 | 12906710 | 12908259 | + | NA | 7.423801691 |
| chr16 | 75703261 | 75703382 | + | NA | 7.423801691 |
| chr5 | 17512525 | 17512550 | + | NA | 7.336884308 |
| chr8 | 99586067 | 99586130 | - | STK3 | 7.268077527 |
| chr10 | 93539029 | 93539135 | + | NA | 7.232403072 |
| chr2 | 220845376 | 220845490 | + | NA | 7.232403072 |
| chr16 | 18436514 | 18436549 | + | NA | 7.220312825 |
| chr5 | 111563948 | 111564049 | + | RP11-526F3.1 | 7.183422009 |
| chr10 | 97802754 | 97802992 | + | CCNJ | 7.170912422 |
| chr5 | 84006708 | 84006811 | + | NA | 7.170912422 |
| chr6 | 48213528 | 48213650 | + | NA | 7.145563057 |
| chr5 | 92840298 | 92840429 | - | NR2F1-AS1 | 7.119760304 |
| chr11 | 89534128 | 89534144 | + | NA | 7.080169698 |
| chr1 | 217380835 | 217380940 | + | NA | 7.066727659 |
| chr5 | 88388853 | 88388964 | - | NA | 7.039461912 |
| chr19 | 48307772 | 48307815 | - | TPRX1 | 7.025633335 |
| chr7 | 12695616 | 12695938 | - | NA | 6.894822624 |
| chr16 | 9068951 | 9069203 | - | USP7 | 6.848443576 |
| chr3 | 23958604 | 23958940 | + | NA | 6.832646543 |
| chr9 | 71974415 | 71974537 | + | FAM189A2 | 6.800523893 |
| chr5 | 17492055 | 17492165 | + | NA | 6.784190313 |
| chr10 | 76900361 | 76900435 | + | SAMD8 | 6.750957693 |
| chr16 | 18432905 | 18433076 | + | NA | 6.716941471 |
| chr4 | 77015192 | 77015395 | + | ART3 | 6.699627789 |
| chr7 | 36363866 | 36365527 | + | NA | 6.699627789 |
| chr19 | 48362485 | 48362528 | + | TPRX2P | 6.664364331 |
| chr13 | 35302378 | 35302513 | - | LINC00457 | 6.628217295 |
| chr19 | 8551137 | 8551289 | - | NA | 6.628217295 |
| chr8 | 122157017 | 122157217 | - | NA | 6.591141261 |
| chr11 | 89699216 | 89699320 | + | TRIM64DP | 6.572239703 |
| chr1 | 13184298 | 13184330 | - | HNRNPCL1 | 6.411389701 |
| chr7 | 20247607 | 20247753 | + | AC005083.1 | 6.389961405 |
| chr2 | 15433566 | 15433584 | - | NBAS | 6.368210031 |
| chr1 | 12987768 | 12987795 | - | NA | 6.346125687 |
| chr10 | 81802825 | 81802932 | - | NA | 6.346125687 |
| chr3 | 190058140 | 190058202 | - | CLDN1 | 6.346125687 |
| chr11 | 48965037 | 48965173 | + | TRIM51CP | 6.254244007 |
| chr5 | 130503196 | 130503322 | - | HINT1 | 6.254244007 |
| chr18 | 59561383 | 59561641 | - | RNF152 | 6.206011324 |
| chr9 | 102883662 | 102883780 | + | INVS | 6.206011324 |
| chr12 | 101138153 | 101138215 | - | NA | 6.156110168 |
| chr4 | 189834247 | 189834313 | - | NA | 6.156110168 |
| chr5 | 17530557 | 17530636 | + | NA | 6.156110168 |
| chr4 | 112508262 | 112508350 | - | NA | 6.130497041 |
| chr8 | 16685227 | 16685373 | - | MSR1 | 6.130497041 |
| chr1 | 13718465 | 13719774 | - | NA | 6.104420957 |
| chr3 | 101395301 | 101396953 | + | NA | 6.104420957 |
| chr4 | 100326575 | 100326741 | - | NA | 6.104420957 |
| chr6 | 73881910 | 73882068 | - | NA | 6.104420957 |
| chr1 | 13673449 | 13673503 | - | HNRNPCL1 | 5.937216385 |
| chr18 | 32485068 | 32486271 | - | NA | 5.937216385 |
| chr1 | 161720240 | 161720512 | + | DUSP12 | 5.907363085 |
| chr11 | 7048541 | 7048670 | - | ZNF214 | 5.907363085 |
| chr13 | 60529503 | 60529602 | + | DIAPH3-AS1 | 5.907363085 |
| chr14 | 20937562 | 20938010 | + | PNP | 5.907363085 |
| chr12 | 101078383 | 101078455 | - | NA | 5.876878962 |
| chr3 | 195078580 | 195078627 | - | ACAP2 | 5.876878962 |
| chr5 | 105790244 | 105790328 | - | NA | 5.84573678 |
| chr10 | 124444203 | 124444241 | - | NA | 5.781360119 |
| chr11 | 127937171 | 127937235 | - | NA | 5.781360119 |
| chr6 | 72266965 | 72266983 | + | NA | 5.781360119 |
| chr7 | 47092177 | 47093375 | + | NA | 5.781360119 |
| chr11 | 94781562 | 94781618 | + | NA | 5.748061483 |
| chr4 | 67651576 | 67651686 | + | NA | 5.748061483 |
| chr5 | 63434738 | 63434800 | - | RP11-158J3.2 | 5.748061483 |
| chr8 | 117688622 | 117688686 | - | EIF3H | 5.748061483 |
| chr1 | 149784825 | 149785233 | - | RP5-998N21.10 | 5.713976091 |
| chr10 | 112312194 | 112312317 | - | NA | 5.713976091 |
| chr11 | 89702032 | 89702079 | - | NA | 5.679065864 |
| chr5 | 83621975 | 83622055 | + | NA | 5.679065864 |
| chr7 | 95955231 | 95955416 | - | SLC25A13 | 5.679065864 |
| chr17 | 48264001 | 48264261 | + | COL1A1 | 5.653860671 |
| chr11 | 89798507 | 89798601 | - | TRIM64B | 5.64328989 |
| chr2 | 97690910 | 97695010 | + | NA | 5.606604134 |
| chr4 | 25649908 | 25651290 | - | NA | 5.606604134 |
| chrX | 38761101 | 38761146 | + | NA | 5.568961115 |
| chr1 | 225706761 | 225707038 | + | NA | 5.538815166 |
| chr11 | 89540328 | 89540433 | - | TRIM49 | 5.530309534 |
| chrX | 134070663 | 134070872 | - | MOSPD1 | 5.530309534 |
| chr12 | 78729371 | 78729457 | + | RP11-754N21.1 | 5.490593856 |
| chr17 | 34443465 | 34443610 | + | CTB-91J4.1 | 5.449753827 |
| chr8 | 12216654 | 12216734 | - | NA | 5.449753827 |
| chr1 | 13359585 | 13362056 | + | PRAMEF1 | 5.407723928 |
| chr4 | 89860398 | 89860505 | - | FAM13A | 5.407723928 |
| chr11 | 98458632 | 98458674 | - | NA | 5.364432738 |
| chr16 | 14967954 | 14969334 | - | NA | 5.364432738 |
| chr2 | 20646843 | 20647150 | + | NA | 5.364432738 |
| chr4 | 47670045 | 47670160 | + | NA | 5.364432738 |
| chr1 | 100783430 | 100783481 | + | NA | 5.273746817 |
| chr11 | 23097173 | 23097315 | + | NA | 5.273746817 |
| chr2 | 53214191 | 53214233 | - | NA | 5.273746817 |
| chr4 | 29906391 | 29906617 | - | NA | 5.273746817 |
| chr5 | 117873726 | 117873872 | - | NA | 5.273746817 |
| chr6 | 41048229 | 41048475 | + | NFYA | 5.273746817 |
| chr7 | 76326291 | 76326376 | - | NA | 5.273746817 |
| chrY | 8692183 | 8692205 | - | NA | 5.273746817 |
| chr1 | 13329020 | 13329084 | + | NA | 5.22617257 |
| chr19 | 21558743 | 21562044 | - | NA | 5.22617257 |
| chr7 | 122777567 | 122777762 | + | NA | 5.22617257 |
| chr21 | 37732834 | 37732900 | + | MORC3 | 5.176975862 |
| chr6 | 130088273 | 130088449 | - | ARHGAP18 | 5.176975862 |
| chr1 | 99139865 | 99140003 | - | NA | 5.126042111 |
| chr13 | 21406926 | 21406948 | - | XPO4 | 5.126042111 |
| chr5 | 33435768 | 33435924 | + | TARS | 5.126042111 |
| chr5 | 34013950 | 34014045 | - | AMACR | 5.126042111 |
| chr6 | 27861221 | 27861759 | + | NA | 5.126042111 |
| chr1 | 72181733 | 72181755 | - | NEGR1 | 5.073244149 |
| chr10 | 3985161 | 3985406 | + | NA | 5.073244149 |
| chr10 | 98277881 | 98281573 | + | NA | 5.073244149 |
| chr11 | 89752368 | 89752463 | - | NA | 5.073244149 |
| chr5 | 17499598 | 17502340 | + | NA | 5.073244149 |
| chr2 | 88256226 | 88256264 | + | NA | 5.018440312 |
| chr2 | 185462995 | 185463797 | - | NA | 5.018440312 |
| chrX | 19807571 | 19807687 | - | SH3KBP1 | 5.018440312 |
| chr2 | 8126628 | 8126780 | - | AC007464.1 | 4.961472148 |
| chr9 | 19379760 | 19380266 | - | RPS6 | 4.934804962 |
| chr1 | 149399955 | 149400170 | + | NA | 4.919156183 |
| chr1 | 13497631 | 13498199 | - | NA | 4.902161655 |
| chr5 | 157705581 | 157705855 | + | NA | 4.902161655 |
| chr5 | 170815096 | 170815269 | + | NPM1 | 4.902161655 |
| chr1 | 71479998 | 71480130 | - | PTGER3 | 4.840307925 |
| chr11 | 309274 | 309402 | - | NA | 4.840307925 |
| chr13 | 80295491 | 80295659 | + | NA | 4.840307925 |
| chr16 | 15204579 | 15204638 | + | NA | 4.840307925 |
| chr3 | 127006722 | 127006869 | - | NA | 4.840307925 |
| chr4 | 170861456 | 170861493 | + | NA | 4.840307925 |
| chr1 | 13452594 | 13452650 | - | HNRNPCL1 | 4.775683043 |
| chr15 | 72768897 | 72769037 | + | ARIH1 | 4.775683043 |
| chr4 | 145955498 | 145955647 | + | ABCE1 | 4.775683043 |
| chr5 | 17631573 | 17631836 | + | NA | 4.775683043 |
| chr8 | 27170348 | 27170473 | - | TRIM35 | 4.775683043 |
| chr10 | 81817002 | 81817039 | - | TMEM254-AS1 | 4.708027008 |
| chr17 | 49337481 | 49338310 | - | MBTD1 | 4.708027008 |
| chr2 | 130910541 | 130912049 | + | NA | 4.708027008 |
| chr3 | 169686341 | 169686379 | + | SEC62 | 4.708027008 |
| chr1 | 12987881 | 12988007 | - | NA | 4.637041422 |
| chr11 | 49039770 | 49039873 | - | RP11-56P9.11 | 4.637041422 |
| chr5 | 140700085 | 140700388 | - | NA | 4.637041422 |
| chr6 | 17661212 | 17661540 | - | NUP153 | 4.591622523 |
| chr11 | 32851299 | 32851408 | + | PRRG4 | 4.562381532 |
| chr11 | 49098722 | 49098850 | + | NA | 4.562381532 |
| chr17 | 72205198 | 72205453 | - | NA | 4.562381532 |
| chr2 | 175620783 | 175622750 | + | NA | 4.562381532 |
| chr2 | 224808860 | 224809124 | + | MRPL44 | 4.562381532 |
| chr5 | 16464017 | 16464242 | - | ZNF622 | 4.562381532 |
| chr7 | 129248461 | 129248597 | + | NRF1 | 4.562381532 |
| chr7 | 129083423 | 129083539 | - | NA | 4.562381532 |
| chr8 | 11960855 | 11960871 | + | ZNF705D | 4.562381532 |
| chr9 | 125693830 | 125694156 | + | NA | 4.562381532 |
| chr12 | 1499759 | 1499870 | - | RP5-951 N9.1 | 4.483646102 |
| chr12 | 94796327 | 94796678 | - | CCDC41 | 4.483646102 |
| chr19 | 47634057 | 47634369 | + | SAE1 | 4.483646102 |
| chr1 | 108741618 | 108742640 | + | NA | 4.400364358 |
| chr11 | 89550812 | 89550932 | + | RP11-358N4.6 | 4.400364358 |
| chr11 | 89759995 | 89760115 | - | NA | 4.400364358 |
| chr12 | 100661840 | 100661910 | - | DEPDC4 | 4.400364358 |
| chr19 | 10370500 | 10370728 | - | CTD-2369P2.4 | 4.400364358 |
| chr6 | 134440910 | 134441004 | + | NA | 4.400364358 |
| chr18 | 33065968 | 33066075 | - | INO80C | 4.387094146 |
| chr10 | 33225170 | 33225394 | - | ITGB1 | 4.311978929 |
| chr17 | 43224924 | 43226690 | + | NA | 4.311978929 |
| chr7 | 76279715 | 76279892 | - | POMZP3 | 4.311978929 |
| chr1 | 6258840 | 6259761 | - | RPL22 | 4.217823212 |
| chr1 | 24745287 | 24745384 | - | NA | 4.217823212 |
| chr1 | 36929820 | 36930025 | - | MRPS15 | 4.217823212 |
| chr12 | 29908642 | 29908935 | + | NA | 4.217823212 |
| chr13 | 20333669 | 20333819 | - | PSPC1 | 4.217823212 |
| chr13 | 31733656 | 31733699 | - | HSPH1 | 4.217823212 |
| chr17 | 53457382 | 53457511 | + | NA | 4.217823212 |
| chr19 | 56372609 | 56373496 | - | NA | 4.217823212 |
| chr5 | 176558884 | 176558935 | + | NSD1 | 4.217823212 |
| chr6 | 170686347 | 170686408 | + | NA | 4.217823212 |
| chr8 | 119122727 | 119124078 | - | NA | 4.217823212 |
| chr1 | 13630020 | 13634666 | - | NA | 4.117090824 |
| chr1 | 204379207 | 204381165 | - | NA | 4.117090824 |
| chr13 | 79974420 | 79974542 | - | RBM26 | 4.117090824 |
| chr14 | 73439836 | 73440220 | - | ZFYVE1 | 4.117090824 |
| chr21 | 23037347 | 23037492 | - | AF241725.6 | 4.117090824 |
| chr3 | 98695309 | 98695403 | - | NA | 4.117090824 |
| chr3 | 140476420 | 140476559 | - | NA | 4.117090824 |
| chr19 | 39923782 | 39924211 | + | NA | 4.04110172 |
| chr1 | 40810510 | 40810582 | + | SMAP2 | 4.008793563 |
| chr12 | 75905258 | 75905441 | - | KRR1 | 4.008793563 |
| chr12 | 116684064 | 116684499 | - | MED13L | 4.008793563 |
| chr13 | 37339203 | 37339373 | - | NA | 4.008793563 |
| chr16 | 15201187 | 15201971 | + | NA | 4.008793563 |
| chr16 | 29624424 | 29625016 | - | RP11-345J4.8 | 4.008793563 |
| chr3 | 100551108 | 100551149 | + | NA | 4.008793563 |
| chr5 | 16814012 | 16814209 | + | NA | 4.008793563 |
| chr5 | 21510284 | 21510466 | - | NA | 4.008793563 |
| chr5 | 59770096 | 59770123 | - | PDE4D | 4.008793563 |
| chr5 | 99711404 | 99711495 | - | NA | 4.008793563 |
| chr6 | 110502451 | 110502640 | + | CDC40 | 4.008793563 |
| chr6 | 160146852 | 160147885 | + | WTAP | 4.008793563 |
| chr6 | 143949483 | 143949679 | - | NA | 4.008793563 |
| chr9 | 86585075 | 86586273 | + | NA | 4.008793563 |
| chr9 | 100831626 | 100832020 | + | NANS | 4.008793563 |
| | | | | | |
| chr2 | 96150354 | 96150479 | - | TRIM43B | 16.0132856 |
| chr2 | 96257766 | 96257897 | + | TRIM43 | 15.59036927 |
| chr6 | 112668532 | 112668609 | + | RFPL4B | 14.85462215 |
| chr6 | 73935020 | 73935493 | - | KHDC1L | 14.44913335 |
| chr19 | 7049332 | 7049429 | + | MBD3L2 | 14.28612047 |
| chr19 | 58181877 | 58181935 | + | ZSCAN4 | 13.8337456 |
| chr19 | 7021364 | 7021442 | - | CTB-25J19.1 | 13.74026273 |
| chr22 | 32590332 | 32590376 | - | RFPL2 | 13.65581338 |
| chr1 | 12834984 | 12835297 | + | PRAMEF12 | 13.27445818 |
| chr13 | 37006409 | 37006866 | + | CCNA1 | 13.23199743 |
| chr16 | 48420965 | 48421141 | - | SIAH1 | 12.77410528 |
| chr1 | 12916941 | 12917002 | + | PRAMEF2 | 11.96820242 |
| chr11 | 89653467 | 89653576 | - | TRIM49D1 | 11.84664353 |
| chr11 | 89575165 | 89575355 | + | TRIM53BP | 11.48039287 |
| chr1 | 12851546 | 12851623 | + | PRAMEF1 | 11.36844009 |
| chr19 | 56270380 | 56270541 | + | RFPL4A | 11.27321198 |
| chr1 | 13673434 | 13673511 | - | PRAMEF14 | 11.06299372 |
| chr8 | 7287595 | 7287870 | - | DEFB103B | 11.05109744 |
| chr8 | 7738726 | 7739001 | + | DEFB103A | 11.05109744 |
| chr19 | 56751773 | 56751906 | + | ZSCAN5D | 10.7282791 |
| chr11 | 89732495 | 89732909 | - | TRIM53AP | 10.65494273 |
| chr1 | 13452579 | 13452656 | - | PRAMEF13 | 10.53438757 |
| chr11 | 89540267 | 89540402 | - | TRIM49 | 10.42011307 |
| chr1 | 13611493 | 13611550 | - | XX-FW84067D5.1 | 10.29887955 |
| chr19 | 56280507 | 56280541 | + | RFPL4AL1 | 10.23379054 |
| chr1 | 13219000 | 13219581 | - | PRAMEF26 | 10.18218354 |
| chr11 | 89765617 | 89765750 | + | TRIM49C | 10.16336199 |
| chr1 | 12976450 | 12976507 | + | PRAMEF7 | 10.07212774 |
| chr1 | 13390708 | 13390765 | - | PRAMEF8 | 9.951150073 |
| chr14 | 20937538 | 20937694 | + | PNP | 9.297342612 |
| chr11 | 55029658 | 55029787 | + | TRIM48 | 9.159928688 |
| chr1 | 13184265 | 13184326 | - | HNRNPCP5 | 9.156760212 |
| chr20 | 52214170 | 52214227 | - | ZNF217 | 8.866349967 |
| chr4 | 55095264 | 55095582 | + | PDGFRA | 8.786309325 |
| chr16 | 75733913 | 75734089 | - | AC025287.1 | 8.71054396 |
| chr20 | 48599536 | 48599678 | + | SNAI1 | 8.501649517 |
| chr8 | 86376081 | 86376344 | + | CA2 | 8.184493576 |
| chr19 | 45579334 | 45579846 | - | ZNF296 | 8.094499632 |
| chr11 | 55065335 | 55065708 | - | TRIM51HP | 8.047505157 |
| chr11 | 55072505 | 55072536 | - | RP11-72M10.5 | 7.948374079 |
| chr16 | 24549014 | 24549254 | + | RBBP6 | 7.92475922 |
| chr12 | 7864050 | 7864248 | + | DPPA3 | 7.88851128 |
| chr1 | 44584522 | 44584660 | + | KLF17 | 7.709799891 |
| chr1 | 13117674 | 13117751 | - | PRAMEF6 | 7.701126063 |
| chr13 | 37005967 | 37006303 | + | CCNA1 | 7.485717092 |
| chr19 | 48306761 | 48306946 | - | TPRX1 | 7.314311384 |
| chr12 | 86383178 | 86383327 | - | MGAT4C | 7.268077527 |
| chr6 | 122931377 | 122931680 | + | PKIB | 7.117289522 |
| chr22 | 22874432 | 22874613 | - | ZNF280A | 7.103056341 |
| chr8 | 58890917 | 58891043 | + | RP11-1112C15.1 | 6.997572061 |
| chr8 | 12213645 | 12213775 | + | ZNF705C | 6.918792793 |
| chr19 | 57656227 | 57656570 | - | ZIM3 | 6.832646543 |
| chr5 | 139725868 | 139726216 | - | HBEGF | 6.827259444 |
| chr1 | 118148556 | 118148775 | + | FAM46C | 6.734049835 |
| chr3 | 113557265 | 113557783 | + | GRAMD1C | 6.706281782 |
| chr12 | 11001973 | 11002074 | - | PRR4 | 6.699627789 |
| chr19 | 56709103 | 56709289 | - | ZSCAN5B | 6.628217295 |
| chr16 | 222846 | 223006 | + | HBA2 | 6.518444239 |
| chr16 | 226679 | 226810 | + | HBA1 | 6.518444239 |
| chr1 | 149400131 | 149400542 | - | HIST2H3PS2 | 6.499659495 |
| chr7 | 16793160 | 16793655 | + | TSPAN13 | 6.370701101 |
| chr18 | 23805900 | 23807240 | + | TAF4B | 6.367731166 |
| chr16 | 48399298 | 48399812 | - | SIAH1 | 6.325552489 |
| chr19 | 53811750 | 53811897 | - | FAM90A28P | 6.323698021 |
| chr5 | 114505305 | 114505658 | - | TRIM36 | 6.290738935 |
| chr8 | 121925305 | 121925399 | + | RP11-369K17.1 | 6.254244007 |
| chr17 | 17399210 | 17399709 | - | RASD1 | 6.181276488 |
| chr18 | 23872210 | 23872345 | + | TAF4B | 6.154920891 |
| chr14 | 75745477 | 75745826 | + | FOS | 6.102831106 |
| chr7 | 100472733 | 100472949 | + | SRRT | 6.098947907 |
| chr2 | 64371215 | 64371588 | - | PELI1 | 5.924357953 |
| chr2 | 219125738 | 219125939 | + | GPBAR1 | 5.84573678 |
| chr10 | 135440069 | 135440299 | - | FRG2B | 5.84573678 |
| chr1 | 71512364 | 71513491 | - | PTGER3 | 5.781360119 |
| chr4 | 190948182 | 190948412 | - | FRG2 | 5.781360119 |
| chr5 | 111312407 | 111312628 | - | NREP | 5.748061483 |
| chr1 | 163038935 | 163039318 | + | RGS4 | 5.730366772 |
| chr9 | 79056582 | 79057231 | + | GCNT1 | 5.713976091 |
| chr2 | 10588247 | 10588630 | - | ODC1 | 5.70592939 |
| chr3 | 67048727 | 67048795 | + | KBTBD8 | 5.679065864 |
| chr8 | 18871073 | 18871196 | - | PSD3 | 5.616239939 |
| chr8 | 7801144 | 7801188 | + | ZNF705B | 5.606604134 |
| chr19 | 6393952 | 6393992 | - | GTF2F1 | 5.568961115 |
| chr15 | 77154186 | 77154285 | - | SCAPER | 5.530309534 |
| chr3 | 197836983 | 197837254 | + | AC073135.3 | 5.490593856 |
| chr16 | 30388884 | 30389023 | + | MYLPF | 5.428661574 |
| chr3 | 75713481 | 75713708 | + | FRG2C | 5.364432738 |
| chr4 | 85504132 | 85504671 | + | CDS1 | 5.364432738 |
| chr7 | 90092554 | 90092685 | + | CLDN12 | 5.364432738 |
| chr17 | 70117161 | 70117963 | + | SOX9 | 5.360184419 |
| chr9 | 33447421 | 33447609 | - | AQP3 | 5.325498196 |
| chr4 | 48485360 | 48486168 | + | SLC10A4 | 5.31980221 |
| chr14 | 64319683 | 64319861 | + | SYNE2 | 5.31980221 |
| chr1 | 183774254 | 183774458 | + | RGL1 | 5.276905414 |
| chr1 | 31538536 | 31538838 | - | PUM1 | 5.273746817 |
| chr15 | 44487151 | 44487450 | - | FRMD5 | 5.228672732 |
| chr2 | 233271553 | 233271671 | + | ALPPL2 | 5.22617257 |
| chr5 | 137801179 | 137801757 | + | EGR1 | 5.21260049 |
| chr11 | 62572801 | 62572964 | - | NXF1 | 5.193737787 |
| chr6 | 13486870 | 13487894 | - | GFOD1 | 5.176975862 |
| chr11 | 8496228 | 8497371 | - | STK33 | 5.176975862 |
| chr2 | 136875616 | 136875735 | - | CXCR4 | 5.126042111 |
| chr8 | 80679810 | 80680098 | - | HEY1 | 5.045901046 |
| chr20 | 43324644 | 43324737 | - | RP11-445H22.3 | 5.018440312 |
| chr5 | 100238547 | 100238970 | - | ST8SIA4 | 5.017336709 |
| chr3 | 35721116 | 35721193 | + | ARPP21 | 4.961472148 |
| chr4 | 113437003 | 113437328 | - | NEUROG2 | 4.961472148 |
| chr17 | 43565094 | 43565228 | - | PLEKHM1 | 4.961472148 |
| chr8 | 99956631 | 99957051 | + | OSR2 | 4.939382759 |
| chr12 | 88974041 | 88974628 | - | KITLG | 4.934384285 |
| chrX | 18372712 | 18372847 | - | SCML2 | 4.930024493 |
| chr6 | 112575661 | 112575917 | - | LAMA4 | 4.840307925 |
| chr6 | 133034965 | 133035188 | - | VNN1 | 4.840307925 |
| chr8 | 95907405 | 95907484 | - | CCNE2 | 4.840307925 |
| chr22 | 50552887 | 50553051 | + | MOV10L1 | 4.840307925 |
| chr1 | 45956524 | 45956872 | - | TESK2 | 4.835288922 |
| chr12 | 104234727 | 104234975 | - | NT5DC3 | 4.834642435 |
| chr5 | 16916164 | 16916636 | - | MYO10 | 4.807979786 |
| chr5 | 142149949 | 142150480 | + | ARHGAP26 | 4.804021527 |
| chr7 | 16685756 | 16685913 | + | BZW2 | 4.774164646 |
| chr6 | 134495870 | 134496034 | - | SGK1 | 4.74766495 |
| chr15 | 75918580 | 75918810 | - | SNUPN | 4.716222341 |
| chr19 | 12163614 | 12163782 | - | CTD-2006C1.10 | 4.708027008 |
| chrX | 110909043 | 110909199 | + | ALG13 | 4.708027008 |
| chr1 | 171810621 | 171810957 | + | DNM3 | 4.665951298 |
| chr2 | 233245949 | 233246077 | + | ALPP | 4.637041422 |
| chr7 | 42276570 | 42276658 | - | GLI3 | 4.637041422 |
| chr4 | 80116988 | 80117086 | + | LINC01088 | 4.562381532 |
| chr5 | 94619560 | 94620279 | - | MCTP1 | 4.562381532 |
| chr6 | 112575644 | 112575912 | - | LAMA4 | 4.562381532 |
| chrX | 101771594 | 101771712 | - | TMSB15A | 4.562381532 |
| chr4 | 170581213 | 170581599 | + | CLCN3 | 4.545162472 |
| chr3 | 137893441 | 137893791 | - | DBR1 | 4.507898874 |
| chr14 | 90798185 | 90798481 | - | NRDE2 | 4.504087821 |
| chr5 | 21459589 | 21459693 | + | GUSBP1 | 4.500775595 |
| chr3 | 190999876 | 191000192 | - | UTS2B | 4.483646102 |
| chr4 | 42658842 | 42659122 | - | ATP8A1 | 4.483646102 |
| chr5 | 31855043 | 31855274 | + | PDZD2 | 4.483646102 |
| chr7 | 106685094 | 106685659 | + | PRKAR2B | 4.483646102 |
| chr12 | 88421541 | 88421740 | - | C12orf50 | 4.483646102 |
| chr14 | 50583005 | 50583318 | - | VCPKMT | 4.431697393 |
| chr3 | 36421836 | 36422246 | + | STAC | 4.400364358 |
| chr5 | 115909881 | 115910630 | - | SEMA6A | 4.400364358 |
| chr9 | 20683977 | 20684292 | + | FOCAD | 4.400364358 |
| chr10 | 20105168 | 20106120 | + | PLXDC2 | 4.400078642 |
| chr7 | 86688641 | 86689015 | - | KIAA1324L | 4.387094146 |
| chr13 | 27844464 | 27845205 | + | RASL11A | 4.387094146 |
| chr8 | 95274318 | 95274573 | - | GEM | 4.386301671 |
| chr5 | 68665484 | 68665840 | - | TAF9 | 4.362950898 |
| chr3 | 4535032 | 4535289 | + | ITPR1 | 4.360896156 |
| chr13 | 77459280 | 77460540 | - | KCTD12 | 4.360896156 |
| chr14 | 56585093 | 56585455 | + | PELI2 | 4.360896156 |
| chr6 | 16129356 | 16129640 | + | MYLIP | 4.358009514 |
| chr2 | 62423248 | 62423490 | + | B3GNT2 | 4.350962764 |
| chr1 | 87797351 | 87797497 | + | LMO4 | 4.332522112 |
| chr1 | 63787987 | 63788129 | - | RP4-792G4.2 | 4.311978929 |
| chr2 | 165477629 | 165478358 | - | GRB14 | 4.311978929 |
| chr4 | 90228766 | 90229161 | - | GPRIN3 | 4.311978929 |
| chr7 | 16700897 | 16700937 | + | BZW2 | 4.311978929 |
| chr12 | 10705962 | 10706115 | + | RP11-291B21.2 | 4.311978929 |
| chr13 | 92050929 | 92051463 | + | GPC5 | 4.311978929 |
| chr21 | 34100257 | 34100359 | - | SYNJ1 | 4.288362549 |
| chr10 | 90712488 | 90712530 | - | ACTA2 | 4.27315395 |
| chr6 | 126112001 | 126112213 | + | NCOA7 | 4.267579352 |
| chr2 | 216877956 | 216878346 | - | MREG | 4.253517997 |
| chr1 | 201390801 | 201390874 | - | TNNI1 | 4.248833977 |
| chr11 | 23186733 | 23186879 | + | RP11-266A24.1 | 4.248833977 |
| chr5 | 128430444 | 128430768 | + | ISOC1 | 4.238721757 |
| chr6 | 117586721 | 117587007 | + | VGLL2 | 4.229981016 |
| chr11 | 82612740 | 82612864 | + | C11orf82 | 4.210791648 |
| chr9 | 23821603 | 23821846 | - | ELAVL2 | 4.20854738 |
| chr13 | 102068664 | 102068813 | - | NALCN | 4.19963727 |
| chr12 | 39836729 | 39837192 | - | KIF21A | 4.159303608 |
| chr5 | 10564442 | 10564945 | + | ANKRD33B | 4.124393381 |
| chr2 | 191334219 | 191334375 | + | MFSD6 | 4.117090824 |
| chr9 | 84887671 | 84888396 | + | RP11-15B24.5 | 4.117090824 |
| chr14 | 38064106 | 38064315 | - | FOXA1 | 4.117090824 |
| chr18 | 57333308 | 57333394 | - | CCBE1 | 4.117090824 |
| chr3 | 153839149 | 153839308 | + | ARHGEF26 | 4.095905557 |
| chr2 | 179059208 | 179059401 | + | OSBPL6 | 4.062792481 |
| chr12 | 8088616 | 8088871 | - | SLC2A3 | 4.048750617 |
| chr8 | 80992550 | 80993051 | - | TPD52 | 4.04110172 |
| chr11 | 3387921 | 3388349 | - | ZNF195 | 4.04110172 |
| chr3 | 9701310 | 9701493 | + | MTMR14 | 4.008793563 |
| chr4 | 175443510 | 175444049 | - | HPGD | 4.008793563 |
| chr5 | 115814238 | 115814373 | - | SEMA6A | 4.008793563 |
| chr5 | 149681626 | 149682516 | - | ARSI | 4.008793563 |
| chr6 | 46293130 | 46293405 | - | RCAN2 | 4.008793563 |
| chr8 | 12275477 | 12275599 | - | FAM90A25P | 4.008793563 |
| chr10 | 90435250 | 90435414 | + | LIPF | 4.008793563 |
| chr10 | 96305547 | 96305664 | + | HELLS | 4.008793563 |
| chr11 | 76502796 | 76503813 | + | TSKU | 4.008793563 |
| chr19 | 52780405 | 52780794 | + | ZNF766 | 4.008793563 |

**Table 9**

| | **start** | **end** | **strand** | **Gene.name** | **diff** |
|---|---|---|---|---|---|
| chr2 | 96150354 | 96150479 | - | TRIM43B | 16.0132856 |
| chr2 | 96257766 | 96257897 | + | TRIM43 | 15.59036927 |
| chr6 | 112668532 | 112668609 | + | RFPL4B | 14.85462215 |
| chr6 | 73935020 | 73935493 | - | KHDC1L | 14.44913335 |
| chr19 | 7049332 | 7049429 | + | MBD3L2 | 14.28612047 |
| chr19 | 58181877 | 58181935 | + | ZSCAN4 | 13.8337456 |
| chr19 | 7021364 | 7021442 | - | CTB-25J19.1 | 13.74026273 |
| chr22 | 32590332 | 32590376 | - | RFPL2 | 13.65581338 |
| chr1 | 12834984 | 12835297 | + | PRAMEF12 | 13.27445818 |
| chr13 | 37006409 | 37006866 | + | CCNA1 | 13.23199743 |
| chr16 | 48420965 | 48421141 | - | SIAH1 | 12.77410528 |
| chr1 | 12916941 | 12917002 | + | PRAMEF2 | 11.96820242 |
| chr11 | 89653467 | 89653576 | - | TRIM49D1 | 11.84664353 |
| chr11 | 89575165 | 89575355 | + | TRIM53BP | 11.48039287 |
| chr1 | 12851546 | 12851623 | + | PRAMEF1 | 11.36844009 |
| chr19 | 56270380 | 56270541 | + | RFPL4A | 11.27321198 |
| chr1 | 13673434 | 13673511 | - | PRAMEF14 | 11.06299372 |
| chr8 | 7287595 | 7287870 | - | DEFB103B | 11.05109744 |
| chr8 | 7738726 | 7739001 | + | DEFB103A | 11.05109744 |
| chr19 | 56751773 | 56751906 | + | ZSCAN5D | 10.7282791 |
| chr11 | 89732495 | 89732909 | - | TRIM53AP | 10.65494273 |
| chr1 | 13452579 | 13452656 | - | PRAMEF13 | 10.53438757 |
| chr11 | 89540267 | 89540402 | - | TRIM49 | 10.42011307 |
| chr1 | 13611493 | 13611550 | - | XX-FW84067D5.1 | 10.29887955 |
| chr19 | 56280507 | 56280541 | + | RFPL4AL1 | 10.23379054 |
| chr1 | 13219000 | 13219581 | - | PRAMEF26 | 10.18218354 |
| chr11 | 89765617 | 89765750 | + | TRIM49C | 10.16336199 |
| chr1 | 12976450 | 12976507 | + | PRAMEF7 | 10.07212774 |
| chr1 | 13390708 | 13390765 | - | PRAMEF8 | 9.951150073 |
| chr14 | 20937538 | 20937694 | + | PNP | 9.297342612 |
| chr11 | 55029658 | 55029787 | + | TRIM48 | 9.159928688 |
| chr1 | 13184265 | 13184326 | - | HNRNPCP5 | 9.156760212 |
| chr20 | 52214170 | 52214227 | - | ZNF217 | 8.866349967 |
| chr4 | 55095264 | 55095582 | + | PDGFRA | 8.786309325 |
| chr16 | 75733913 | 75734089 | - | AC025287.1 | 8.71054396 |
| chr20 | 48599536 | 48599678 | + | SNAI1 | 8.501649517 |
| chr8 | 86376081 | 86376344 | + | CA2 | 8.184493576 |
| chr19 | 45579334 | 45579846 | - | ZNF296 | 8.094499632 |
| chr11 | 55065335 | 55065708 | - | TRIM51 HP | 8.047505157 |
| chr11 | 55072505 | 55072536 | - | RP11-72M10.5 | 7.948374079 |
| chr16 | 24549014 | 24549254 | + | RBBP6 | 7.92475922 |
| chr12 | 7864050 | 7864248 | + | DPPA3 | 7.88851128 |
| chr1 | 44584522 | 44584660 | + | KLF17 | 7.709799891 |
| chr1 | 13117674 | 13117751 | - | PRAMEF6 | 7.701126063 |
| chr13 | 37005967 | 37006303 | + | CCNA1 | 7.485717092 |
| chr19 | 48306761 | 48306946 | - | TPRX1 | 7.314311384 |
| chr12 | 86383178 | 86383327 | - | MGAT4C | 7.268077527 |
| chr6 | 122931377 | 122931680 | + | PKIB | 7.117289522 |
| chr22 | 22874432 | 22874613 | - | ZNF280A | 7.103056341 |
| chr8 | 58890917 | 58891043 | + | RP11-1112C15.1 | 6.997572061 |
| chr8 | 12213645 | 12213775 | + | ZNF705C | 6.918792793 |
| chr19 | 57656227 | 57656570 | - | ZIM3 | 6.832646543 |
| chr5 | 139725868 | 139726216 | - | HBEGF | 6.827259444 |
| chr1 | 118148556 | 118148775 | + | FAM46C | 6.734049835 |
| chr3 | 113557265 | 113557783 | + | GRAMD1C | 6.706281782 |
| chr12 | 11001973 | 11002074 | - | PRR4 | 6.699627789 |
| chr19 | 56709103 | 56709289 | - | ZSCAN5B | 6.628217295 |
| chr16 | 222846 | 223006 | + | HBA2 | 6.518444239 |
| chr16 | 226679 | 226810 | + | HBA1 | 6.518444239 |
| chr1 | 149400131 | 149400542 | - | HIST2H3PS2 | 6.499659495 |
| chr7 | 16793160 | 16793655 | + | TSPAN13 | 6.370701101 |
| chr18 | 23805900 | 23807240 | + | TAF4B | 6.367731166 |
| chr16 | 48399298 | 48399812 | - | SIAH1 | 6.325552489 |
| chr19 | 53811750 | 53811897 | - | FAM90A28P | 6.323698021 |
| chr5 | 114505305 | 114505658 | - | TRIM36 | 6.290738935 |
| chr8 | 121925305 | 121925399 | + | RP11-369K17.1 | 6.254244007 |
| chr17 | 17399210 | 17399709 | - | RASD1 | 6.181276488 |
| chr18 | 23872210 | 23872345 | + | TAF4B | 6.154920891 |
| chr14 | 75745477 | 75745826 | + | FOS | 6.102831106 |
| chr7 | 100472733 | 100472949 | + | SRRT | 6.098947907 |
| chr2 | 64371215 | 64371588 | - | PELI1 | 5.924357953 |
| chr2 | 219125738 | 219125939 | + | GPBAR1 | 5.84573678 |
| chr10 | 135440069 | 135440299 | - | FRG2B | 5.84573678 |
| chr1 | 71512364 | 71513491 | - | PTGER3 | 5.781360119 |
| chr4 | 190948182 | 190948412 | - | FRG2 | 5.781360119 |
| chr5 | 111312407 | 111312628 | - | NREP | 5.748061483 |
| chr1 | 163038935 | 163039318 | + | RGS4 | 5.730366772 |
| chr9 | 79056582 | 79057231 | + | GCNT1 | 5.713976091 |
| chr2 | 10588247 | 10588630 | - | ODC1 | 5.70592939 |
| chr3 | 67048727 | 67048795 | + | KBTBD8 | 5.679065864 |
| chr8 | 18871073 | 18871196 | - | PSD3 | 5.616239939 |
| chr8 | 7801144 | 7801188 | + | ZNF705B | 5.606604134 |
| chr19 | 6393952 | 6393992 | - | GTF2F1 | 5.568961115 |
| chr15 | 77154186 | 77154285 | - | SCAPER | 5.530309534 |
| chr3 | 197836983 | 197837254 | + | AC073135.3 | 5.490593856 |
| chr16 | 30388884 | 30389023 | + | MYLPF | 5.428661574 |
| chr3 | 75713481 | 75713708 | + | FRG2C | 5.364432738 |
| chr4 | 85504132 | 85504671 | + | CDS1 | 5.364432738 |
| chr7 | 90092554 | 90092685 | + | CLDN12 | 5.364432738 |
| chr17 | 70117161 | 70117963 | + | SOX9 | 5.360184419 |
| chr9 | 33447421 | 33447609 | - | AQP3 | 5.325498196 |
| chr4 | 48485360 | 48486168 | + | SLC10A4 | 5.31980221 |
| chr14 | 64319683 | 64319861 | + | SYNE2 | 5.31980221 |
| chr1 | 183774254 | 183774458 | + | RGL1 | 5.276905414 |
| chr1 | 31538536 | 31538838 | - | PUM1 | 5.273746817 |
| chr15 | 44487151 | 44487450 | - | FRMD5 | 5.228672732 |
| chr2 | 233271553 | 233271671 | + | ALPPL2 | 5.22617257 |
| chr5 | 137801179 | 137801757 | + | EGR1 | 5.21260049 |
| chr11 | 62572801 | 62572964 | - | NXF1 | 5.193737787 |
| chr6 | 13486870 | 13487894 | - | GFOD1 | 5.176975862 |
| chr11 | 8496228 | 8497371 | - | STK33 | 5.176975862 |
| chr2 | 136875616 | 136875735 | - | CXCR4 | 5.126042111 |
| chr8 | 80679810 | 80680098 | - | HEY1 | 5.045901046 |
| chr20 | 43324644 | 43324737 | - | RP11-445H22.3 | 5.018440312 |
| chr5 | 100238547 | 100238970 | - | ST8SIA4 | 5.017336709 |
| chr3 | 35721116 | 35721193 | + | ARPP21 | 4.961472148 |
| chr4 | 113437003 | 113437328 | - | NEUROG2 | 4.961472148 |
| chr17 | 43565094 | 43565228 | - | PLEKHM1 | 4.961472148 |
| chr8 | 99956631 | 99957051 | + | OSR2 | 4.939382759 |
| chr12 | 88974041 | 88974628 | - | KITLG | 4.934384285 |
| chrX | 18372712 | 18372847 | - | SCML2 | 4.930024493 |
| chr6 | 112575661 | 112575917 | - | LAMA4 | 4.840307925 |
| chr6 | 133034965 | 133035188 | - | VNN1 | 4.840307925 |
| chr8 | 95907405 | 95907484 | - | CCNE2 | 4.840307925 |
| chr22 | 50552887 | 50553051 | + | MOV10L1 | 4.840307925 |
| chr1 | 45956524 | 45956872 | - | TESK2 | 4.835288922 |
| chr12 | 104234727 | 104234975 | - | NT5DC3 | 4.834642435 |
| chr5 | 16916164 | 16916636 | - | MYO10 | 4.807979786 |
| chr5 | 142149949 | 142150480 | + | ARHGAP26 | 4.804021527 |
| chr7 | 16685756 | 16685913 | + | BZW2 | 4.774164646 |
| chr6 | 134495870 | 134496034 | - | SGK1 | 4.74766495 |
| chr15 | 75918580 | 75918810 | - | SNUPN | 4.716222341 |
| chr19 | 12163614 | 12163782 | - | CTD-2006C1.10 | 4.708027008 |
| chrX | 110909043 | 110909199 | + | ALG13 | 4.708027008 |
| chr1 | 171810621 | 171810957 | + | DNM3 | 4.665951298 |
| chr2 | 233245949 | 233246077 | + | ALPP | 4.637041422 |
| chr7 | 42276570 | 42276658 | - | GLI3 | 4.637041422 |
| chr4 | 80116988 | 80117086 | + | LINC01088 | 4.562381532 |
| chr5 | 94619560 | 94620279 | - | MCTP1 | 4.562381532 |
| chr6 | 112575644 | 112575912 | - | LAMA4 | 4.562381532 |
| chrX | 101771594 | 101771712 | - | TMSB15A | 4.562381532 |
| chr4 | 170581213 | 170581599 | + | CLCN3 | 4.545162472 |
| chr3 | 137893441 | 137893791 | - | DBR1 | 4.507898874 |
| chr14 | 90798185 | 90798481 | - | NRDE2 | 4.504087821 |
| chr5 | 21459589 | 21459693 | + | GUSBP1 | 4.500775595 |
| chr3 | 190999876 | 191000192 | - | UTS2B | 4.483646102 |
| chr4 | 42658842 | 42659122 | - | ATP8A1 | 4.483646102 |
| chr5 | 31855043 | 31855274 | + | PDZD2 | 4.483646102 |
| chr7 | 106685094 | 106685659 | + | PRKAR2B | 4.483646102 |
| chr12 | 88421541 | 88421740 | - | C12orf50 | 4.483646102 |
| chr14 | 50583005 | 50583318 | - | VCPKMT | 4.431697393 |
| chr3 | 36421836 | 36422246 | + | STAC | 4.400364358 |
| chr5 | 115909881 | 115910630 | - | SEMA6A | 4.400364358 |
| chr9 | 20683977 | 20684292 | + | FOCAD | 4.400364358 |
| chr10 | 20105168 | 20106120 | + | PLXDC2 | 4.400078642 |
| chr7 | 86688641 | 86689015 | - | KIAA1324L | 4.387094146 |
| chr13 | 27844464 | 27845205 | + | RASL11A | 4.387094146 |
| chr8 | 95274318 | 95274573 | - | GEM | 4.386301671 |
| chr5 | 68665484 | 68665840 | - | TAF9 | 4.362950898 |
| chr3 | 4535032 | 4535289 | + | ITPR1 | 4.360896156 |
| chr13 | 77459280 | 77460540 | - | KCTD12 | 4.360896156 |
| chr14 | 56585093 | 56585455 | + | PELI2 | 4.360896156 |
| chr6 | 16129356 | 16129640 | + | MYLIP | 4.358009514 |
| chr2 | 62423248 | 62423490 | + | B3GNT2 | 4.350962764 |
| chr1 | 87797351 | 87797497 | + | LMO4 | 4.332522112 |
| chr1 | 63787987 | 63788129 | - | RP4-792G4.2 | 4.311978929 |
| chr2 | 165477629 | 165478358 | - | GRB14 | 4.311978929 |
| chr4 | 90228766 | 90229161 | - | GPRIN3 | 4.311978929 |
| chr7 | 16700897 | 16700937 | + | BZW2 | 4.311978929 |
| chr12 | 10705962 | 10706115 | + | RP11-291B21.2 | 4.311978929 |
| chr13 | 92050929 | 92051463 | + | GPC5 | 4.311978929 |
| chr21 | 34100257 | 34100359 | - | SYNJ1 | 4.288362549 |
| chr10 | 90712488 | 90712530 | - | ACTA2 | 4.27315395 |
| chr6 | 126112001 | 126112213 | + | NCOA7 | 4.267579352 |
| chr2 | 216877956 | 216878346 | - | MREG | 4.253517997 |
| chr1 | 201390801 | 201390874 | - | TNNI1 | 4.248833977 |
| chr11 | 23186733 | 23186879 | + | RP11-266A24.1 | 4.248833977 |
| chr5 | 128430444 | 128430768 | + | ISOC1 | 4.238721757 |
| chr6 | 117586721 | 117587007 | + | VGLL2 | 4.229981016 |
| chr11 | 82612740 | 82612864 | + | C11orf82 | 4.210791648 |
| chr9 | 23821603 | 23821846 | - | ELAVL2 | 4.20854738 |
| chr13 | 102068664 | 102068813 | - | NALCN | 4.19963727 |
| chr12 | 39836729 | 39837192 | - | KIF21A | 4.159303608 |
| chr5 | 10564442 | 10564945 | + | ANKRD33B | 4.124393381 |
| chr2 | 191334219 | 191334375 | + | MFSD6 | 4.117090824 |
| chr9 | 84887671 | 84888396 | + | RP11-15B24.5 | 4.117090824 |
| chr14 | 38064106 | 38064315 | - | FOXA1 | 4.117090824 |
| chr18 | 57333308 | 57333394 | - | CCBE1 | 4.117090824 |
| chr3 | 153839149 | 153839308 | + | ARHGEF26 | 4.095905557 |
| chr2 | 179059208 | 179059401 | + | OSBPL6 | 4.062792481 |
| chr12 | 8088616 | 8088871 | - | SLC2A3 | 4.048750617 |
| chr8 | 80992550 | 80993051 | - | TPD52 | 4.04110172 |
| chr11 | 3387921 | 3388349 | - | ZNF195 | 4.04110172 |
| chr3 | 9701310 | 9701493 | + | MTMR14 | 4.008793563 |
| chr4 | 175443510 | 175444049 | - | HPGD | 4.008793563 |
| chr5 | 115814238 | 115814373 | - | SEMA6A | 4.008793563 |
| chr5 | 149681626 | 149682516 | - | ARSI | 4.008793563 |
| chr6 | 46293130 | 46293405 | - | RCAN2 | 4.008793563 |
| chr8 | 12275477 | 12275599 | - | FAM90A25P | 4.008793563 |
| chr10 | 90435250 | 90435414 | + | LIPF | 4.008793563 |
| chr10 | 96305547 | 96305664 | + | HELLS | 4.008793563 |
| chr11 | 76502796 | 76503813 | + | TSKU | 4.008793563 |
| chr19 | 52780405 | 52780794 | + | ZNF766 | 4.008793563 |

### EXAMPLE 4 - DUX4 regulates a gene expression program in Facioscapulohumeral Dystrophy that is rich in repetitive elements and repetitive gene clusters

DUX4 is a double-homeodomain retrogene located in the D4Z4 macrosatellite arrays. It is normally expressed in the testis and epigenetically repressed in somatic tissues. Deletions of the D4Z4 array to < 10 repeat units or mutations in SMCHD1 decrease the epigenetic repression of DUX4. Mis-expression of DUX4 in skeletal muscle causes Facioscapulohumeral muscular dystrophy (FSHD) (**FIG. 11**).

DUX4 binds a double-homeobox motif at ∼60,000 sites in the mappable genome (**FIG. 12**).

∼1/3 of binding sites overlap with MaLR (mammalian apparent LTR-retrotransposon) elements, and enriched in endogenous retrovirus (ERVL and ERVK) elements and pericentromeric satellite HSATII sequences (**FIG. 13**).

Compare RNA-Seq data from Dux4 induced muscle cells, FSHD/normal cultured muscle cells and biopsies. DUX4 target gene expression is the major molecular signature in FSHD muscle. DUX4 targets expression in biopsies (**FIG. 14**). DUX4 activates repetitive gene clusters (**FIG. 15** and **FIG. 16**).

DUX4 binds at the basal promoter regions of its top targets, and activates many novel genes or novel promoters of known genes. Examine DUX4 binding sites and motifs within +/- 3000 bp of annotated or predicted TSS of its top targets

Dux4 binding sites are located in inaccessible chromatin in myoblasts based on DNasel hypersensitivity, in contrasts to classical transcription factors such as MyoD.

DUX4 binds regions enriched for repetitive elements and activates a gene expression program dominated by repetitive gene clusters that are silent in somatic tissues.

### REFERENCES

The following references provide exemplary procedural or other details supplementary to those set forth herein.
Arashiro, et al., Proc Natl Acad Sci USA. 106: 6220-6225, 2009.
Balog, et al., Epigenetics. 7: 579-584, 2012.
Block, et al., Hum Mol Genet. 22: 4661-4672, 2013.
Caruso, et al., PLoS Genet. 9: el003550, 2013.
Friedman, et al., Muscle Nerve. 45: 500-506, 2012.
Friedman, et al., Muscle Nerve. Doi: 10.1002/mus.23911, 2013.
Frisullo, et al., J Clin Immunol. 31: 155-166, 2011.
Gabellini, et al., Nature. 439: 973-977, 2006.
Geng, et al., Dev Cell. 22: 38-51, 2012.
Jones, et al., Hum Mol Genet. 21: 4419-4430, 2012.
Krom, et al., Am JPathol. 181: 1387-1401, 2012.
Laoudj-Chenivesse, et al., J Mol Med (Berl). 83: 216-224, 2005.
Lemmers, et al., Nat Genet. 44: 1370-1374, 2012.
Lemmers, et al., Science. 329: 1650-1653,2010.
Osborne, et al., Neurology. 68: 569-577, 2007.
Pandya, et al., Phys Ther. 88: 105-113, 2008.
Rahimov, et al., Proc Natl Acad Sci USA. 109: 16234-16239, 2012.
Ricci, et al., Ann Neurol. 45: 751-757, 1999.
Semple, et al., Eur J Immunol. 40: 1073-1078, 2010.
Snider, et al., PLoS Genet. 6: e1001181, 2010.
van der Maarel, et al., Curr Opin Neurol. 25: 614-620, 2012.
van Overveld, et al., Ann Neurol. 58: 569-576, 2005.
Welle, et al., J Appl Physiol (1985). 89: 297-304, 2000.
Welle, et al., Exp Gerontol. 39: 369-377, 2004.
Winokur, et al., Hum Mol Genet. 12: 2895-2907, 2003.
Young, et al., PLoS Genet. 9: e1003947, 2013.

## Claims

1. A method of determining the presence of, or risk of developing, facioscapulohumeral dystrophy (FSHD) in a patient, comprising:
a) measuring in a sample obtained from a patient increased expression of at least one FSHD biomarker relative to an expression level in a normal reference standard or normal control sample, wherein the FSHD biomarker comprises LEUTX; and
b) identifying the patient as having a risk of developing FSHD.

2. The method of claim 1, wherein the sample is a tissue sample, a blood sample, a urine sample, a saliva sample, a serum sample, a plasma sample, a tear sample, a fetus sample, or a fecal sample.

3. The method of claim 1 or 2, wherein the method comprises assaying nucleic acid or protein level in the sample.

4. The method of any one of claims 1-3, further comprising calculating a risk score of developing FSHD for the patient based on the increased expression.

5. The method of any one of claims 1-4, further comprising measuring in the sample the expression of FSHD biomarkers comprising at least 2, 3, 4, 5 or 10 of the genes listed in Table 6 or of the genes or exons listed in Table 1 and/or Table 2.

6. The method of claim 5, further comprising measuring in the sample the expression of FSHD biomarkers comprising at least 1 or 2 of the genes PRAMEF2, TRIM43 and KHDC1L.

7. The method of any one of claims 1-6, comprising measuring in the sample the expression of the FSHD biomarkers LEUTX, PRAMEF2, TRIM43 and KHDC1L.

8. The method of any one of claims 1-7, wherein the sample is a muscle sample.

## Patentansprüche

1. Verfahren des Bestimmens des Vorhandenseins oder des Risikos der Entwicklung einer facioscapulohumeralen Dystrophie (FSHD) in einem Patienten, umfassend:
a) Messen in einer von einem Patienten erhaltenen Probe von mindestens einem FSHD-Biomarkers relativ zu einem Expressionsniveau in einem normalen Referenzstandard oder einer normalen Kontrollprobe, wobei der FSHD-Biomarker LEUTX umfasst; und
b) Identifizieren des Patienten als Patient, bei dem ein Risiko für die Entwicklung von FSHD besteht.

2. Verfahren nach Anspruch 1, worin die Probe eine Gewebeprobe, eine Blutprobe, eine Urinprobe, eine Speichelprobe, eine Serumprobe, eine Plasmaprobe, eine Tränenprobe, eine fötale Probe oder eine Fäkalprobe ist.

3. Verfahren nach Anspruch 1 oder 2, wobei das Verfahren das Testen des Nukleinsäure- oder Proteingehalts in der Probe umfasst.

4. Verfahren nach einem der Ansprüche 1-3, ferner umfassend das Berechnen einer Risikobewertung der Entwicklung von FSHD für den Patienten basierend auf der erhöhten Expression.

5. Verfahren nach einem der Ansprüche 1-4, ferner umfassend das Messen in der Probe der Expression von FSHD-Biomarkern, die mindestens 2, 3, 4, 5 oder 10 der in der Tabelle 6 aufgeführten Gene oder der in der Tabelle 1 und/oder Tabelle 2 aufgeführten Gene oder Exons umfassen.

6. Verfahren nach Anspruch 5, ferner umfassend das Messen in der Probe der Expression von FSHD-Biomarkern, die mindestens 1 oder 2 der Gene PRAMEF2, TRIM43 und KHDC1L umfassen.

7. Verfahren nach einem der Ansprüche 1-6, umfassend das Messen in der Probe der Expression der FSHD-Biomarker LEUTX, PRAMEF2, TRIM43 und KHDC1L.

8. Verfahren nach einem der Ansprüche 1-7, wobei die Probe eine Muskelprobe ist.

## Revendications

1. Procédé de détermination de la présence de dystrophie facio-scapulo-humérale (FSHD) ou du risque de développer celle-ci chez un patient, comprenant :
a) la mesure dans un échantillon prélevé sur un patient d'une expression accrue d'au moins un biomarqueur de la FSHD par rapport à un niveau d'expression dans une norme de référence normale ou un échantillon témoin normal, le biomarqueur de la FSHD comprenant LEUTX ; et
b) l'identification du patient comme présentant un risque de développer la FSHD.

2. Procédé selon la revendication 1, dans lequel l'échantillon est un échantillon de tissu, un échantillon de sang, un échantillon d'urine, un échantillon de salive, un échantillon de sérum, un échantillon de plasma, un échantillon de larme, un échantillon de fœtus ou un échantillon fécal.

3. Procédé selon la revendication 1 ou 2, le procédé comprenant l'analyse du niveau d'acide nucléique ou de protéine dans l'échantillon.

4. Procédé selon l'une quelconque des revendications 1 à 3, comprenant en outre le calcul d'un niveau de risque de développer la FSHD pour le patient en fonction de l'expression accrue.

5. Procédé selon l'une quelconque des revendications 1 à 4, comprenant en outre la mesure dans l'échantillon de l'expression de biomarqueurs de la FSHD comprenant au moins 2, 3, 4, 5 ou 10 des gènes énumérés dans le tableau 6 ou des gènes ou exons énumérés dans le tableau 1 et/ou le tableau 2.

6. Procédé selon la revendication 5, comprenant en outre la mesure dans l'échantillon de l'expression de biomarqueurs de la FSHD comprenant au moins un ou deux des gènes PRAMEF2, TRIM43 et KHDC1L.

7. Procédé selon l'une quelconque des revendications 1 à 6, comprenant la mesure dans l'échantillon de l'expression des biomarqueurs LEUTX, PRAMEF2, TRIM43 et KHDC1L de la FSHD.

8. Procédé selon l'une quelconque des revendications 1 à 7, dans lequel l'échantillon est un échantillon de muscle.
